# EUROPÄISCHE PATENTSCHRIFT

(11) **EP 1 051 495 B1**
(45) Veröffentlichungstag und Bekanntmachung des Hinweises auf die Patenterteilung: **25.01.2006**
(21) Anmeldenummer: 99910186.8
(22) Anmeldetag: 03.02.1999
(51) Int. Cl.: C12N 15/29, C12N 15/11, C12N 15/82, C12N 15/86, C12N 5/10, C12N 1/21, C07K 14/42, A61K 31/70, A61K 38/16, A61K 48/00

(54) **REKOMBINANTE MISTELLEKTINE**
RECOMBINANT MISTLETOE LECTINES
LECTINES DE GUI RECOMBINEES

(30) Priorität: 03.02.1998 DE 19804210
(43) Veröffentlichungstag der Anmeldung: 15.11.2000
(73) Patentinhaber: Biosyn Arzneimittel GmbH, 70702 Fellbach (DE)
(72) Erfinder: MORRIS, Peter, Roslin Midlothian EH25 9LJ (GB); STIEFEL, Thomas, D-70184 Stuttgart (DE); VOELTER, Wolfgang, D-72070 Tübingen (DE); WELTERS, Peter, D-41334 Nettetal (DE)
(74) Vertreter: Grünecker, Kinkeldey, Stockmair & Schwanhäusser Anwaltssozietät
(86) Internationale Anmeldenummer: PCT/EP1999/000696
(87) Internationale Veröffentlichungsnummer: WO 1999/040109

(56) Entgegenhaltungen:
- EP-A- 0 751 221
- EP-A- 0 884 388
- WO-A-96/21458
- WO-A-98/29540
- US-A- 5 091 309
- HARRIS J D ET AL: "Strategies for targeted gene therapy" TRENDS IN GENETICS, Bd. 12, Nr. 10, 1. Oktober 1996 (1996-10-01), Seite 400-405 XP004037179 ISSN: 0168-9525
- GABIUS H -J ET AL: "THE IMMUNOMODULATORY BETA-GALACTOSIDE-SPECIFIC LECTIN FROM MISTLETOE: PARTIAL SEQUENCE ANALYSIS, CELL AND TISSUE BINDING, AND IMPACT ON INTRACELLULAR BIOSIGNALLING OF MONOCYTIC LEUKEMIA CELLS" ANTICANCER RESEARCH, Bd. 12, Nr. 3, 1. Mai 1992 (1992-05-01), Seiten 669-675, XP002019005
- PAPROCKA M ET AL: "THE ACTIVITY OF TWO IMMUNOTOXINS COMPOSED OF MONOCLONAL ANTIBODY MOAB-16 AND A-CHAIN OF RICIN (MOAB-16-RTA) OR A-CHAIN OF MISTLETOE LECTINI (MOAB-16-MLIA)" ARCHIVUM IMMUNOLOGIAE ET THERAPIAE EXPERIMENTALIS, Bd. 40, Nr. 3/04, 1. Januar 1992 (1992-01-01), Seiten 223-227, XP002019006 ISSN: 0004-069X
- HUGUET SOLER, M. ET AL.: "Complete amino acid sequence of the A chain of mistletoe lectin I." FEBS LETTERS, Bd. 399, 9. Dezember 1996 (1996-12-09), Seiten 153-7, XP002110560
- NAKAMURA, Y.: "Brassica napus [gbpln]: 220 cds's (79094 codons)" CODON USAGE DATABASE, [Online] XP002110561 Retrieved from the Internet: <URL:http://www.dna.affrc.go.jp/nakamura-b in/showcodon.cgi?species=Brassica+napus+[g bpln]> [retrieved on 1999-07-26]
- HUGUET SOLER, M. ET AL.: "Complete amino acid sequence of the B chain of mistletoe lectin I." BIOCHEMICAL AND BIOPHYSICAL RESEARCH COMMUNICATIONS, Bd. 246, Nr. 3, 29. Mai 1998 (1998-05-29), Seiten 596-601, XP002110562

## Beschreibung

Die vorliegende Erfindung betrifft Verfahren zum Herstellen von Mistellektin-Polyeptiden in homologen und heterologen Wirtssystemen sowie Mistellektin-Peptide als solche. Weiter werden Nukleinsäuremoleküle zur Verfügung gestellt, die für diese Mistellektin-Polypeptide kodieren, sowie pharmazeutische Zusammensetzungen, die diese Mistellektin-Polypeptide oder Mistellektin-Nukleinsäuren umfassen.

Die Mistel (Viscum album) ist seit dem Altertum als Heilpflanze bekannt. Die halbstrauchige Pflanze lebt als Halbparasit auf den Ästen von Holzgewächsen und ist vor allem in Europa, Nordaustralien, Asien und im tropischen und subtropischen Afrika weit verbreitet Anfang dieses Jahrhunderts wurde die zyto- und tumortoxische Wirkung des Mistelextraktes erkannt, welches seitdem gezielt zur Krebstherapie eingesetzt wird. Dabei wird das Extrakt sowohl als Einzeltherapeutikum als auch in Kombination mit Chemo- oder Strahlentherapie eingesetzt. Besonders häufig werden Mistelpräparate z.B. nach chirurgischer Tumorentfemung als Rezidivprophylaktikum verwendet.

Systematische Untersuchungen der Wirkweise zeigten, daß wäßriges Mistelextrakt nach Injektion neben seiner zytotoxischen. Wirkung ebenfalls immunmodulierend wirkt und außerdem allgemein stimmungsaufhellende Effekte zeigt Nach Injektion von Mistelextrakt kann eine signifikante Steigerung der Zellzahlen bestimmter Lymphozytensubpopulationen (u.a. T- Helfer-Lymphozyten, natürliche Killerzellen(NK)-Zellen, Makrophagen) und der Phagozytoseaktivität bei Granulo- und Monozyten beobachtet werden, die unmittelbar an der Tumorabwehr beteiligt sind (Hajto, T., Hostanska, K, Gabius, H.-J. (1990), Therapeutikum 4, S. 135-145; Beuth, J., Ko, H.L., Tunggal, L., Gabius, H.-J., Steuer, M., Uhlenbruck, G., Pulverer, G. (1993), Med. Welt 44, S. 217-220; Beuth J., Ko, H.L., Tunggal, L, Geisel, J., Pulverer, G. (1993), Arnzeim.-Forsch./Drug Res. 43 (I), S. 166-169; Beuth, J., Ko, H.L., Gabius, H.-J., Burrichter, H., Oette, Kl., Pulverer, G. (1992), Clin. Investing, 70, S. 658-661). Weiterhin kann eine signifikante Steigerung definierter Akutphaseproteine im Serum, welche durch die Zytokine IL-1, IL-6 und TNF-α vermittelt wird, nachgewiesen werden (Hajto, T., Hostanska, K, Frei, K, Rordorf, C., Gabius, H.-J. (1990), Cancer Res. 50, S. 3322-3326; Beuth, J., Ko, H.-L., Gabius, H.-J., Pulverer, G. (1991), In Vivo 5, 29-32; Beuth, J., Ko, H.-L., Tunggal, L., Jeljaszewicz, J., Steuer, M.K, Pulverer, G. (1994), In Vivo 8, (S. 989-992; Beuth, J., Ko, H.-L., Tunggal, L., Jeljaszewicz, J., Steuer, M.K., Pulverer, G. (1994), Dtsch. Zschr. Onkol. 26, S. 1-6: Beuth, J., Ko, H.-L., Tunggal, L., Steuer, M.K, Geisel, J., Jeljaszewicz, J., Pulverer, G. (1993), In Vivo 7, S. 407-410; Kayser, K., Gabius, S., Gabius, H.-J., Hagemeyer, O. (1992), Tumordiag. und Ther. 13, S. 190-195). Neben der durch Mistelextraktbehandlung erzielbaren Verlängerung der Überlebenszeit von Krebspatienten wird ebenfalls eine Steigerung der Lebensqualität der Patienten beobachtet, welche auf den Anstieg an β-Endorphinen im Blut zurückgeführt wird (Heiny, B.-M., Beuth, J. (1994), Anticancer Res. 14, S. 1339-1342; Heiny, B.-M., Beuth, J. (1994), Dtsch. Zschr. Onkol. 26, S. 103-108). β-Endorphine als körpereigene Opioide verbessern das allgemeine Wohlbefinden, indem sie z.B. schmerzlindernd wirken und den Angstindex verbessern (Falconer, J., Chan, E.C., Madsens, G. (1988), J. Endocrinol. 118, S. 5-8).

Die Analyse der Wirkstoffe des Mistelextraktes hat gezeigt, daß der immunstimulierende Effekt auf eine bestimmte Gruppe von Glykoproteinen, die Mistellektine, zurückzuführen ist. Bisher wurden drei mistelspezifische Lektine mit unterschiedlichen Molekulargewichten und Zuckerbindungspezifitäten identifiziert. Die Konzentration des Mistellektins I (ML-1) im wäßrigen Pflanzenextrakt ist deutlich höher als die von Mistellektin II (ML-II) und Mistellektin III (ML-III). Es konnte gezeigt werden, daß der immunstimulierende Effekt des Mistelextrakts auf die Anwesenheit von ML-I zurückzuführen ist: wird das ML-I-Lektin aus dem Mistelextrakt entfernt, verliert das Extrakt seine immunstimulierende Wirkung (Beuth, J. Stoffel, B., Ko, H.-L., Jeljaszewicz, J., Pulverer, G. (1995), Arzneim.-Forsch./Drug Res. 45(II), S. 1240-1242). Das β-Galactosid-spezifische ML-I-Lektin besteht aus jeweils zwei glykosylierten A- und B-Ketten (MLA bzw. MLB), deren Molekulargewichte bei 29 kDa bzw. 34 kDa liegen. Die Aminosäuresequenz von MLA enthält eine potentielle Glykosylierungsstelle, während MLB drei Glykosylierungsstellen im N-terminalen Bereich der Aminosäuresequenz enthält. Die beiden Ketten sind durch eine Disulfidbrücke miteinander verknüpft (Abbildung A; Ziska, P., Franz, H., Kindt, A. (1978), Experientia 34, S. 123-124). Die resultierenden Mistellektin-Monomere können sich unter Ausbildung von nicht kovalenten Bindungen zu Dimeren zusammenlagem.

Die Untersuchungen des Sedimentationsverhaltens von ML-I während analytischer Zentrifugation zeigen, daß ML-I in vivo in einem Monomer-Dimer-Gleichgewicht vorliegt (Luther, P., Theise, H., Chatterjee, B., Karduck, D., Uhlenbruck, G. (1980), Int. J. Biochem. 11, S. 429-435). Die MLB-Kette ist in der Lage, an galactosehaltige Strukturen auf der Oberfläche von Zellmembranen (z.B. Rezeptormolekülen) zu binden und dadurch Zytokinfreisetzung auszulösen. Durch Endozytose gelangen ML-I-Dimere und -Monomere in die Zelle, wo durch Reduktion der Disulfidbrückenbindungen die Proteinkomplexe in MLA- und MLB-Ketten zerfallen. Die MLA-Ketten sind daraufhin in der Lage, an die ribosomale 28 S-Untereinheit zu binden und diese zu inaktivieren.

Die Untersuchung von ML-I-Monomeren mit Hilfe der 2-D-Gelelektrophorese ergab 25 verschiedene Isoformen, die auf unterschiedliche Kombinationen verschiedener A- und B-Ketten sowie unterschiedliche Glykosylierungszustände der Ketten zurückzuführen sind (Schink et al., 1992, Naturwissenschaften 79, S. 80 - 81). Es wird vermutet, daß die einzelnen Isoformen spezifische Funktionen erfüllen und jede dieser Isoformen zum anti-tumorigenen Effekt des Mistelextraktes beiträgt.

Bisher ist bereits aus der europäischen Patentanmeldung EP 0 751 211 A1 eine Nukleinsäuresequenz und die davon abgeleitete Aminosäuresequenz eines ML-I-Lektins bekannt. Dieses eine Polypeptid ist jedoch nicht in der Lage, die Wirkung der zahlreichen in natürlichem Mistelextrakt enthaltenen ML-I-isoenzyme in bezug auf den anti-tumorigenen und stimmungsaufhellenden Effekt zufriedenstellend nachzuahmen.

Aufgabe der vorliegenden Erfindung ist es daher, ein Verfahren zur Verfügung zu stellen, das es erlaubt, Mistellektine in ausreichenden Mengen herzustellen und gleichzeitig die Diversität an ML-I-Isoenzymen des natürlichen Mistelextraktes nachzuempfinden.

Die Aufgabe wird erfindungsgemäß durch das Bereitstellen eines Verfahrens nach Anspruch 1 und/oder 40 gelöst.

Die vorliegende Erfindung stellt außerdem 2 neue Polypeptide der MLA-Kette und 6 neue Polypeptide der MLB-Kette von ML-I zur Verfügung, die einzeln oder in Kombination in einem geeigneten Wirtssystem exprimiert werden können. Dabei entstehen "homologe" und "heterologe" ML-1-Dimere, wobei der Begriff "homolog" ein Dimer bezeichnet, das aus jeweils zwei gleichen MLA- und MLB-Ketten besteht, und der Begriff "heterolog" ein Dimer beschreibt, das aus zwei verschiedenen MLA- und/oder zwei verschiedenen MLB-Ketten besteht. Die Diversität der MLA- und MLB-Ketten gestattet es, eine Vielzahl von verschiedenen MLA/MLB-Komplexen zu erzeugen, welche in ihrer therapeutischen Wirkung der oben beschriebenen Wirkung des Lektingemisches, das in wäßrigem Mistelextrakt nachgewiesen wurde, nachempfunden ist.

Einer der Vorteile, den die vorliegende Erfindung gegenüber der herkömmlichen Gewinnung von Mistelextrakten aus Frischpflanzen bietet, ist, daß die immunmodulierenden Komponenten des Mistelextraktes durch ein biotechnologisches Verfahren hergestellt werden können. Dies bedeutet, daß unabhängig vom pflanzlichen Rohmaterial, welches nur eingeschränkt zur Verfügung steht und nur zu einer bestimmten Zeit des Jahres geerntet werden kann, ausreichende Mengen an Mistellektin I produziert werden können. Darüberhinaus enthält ein auf diese Weise biotechnologisch hergestelltes Gemisch an Mistellektinen keine der in natürlichem Mistelextrakt auftretenden "Verunreinigungen", wie z.B. Viscotoxine.

Weiter wird es dadurch, daß die vorliegende Erfindung eine Vielzahl von unterschiedlichen MLA- und MLB-Polypeptiden von ML-I zur Verfügung stellt, möglich, zielgerichtet pharmakologische Zusammensetzungen zu "designen". Das bedeutet, daß z.B. durch die Auswahl bestimmter MLB-Polypeptide, die die Bindungsaffinität des MLA/MLB-Komlexes an die Zielzellen definieren, die immunmodulatorische Wirkung einer Zusammensetzung beeinflußt werden kann. Desweiteren kann durch die Verwendung bestimmter MLA-Polypeptide die Zytotoxizität einer Zusammensetzung variiert werden.

Um das in Mistelextrakten enthaltene Gemisch an Mistellektinen biotechnologisch herstellen zu können, wurde zunächst die Aminosäuresequenz eines pharmakologisch interessanten Mistellektins aufgeklärt. Dazu wurde ein Mistelextrakt aus *Viscum album L. ssp. platyspermum* Kell, welche von Pappeln geerntet wurden, gewonnen und Mistellektin I durch Affinitätschromatographie partiell gereinigt (Beispiel 1.). Die anschließende Analyse durch SDS-PAGE, HPLC und Sequenzanalyse durch Edman-Abbau ergab 2 MLA-Isoformen und 6 MLB-Isoformen.

Aus kurzen Bereichen der Aminosäuresequenzen wurden degenerierte Oligonukleotide abgeleitet, mit deren Hilfe die genomische Mistellektin-I-DNA-Sequenz unter Verwendung des PCR-Verfahrens ermittelt wurde. Überraschenderweise konnte trotz der zahlreichen identifizierten ML-I-Aminosäuresequenzen nur eine einzige dieser Sequenzen mehr oder weniger entsprechende Nukleinsäuresequenz identifiziert werden. Durch Southem-Blot-Analyse wurde bestätigt, daß das ML-I-Gen in lediglich einer Kopie pro Genom auftritt. Die Sequenzvariabilität der MLA- und MLB-Polypeptide ist daher lediglich durch das Auftreten von RNA-Editing oder anderen posttranskriptionalen oder posttranslationalen Modifikationen in Mistelzellen zu erklären.

Als "RNA-Editing" werden alle Prozesse bezeichnet, die zu Unterschieden zwischen der endgültigen mRNA-Sequenz und der entsprechenden "Template"- DNA führen, ausgenommen "RNA-Splicing" und tRNA-Modifikationen. Das "mRNA-Splicing" sowie das Auftreten von modifizierten tRNAs ist allgemein bekannt und wird daher hier nicht näher erläutert. Beim "RNA-Editing" werden co- oder posttranskriptional einzelne Nukleotide oder Stränge von bis zu mehreren 100 Nukleotiden Länge ausgetauscht, inseriert oder deletiert, was unter Umständen zu Leserasterveränderungen der kodierten Sequenz führen kann. Das erste Beispiel für RNA-Editing wurde bei Untersuchungen des coxII-Transkripts der mitochondrialen DNA von Trypanosomen entdeckt (Benne R. et al. (1986), Cell, 46, S. 819-826). Weiterhin wurde dieser Prozeß in Mitochondrien und Chloroplasten von höheren Pflanzen sowie singulären nukleären Transkripten in Säugerzellen nachgewiesen. Der genaue Mechanismus des RNA-Editings ebenso wie die Mechanismen für posttranslationale Modifikationen der primären Aminosäuresequenz sind bisher jedoch erst sehr unvollständig in der Literatur beschrieben.

Da dieser Vorgang jedoch bisher nur in sehr wenigen Pflanzen nachgewiesen wurde und eine biotechnologische Herstellung der verschiedenen Mistellektin I-Polypeptide auch in anderen Pflanzenzellen außer Mistelzellen soweit wie möglich unabhängig von posttranskriptionalen oder posttranslationalen Veränderungen ermöglicht werden soll, wurde die genomische DNA durch gezielte Mutationen an die Sequenz der verschiedenen isolierten Polypeptide angepaßt. Weiterhin wurde die genomische Sequenz durch Mutationen an die bevorzugte Kodonbenutzung von *Brassica* angepaßt, um eine optimale Expression in z.B. Rapszellen zu ermöglichen.

Die vorliegende Erfindung stellt daher ein Verfahren zum Herstellen eines Mistellektin-Polypeptides oder eines Fragmentes davon im heterologen System mit der folgenden Sequenz zur Verfügung: umfassend den Schritt des Exprimierens eines eukaryontischen oder prokaryontischen Vektors, in den eine nach dem üblichen genetischen Code für das Mistellektin-Polyeptid oder ein Fragment davon kodierende Nukleinsäure kloniert ist, in einem geeigneten heterologen eukaryontischen oder prokaryontischen Wirt,
wobei X1 D oder E ist; X2 G oder Q ist; X3 I oder V ist; X4 L oder A ist; X5 DR ist oder fehlt; X6 N oder T ist; X7 P oder T ist; X8 D oder E ist; X9 S oder T ist; X10 F oder Y ist; X11 T oder A ist; X12 A oder Y ist; X13 Y oder D ist; X14 A oder E ist; X15 V oder M ist; X16 I oder F ist; X17 P oder S ist; X18 P oder T ist; X19 T oder S ist; X20 D oder S ist; X21 N oder S ist; X22 C oder R ist; X23 G oder N ist; 24 G oder D ist; X25 G oder Q ist; X26 V oder D ist; X27 Q oder K ist; X28 G ist oder fehlt; X29 R oder K ist; X30 C oder S oder V ist; X31 A oder G ist; X32 G oder A ist; X33 S oder G ist; X34 G oder S ist; X35 G oder Y ist; X36 N oder S oder T oder K ist; X37 S oder G ist; X38 L oder P ist; X39 A oder M ist; X40 M oder V ist; X41 P oder F ist.

Analog zu diesem Verfahren werden zwei weitere Herstellungsverfahren für die Mistellektin-A-Kette (MLA) und die Mistellektin-B-Kette (MLB) zur Verfügung gestellt, weiche die folgenden Sequenzen oder ein Fragment davon umfassen:

### Mistellektin-A:

### Mistellektin-B:

wobei X1 bis X41 die oben angegebene Bedeutung haben.

Des weiteren wird ein Mistellektin-Polypeptid oder ein Fragment davon, das die Sequenzvariabilität der verschiedenen MLA- und MLB-Ketten umfaßt, mit der folgenden Sequenz zur Verfügung gestellt:

Außerdem werden Mistellektin-Polypeptide der Mistellektin-A-Kette und Mistellektin-B-Kette oder Fragmente dieser Sequenzen bereitgestellt, die die folgenden Sequenzen umschließen.

### Mistellektin-A:

### Mistellektin-B:

wobei X1 bis X41 die oben angegebene Bedeutung haben.

Die Sequenz, die die oben beschriebene Variabilität der in Mistelzellen auftretenden ML-I-Polypeptide umfaßt, ist in Abbildung 1 b dargestellt Eine spezifische Sequenz für MLA2 des Mistellektins I, welche ebenfalls nach dem oben dargestellten Verfahren hergestellt wurde, ist in Abbildung 3b dargestellt Die Abbildungen 7b bis 12b umfassen spezifische Misteilektin-B-Kettensequenzen, die ebenfalls nach dem oben beschriebenen Verfahren hergestellt wurden.

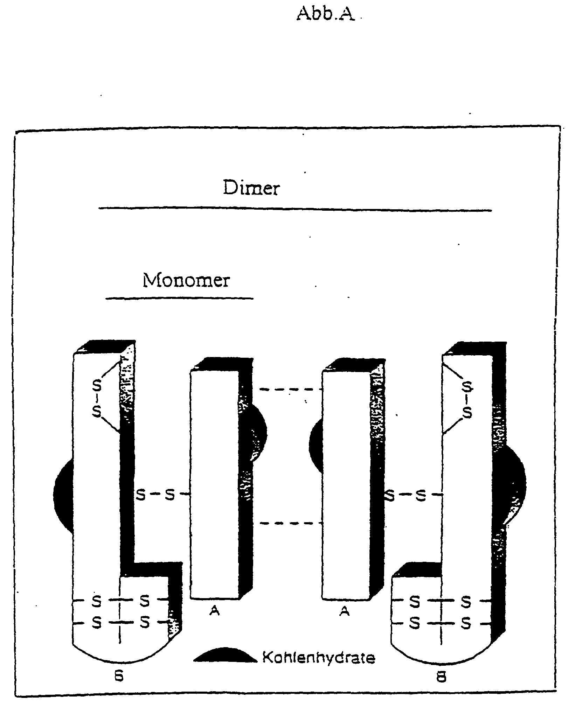

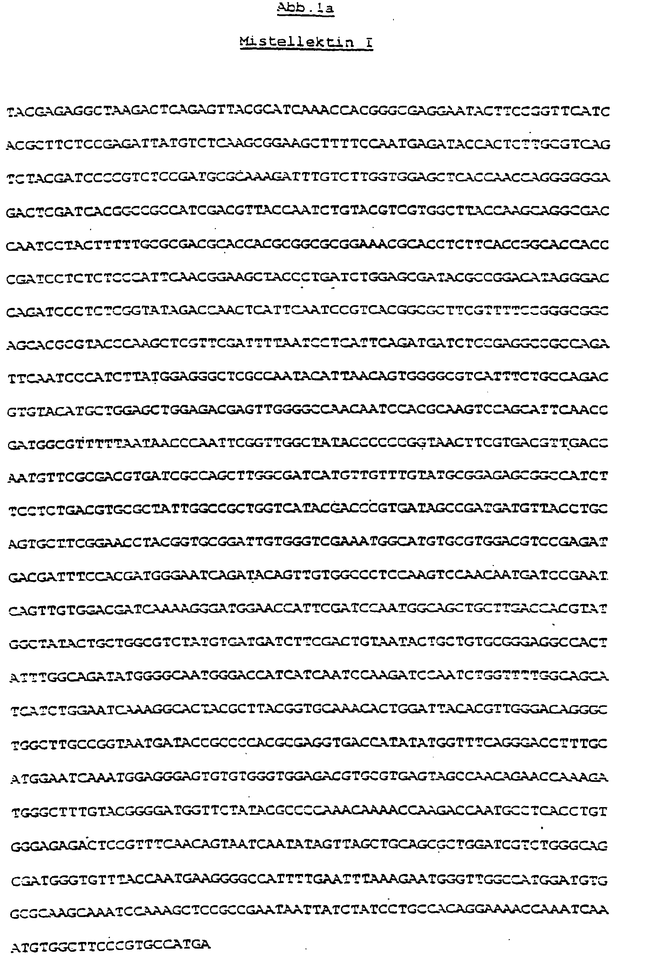

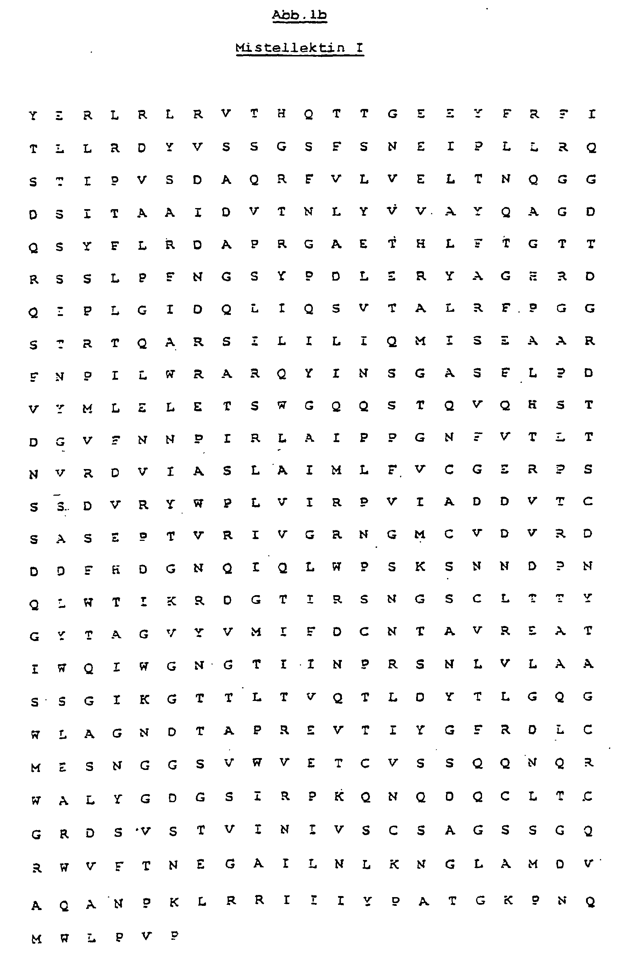

Ein weiterer Aspekt der vorliegenden Erfindung ist ein Verfahren zum Bereitstellen eines Nukleinsäuremoleküls, das in einem heterologen Wirt für ein Mistellektin-Polypeptid wie oben beschrieben kodiert und die folgenden Schritte umfaßt
a) Präparieren von Mistelzell-RNA oder chromosomaler Mistelzell-DNA und
b) Amplifizieren von Mistelzell-RNA oder chromosomaler Mistellektin-DNA durch PCR unter Verwendung von Oligonukleotiden, die von dem in Abb.1b dargestellten Mistellektin-Polypeptid abgeleitet sind, und
c) gegebenenfalls Identifizieren von Sequenzen, die 5' und 3' von der amplifizierten Nukleinsäure liegen sowie deren Amplifikation, und
d) Isolieren der in Schritt b) und/oder c) amplifizierten Nukleinsäuremoleküle, und
e) gegebenenfalls Ligieren mehrerer der in Schritt b) und/oder c) amplifizierten Nukleinsäuremoleküle, so daß ein Nukleinsäuremolekül mit vollständigem offenen Leseraster erhalten wird und
f) gezieltes Mutieren des erhaltenen Nukleinsäuremoleküls um das Nukleinsäuremolekül dem üblichen genetischen Code des heterologen Wirtes für eine der in Mistelzellen identifizierten Mistellektin-Polypeptidisoformen anzupassen.

Zur Präparation von Mistelzell-DNA wurden Mistelpflanzen (*Viscum album L. ssp. platyspermum* Kell), die von Pappeln aus dem Elsaß geerntet wurden, in flüssigem Stickstoff zerstoßen und die chromosomale DNA extrahiert (Beispiel 1). Unter Verwendung der unten dargestellten, degenerierten Oligonukleotide wurden mit Hilfe des PCR-Verfahrens Fragmente der genomischen Mistellektin-DNA amplifiziert (Beispiel 2). Die in der PCR-Reaktion eingesetzten degenerierten Oligonukleotide, die an Bereiche der MLB-Ketten-DNA hybridisieren, haben die Sequenz:

### (BI):

### (BII):

Die Abkürzungen der Nukleotide richten sich dabei nach der von der IUPAC-IUB Biochemischen Nomenklatur-Kommission vorgeschlagenen Bezeichnung.

In einem weiteren Reaktionsschritt wurden unter Verwendung von spezifischen Oligonukleotiden mit Hilfe der RACE-Technik die 5'- und 3'-liegenden Sequenzen des ersten Amplifikationsproduktes ermittelt (Beispiel 3). Das für die 5'-RACE-Reaktion benutzte Oligonukleotid hat die folgende Sequenz:

Das für die 3'-RACE-Reaktion benutzte Oligonukleotid hat die folgende Sequenz:

Die so erhaltene vollständige Nukleinsäuresequenz wurde zur Synthese spezifischer Oligonukleotide benutzt, um mittels PCR einen Gesamtklon zu bekommen. Alternativ wurden die teilweise überlappenden Klone unter Verwendung von geeigneten Restriktionsschnittstellen gespalten, um in einem geeigneten Vektor zusammengesetzt zu werden, so daß ein vollständiges offenes Leseraster des Mistellektin I-Gens erhalten wurde. In diese DNA-Konstrukte können durch bekannte Techniken gezielt Mutationen eingeführt werden, z.B. durch Ersetzen bestimmter DNA-Bereiche durch andere DNA-Fragmente, Einfügen von nicht vollständig homologen Oligonukleotiden, etc. Diese Mutationen können dazu dienen, einerseits die davon abgeleitete Aminosäuresequenz zu verändem und so die Aktivität des Polypeptides zu beeinflussen oder andererseits die Nukleinsäuresequenz zu variieren, ohne die Aminosäuresequenz zu verändern, um z.B. den bevorzugten Kodongebrauch eines Wirtsorganismus nachzuempfinden.

Nukleinsäuremoleküle, die durch dieses Verfahren zur Verfügung gestellt werden und für ein Polypeptid, wie oben beschrieben, kodieren, umfassen die folgenden Sequenzen für ML-1, MLA und MLB oder Fragmente davon:

### I) ML-I-Sequenz:

### II) MLA-Sequenz:

### III) MLB-Sequenz:

Die Nukleotide sind gemäß dem IUPAC-IUB Code definiert; Z₁ bezeichnet die Nukleotidsequenz GAT AGA oder fehlt, während Z₂ die Nukleotidsequenz GGC bezeichnet oder fehlt.

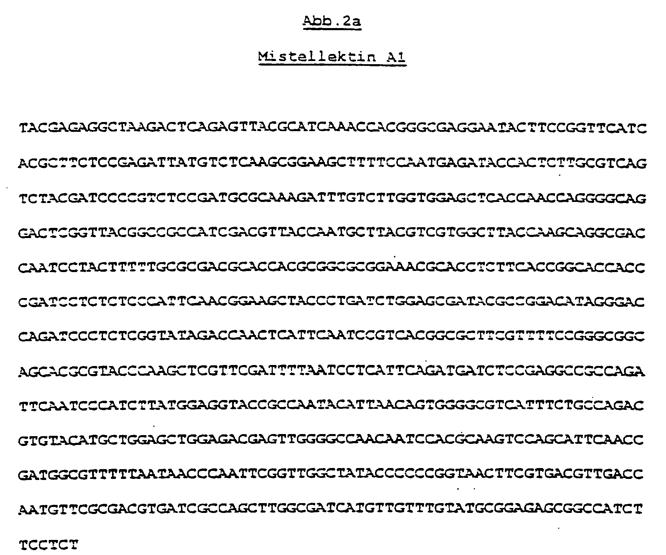

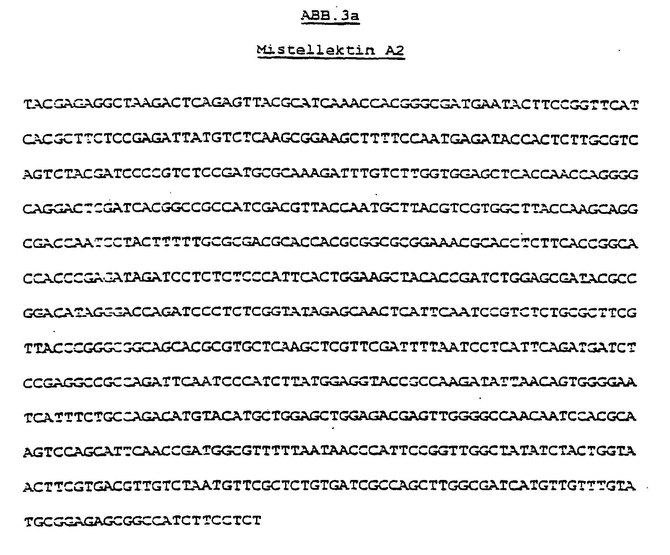

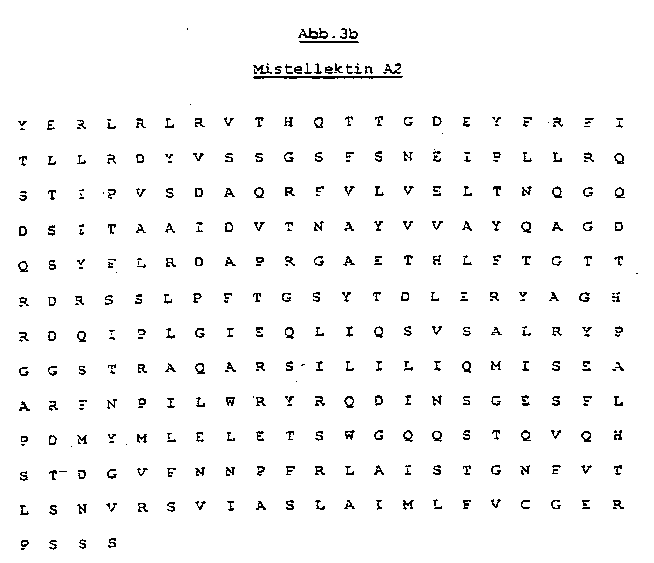

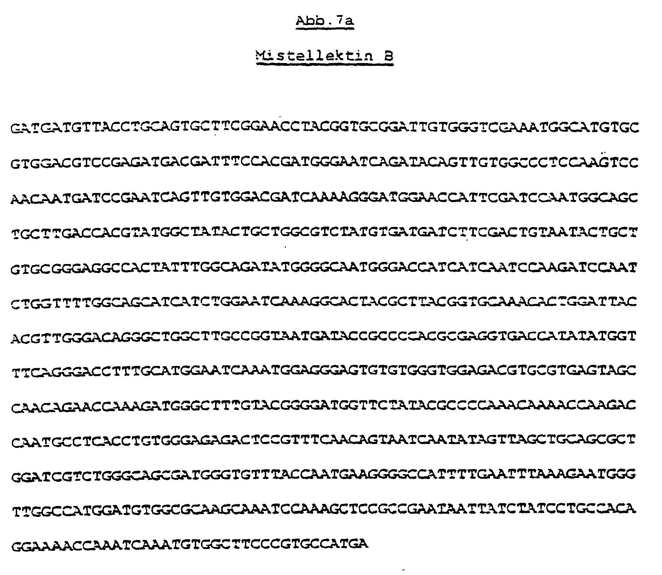

Ein spezifisches Nukleinsäuremolekül, das nach dem oben angegebenen Verfahren dargestellt wurde und die gesamte ML-1 kodierende Sequenz umfaßt, ist in der Abbildung 1a gezeigt. Weitere spezifische Nukleinsäuremoleküle, die für die MLA-Kette von Mistellektin I kodieren und nach dem oben angegebenen Verfahren dargestellt wurden, werden in Abbildung 2a und Abbildung 3a gezeigt. In Abbildungen 7a bis 12a sind spezifische Sequenzen für MLB-Nukleinsäuremoleküle, die nach dem oben beschriebenen Verfahren dargestellt wurden, aufgeführt. Dabei kodiert jede dieser Nukleinsäuresequenzen für ein Polypeptid, das durch Proteinsequenzierung des ML-1-Gernisches aus natürlichem Mistelextrakt hervorgegangen ist.

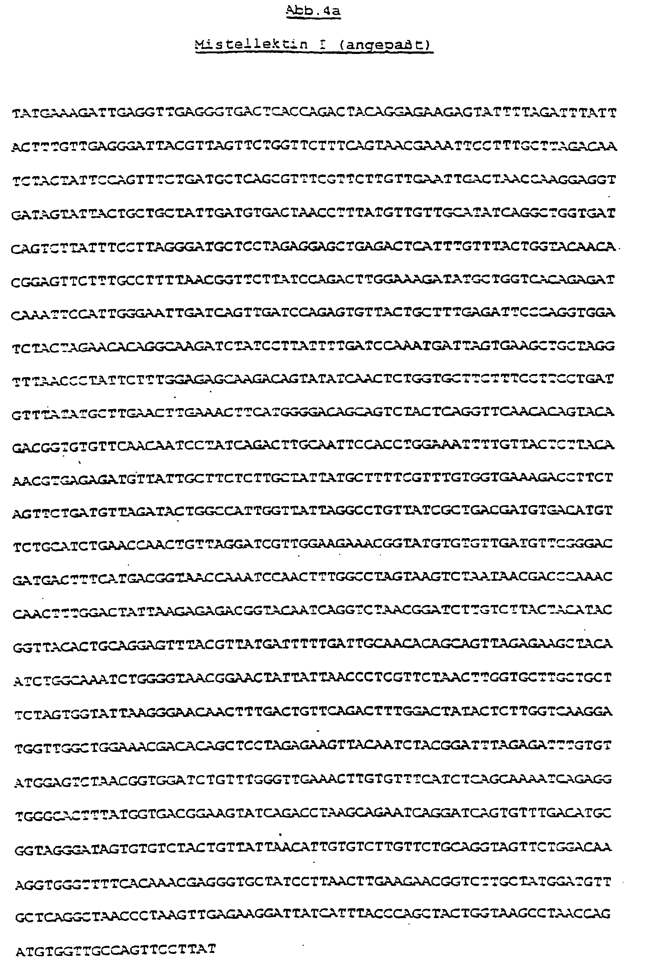

Die vorliegende Erfindung umfaßt außerdem Nukleinsäuremoleküle, die für ein Mistellektin-Polypeptid, wie oben beschrieben, kodieren und dadurch gekennzeichnet sind, daß der Kodongebrauch an die Bedürfnisse eines heterologen Wirts angepaßt ist. In Abbildung 4a ist eine solche Nukleinsäuresequenz dargestellt wobei der Kodongebrauch an die bevorzugte Kodonverwendung der Gattung Brassica angepaßt ist. Diese Gattung wurde ausgewählt, da sie sowohl als Sommer- als auch als Winterform in den mittleren Breiten Europas, Nordamerikas und Asiens hervorragend gedeiht. Die Einsatzmöglichkeiten für Raps zur Produktion von rekombinanten Proteinen konnte von verschiedenen Firmen und Forschungsinstituten gezeigt werden. Anwendungsbeispiele sind die Produktion von gastrischer Lipase zum Einsatz in der Mukoviszidose-Behandlung oder die Kopplung an Oleosine zur erleichterten Aufreinigung der rekombinanten Proteine aus der Lipidphase der Rapsölsamen.

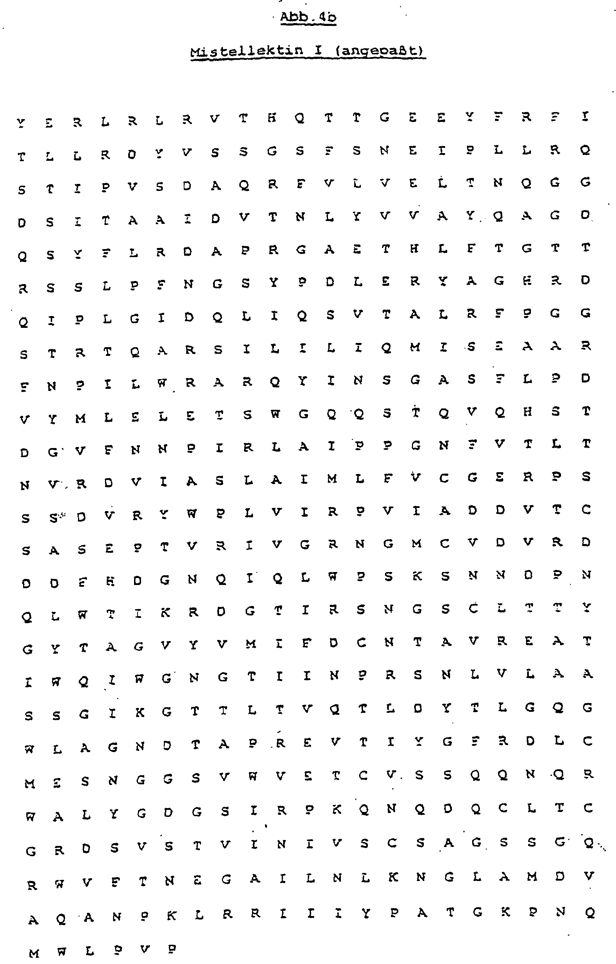

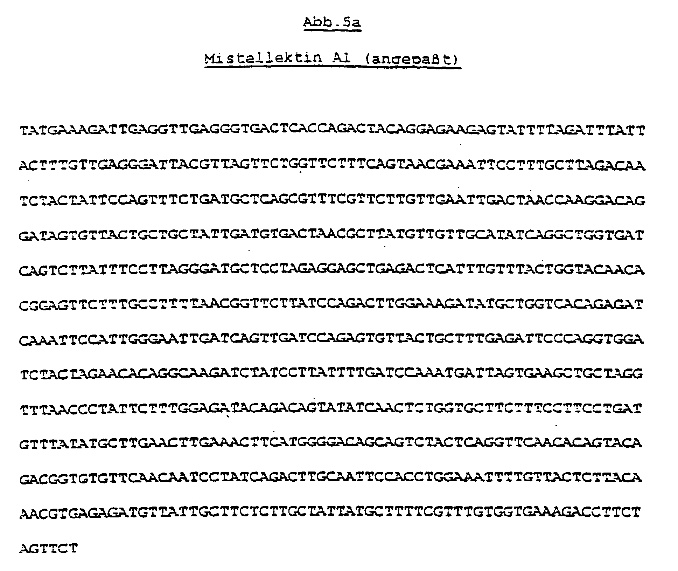

Die in Abbildungen 5a, 6a und 13a bis 18a dargestellten Sequenzen repräsentieren Nukleinsäuremoleküle, die für MLA-Polypeptide bzw. für MLB-Polypeptide des Mistellektins I kodieren und ebenfalls in ihrer Kodonverwendung an die Gattung *Brassica* angepaßt sind. Der Homologiegrad zwischen diesen angepaßten Sequenzen zu den in Abb. 2a und 7a dargestellten Nukleinsäuresequenzen beträgt für MLA ca. 61 % und für MLB ungefähr 63%.

Durch die vorliegende Erfindung wird weiterhin ein Vektor zur Verfügung gestellt, der eines der oben beschriebenen Nukleinsäuremoleküle oder ein Fragment davon sowie einen die Expression dieses Nukleinsäuremoleküls regulierenden Promotor umfaßt. In einer bevorzugten Ausführungsform enthält dieser Vektor in funktioneller Verbindung mit den oben beschriebenen Nukleinsäuremolekülen einen Promotor, der nur in der vorgesehenen Wirtszelle aktiviert werden kann. Die Wirtszelle kann dabei eine pflanzliche oder eine tierische Zelle sein. Wirtszellspezifische Promotoren werden bereits, teilweise zusammen mit zelltypspezifischen, regulierbaren Enhancersequenzen, zur selektiven Expression von therapeutischen Genen eingesetzt (Walter W. und Stein U., Molecular Biotechnology, 1996, 6 (3), S. 267-86). Es wurden ebenfalls Systeme entwickelt, in denen Induktoren und Repressoren auf einen gentechnisch modifizierten Transkriptionsfaktor wirken, der spezifisch einen ebenfalls modifizierten Promotor erkennt. Dies erlaubt die regulierte Expression von z.B. therapeutischen Proteinen, ohne dabei zelluläre Promotoren unspezifisch zu aktivieren (Miller N. und Whelan J., Human Gene Therapy. 1997, 8(7), S. 803-815).

Ein bevorzugter Vektor ist ein RNA-Vektor, wie zB. in Kumagai et al. Proc. Natl. Acad. Sci., USA, 1993, 90, S. 427-430, beschrieben. Dieses System bietet im Vergleich zu anderen Pflanzenexpressionssystemen die Vorteile, daß erstens hohe Ausbeuten an rekombinanten Proteinen erzielt werden können und zweitens eine wesentlich schnellere Etablierung des Verfahrens stattfindet, da nur der RNA-Vektor genetisch verändert wird und die Pflanze nach Infektion die Produktion des rekombinanten Proteins aufnimmt.

Wirtssysteme, die zur heterologen Expression der oben beschriebenen Nukleinsäuren dienen sollen, können aus der Bakterienzellen, Pflanzenzellen mit der Ausnahme von Mistelzellen; Insektenzellen; Insektenlarven; Vertebratenzellen, bevorzugt Säugerzellen; Hefezellen; Pilzzellen; transgene Vertebraten mit Ausnahme des Menschen und/oder transgene Pflanzen mit der Ausnahme von Mistelpflanzen umfassenden Gruppe ausgewählt sein. Dabei werden bevorzugterweise *Escherichia* coli als Bakterienzellen, Rapszellen als Pflanzenzellen, Trichoplusia ni als Insektenlarven, *Spodoptera frugiperda*-Zellen als Insektenzellen und Zebrafische als Vertebraten verwendet.

Die vorliegende Erfindung umfaßt pharmazeutische Zusammensetzungen, die mindestens eines der oben erwähnten Nukleinsäuremoleküle oder einen der oben beschriebenen Vektoren enthalten.

Eine bevorzugte pharmazeutische Zusammensetzung enthält zusätzlich liposomen, die die linearen Nukleinsäuremoleküle bzw. die Vektoren einschließen, um sie gegen nukleolytischen Abbau zu schützen. Dabei können diese Liposomen auf ihrer Oberfläche Zellerkennungsmoleküle tragen, die eine selektive Anlagerung an spezifische Zielzellen ermöglichen. Solche obernachenmodifizierten Liposomen der sogenannten "zweiten Generation" (z.B. Immunliposomen und "long-circulating liposomes") werden bereits zur gezielten Transfektion spezifischer Zelltypen von Krebspatienten erfolgreich eingesetzt (Storm G. und Crommelin D.J., Hybridoma, 1997, 16 (1), S. 119-125; Thierry A.R. et al., Gene Therapy, 1997,4 (3), S. 226-237).

Eine weitere pharmazeutische Zusammensetzung ist vorgesehen, bei der das lineare Nukleinsäuremolekül bzw. der Vektor direkt oder über ein Linkersystem (z.B. Biotin-Streptavidin-Kopplung) an eines der oben beschriebenen MLB-Polypeptide gekoppelt wird. Die MLB-Polypeptideinheit vermittelt dabei die Anlagerung des Komplexes an zukkerhaltige Strukturen auf der Zellmembran und induziert die endozytotische Aufnahme des Komplexes. Auf diese Weise kann z.B. eine für das zytotoxische MLA kodierende Nukleinsäure gerichtet in eine Zelle transportiert werden, wo sie anschließend in ein Protein translatiert wird und dann die zelleigenen Ribosomen inaktiviert. Zusätzlich kann ein solcher Komplex Peptide wie z.B. Antikörper, Antikörperfragmente oder rezeptorbindende Peptide (Liganden) enthalten, die in der Lage sind, zellspezifische Bindungen durchzuführen.

Eine weitere bevorzugte pharmakologische Zusammensetzung umfaßt neben dem linearen Nukleinsäuremolekül oder dem Vektor einen Viruspartikel. In diesem Fall ist ein Virus-Vektor bevorzugt Der Viruspartikel kann dabei auf seiner Oberfläche ebenfalls Zellerkennungsmoleküle zur spezifischen Zellerkennung tragen. Diese Moleküle können z.B. Fusionsproteine von viralen Proteinen mit zellspezifisch bindenden Polypeptiden sein. Durch Präsentation dieser Peptide auf der Oberfläche des Viruspartikels kann eine gezielte Anlagerung dieser Partikel erreicht werden (Joelson T. et al., Journal of General Virology, 1997, 78 (6), S. 1213-1217; Grabherr R. et al., Biotechnics, 1997, 22 (4), S. 730-735).

Die vorliegende Erfindung umfaßt weiterhin eine pharmazeutische Zusammensetzung, die mindestens eines der oben beschriebenen Mistelleklin-Polypeptide und/oder mindestens ein Fragment derselben als zytotoxische Komponente enthält. Die pharmazeutische Effizienz einer solchen Zusammensetzung kann wiederum durch Kopplung der polypeptide oder Polypeptid-Fragmente mit Zetterkennungsmolekülen, die setektiv an Zielzellen binden, erhöht werden. In einer bevorzugten Ausführungsform der pharmazeutischen Zusammensetzung ist das Zellerkennungsmolekül ein Antikörpermolekül, ein Antikörper-Fragment oder jedes andere Protein- und Peptidmolekül, welches die Fähigkeit besitzt, spezifisch an die Zielzellen zu binden, zB. ein Peptidhormon oder ein Fragment dieses Hormons wie das "gonadotropin-releasing hormone" und solche Fragmente, die spezifisch an Rezeptoren von Adenokarzinomzellen binden oder Peptide, die bei einer speziellen Leukämie-Form an den Interleukin-2-Wachstumsfaktor Rezeptor der Lymhomzellen ("cutaneous T cell lymphoma") binden. Auch Nicht-Protein-Moleküle, die sich in Zielzetten anreichem bzw. an sie binden, wie cis-Platin oder Häm und dessen Vorstufen, können geeignete Zelterkennungsmoleküle für die Kopplung an die zytotoxische Komponente des ML-I sein. Dadurch, daß die zytotoxische Komponente gezielt ins Zellinnere der entarteten Zellen gelangt, können die Dosis an Toxin relativ gering gehalten werden und Nebenwirkungen auf gesundes Gewebe minimiert werden.

Dabei können diese Zielerkennungsmoleküle durch bekannte chemische Verfahren an die Mistellektin-Palypeptide gekoppelt werden. Des weiteren ist es möglich, Fusionsproteine aus oben beschriebenen Polypeptiden und einem geeigneten Antikörper bzw. einem Fragment davon in einem der ebenfalls oben beschriebenen Wirtssysteme zu erzeugen. Als Fusionsproteine eignen sich z.B. auch rekombinante Proteine, die aus einem hier beschriebenen Polypeptid und einer IL-2 Rezeptor-bindenden "homing"-Komponente oder einem gentechnisch modifizierten Fragment des Gonadotropin-Releasing-Faktors bestehen.

Eine erfindungsgemäße pharmazeutische Zusammensetzung umfaßt mindestens eines der oben beschriebenen Polypeptide und/oder ein Fragment davon; in der Regel zusammen mit einem pharmazeutisch verträglichen Träger. Dabei läßt sich den Bedürfnissen des Patienten entsprechend eine bestimmte Mischung verschiedener MLA-und/oder MLB-Polypeptide zusammenstellen. Um die Diversität der Mistellektin-I-Isoenzyme des natürlichen Mistelextraktes nachzuempfinden, enthält eine zytotoxische Zusammensetzung bevorzugt mehrere bzw. alle der oben angegebenen MLA/MLB-Polypeptide. Der pharmazeutisch verträgliche Träger kann ein Puffer, ein Verdünnungsmittel, ein Füllstoff, Lösungsmittel, Gleitmittel, Geschmacksstoff, Bindungsmittel, Konservierungsmittel und/oder einschließendes Material sein. Die pharmazeutische Zusammensetzung wird so formuliert, daß sie sowohl zur oralen als auch parenteralen Verabreichung, insbesondere der subkutanen, intramuskulären und intravenösen Verabreichung geeignet ist. Bei bestimmten Erkrankungen können auch inhalative, rektale, vaginale und kutane Darreichungsformen zur Anwendung kommen.

Ein oben erwähntes erfindungsgemäßes Mistellektin-Polypeptid oder ein Fragment davon kann aufgrund einer anti-tumorigenen Wirkung zur Herstellung eines Medikamentes zur Behandlung von unkontrolliertem Zellwachstum, z.B. von Krebs eingesetzt werden. Des weiteren kann ein solches Mistellektin-Polypeptid oder ein Fragment davon, dessen zytotoxische Aktivität, z.B. durch Veränderungen am aktiven Zentrum (Aminosäuren Y₇₆, Y₁₁₅, E₁₆₅, R_{168,} W₁₉₉) blockiert wurde, in Verbindung mit mindestens einem weiteren Antigen zur Herstellung eines Medikamentes dienen, welches die Immunreaktion gegen das weitere Antigen zu stärken vermag. Aus z.B. dem europäischen Patent 0 320 528 sind bereits Proteine bekannt (Hämocyanine und Arylphorine), die eine stark antigene Reaktion hervorrufen können. Ähnlich wie diese Substanzen können auch die erfindungsgemäßen Mistellektine eine Aktivierung von T-lymphozyten und Lymphokinproduzierenden Makrophagen auslösen und dadurch die körpereigene Abwehr stärken.

Weiterhin umfaßt die vorliegende Erfindung ein Verfahren zum Herstellen eines Mistellektin-Polypeptides in Mistelzellen und/oder einer transgenen Mistelpflanze mit der folgenden Sequenz: umfassend den Schritt des Exprimierens eines eukaryontischen Vektors, der eine für das Mistellektin-Polypeptid oder ein Fragment davon kodierende Nukleinsäure mit der ursprünglich in Mistelzell-DNA gefundenen Nukleinsäuresequenz enthält, in einer Mistelzelle oder einer transgenen Mistelpflanze, wobei das Transkriptionsprodukt dieses Nukleinsäuremoleküls in Mistelzellen oder transgenen Mistelpflanzen durch RNA-Editing und weitere normalerweise vorkommende posttranskriptionale und/oder posttranslationale Mechanismen modifiziert wird und damit gegebenenfalls zur Herstellung des natürlichen Mistellekingemisches führt,
wobei X1 D oder E ist; X2 G oder Q ist; X3 I oder V ist; X4 L oder A ist; X5 DR ist oder fehlt; X6 N oder T ist; X7 P oder T ist; X8 D oder E ist; 9 S oder T ist; X10 F oder Y ist; X11 T oder A ist; X12 A oder Y ist; X13 Y oder D ist; X14 A oder E ist; X15 V oder M ist X16 I oder F ist; X17 P oder S ist; X18 P oder T ist; X19 T oder S ist; X20 D oder S ist; X21 N oder S ist; X22 C oder R ist; X23 G oder N ist; X24 G oder D ist; X25 G oder Q ist; X26 V oder D ist; X27 Q oder K ist; X28 G ist oder fehlt; X29 R oder K ist: X30 C oder S oder V ist; X31 A oder G ist; X32 G oder A ist; X33 S oder G ist; X34 G oder S ist; X35 G oder Y ist; X36 N oder S oder T oder K ist; X37 S oder G ist; X38 L oder P ist; X39 A oder M ist; X40 M oder V ist; X41 P oder F ist.

In Anlehnung an das oben beschriebene Verfahren werden zwei weitere Verfahren zum Herstellen der Mistelleklin-A-Kette und der Mistellektin-B-Kette oder einem Fragment davon bereitgestelllt, welche die folgenden Sequenzen oder ein Fragment davon umfassen:

### Mistellektin-A:

### Mistellektin-B:

Ein erfindungsgemäßes Verfahren zum Bereitstellen eines Nukleinsäuremoleküls, das in einer Mistelzelle oder einer transgenen Mistelpflanze für die oben erwähnten Mistellektin-Polypeptide kodiert, umfaßt die folgenden Schritte:
a) Präparieren von Mistelzell-RNA oder chromosomaler Mistelzell-DNA und
b) Amplifizieren von Mistelzell-RNA oder chromosomaler Mistellektin-DNA durch PCR unter Verwendung von Oligonukleotiden, die von der in Abb.1 b dargestellten Mistellektin abgeleitet sind, und
c) gegebenenfalls Identifizieren von Sequenzen, die 5' und 3' von der amplifizierten Nukleinsäure liegen sowie deren Amplifikation, und
d) Isolieren der in Schritt b) und/oder c) isolierten Nukleinsäuremoleküle, und
e) gegebenenfalls Ligieren mehrerer der in Schritt b) und/oder c) isolierten Nukleinsäuremoleküle, so daß ein Nukleinsäuremolekül mit vollständigem offenen Leseraster erhalten, und
f) gegebenenfalls gezieltes Mutieren des erhaltenen Nukleinsäuremoleküls, um das Nukleinsäuremolekül dem üblichen genetischen Code für eine der in Mistetzellen identifizierten Mistellektin-Polypeptidisoformen anzupassen und/oder die Expression zu optimieren.

Zuerst wird pflanzliche RNA oder DNA vorzugsweise aus Frischmaterial durch verschiedene, allgemein bekannte Verfahren isoliert (Quiagen Versuchsprotokoll, Nickrent, D.L. et. al., American Journal of Botany, v.81, n.9, (1994): 1149-1160; Beispiel 1). Unter Verwendung der im Beispiel 1 beschriebenen degenerierten Oligonukleotide BI und BII, die von dem in Abbildung 1b dargestellten Mistellektin-Polypeptid abgeleitet sind, wird das Mistellektin-I-Gen in einer PCR-Reaktion, deren Bedingungen in Beispiel 2 aufgeführt sind, amplifiziert. Falls dieser Amplifikationsschritt nicht das vollständige offene Leseraster von ML-I umfaßt, kann unter Verwendung der RACE-Technik mit den in Beispiel 3 angegebenen entsprechenden Oligonukleotiden der 5'- und 3'-Bereich der amplifizierten Nukleinsäuren identfiziert werden. Die so gewonnenen Nukleinsäuremoleküle werden isoliert und gegebenenfalls unter Verwendung geeigneter Restriktionsschnittstellen so in einen Vektor ligiert, daß dieser das vollständige offene Leseraster enthält Ein in diesem Vektor enthaltenenes Nukleinsäuremolekül oder ein Fragment davon, das in einer Mistelzelle oder einer transgenen Mistelpflanze für ein wie oben beschriebenes Polypeptid kodiert, umfaßt die folgende Sequenz:

### I) ML-I-Sequenz:

Ein erfindungsgemäßes Nukleinsäuremolekül oder ein Fragment davon, das in einer Mistelzelle oder einer transgenen Mistelpflanze für eine der oben erwähnten MLA-Polypeptide kodiert, umfaßt die folgende Sequenz:

### II) MLA-Sequenz

Des weiteren wird ein Nukleinsäuremolekül oder ein Fragment davon, das in einer Mistelzelle oder in einer transgenen Mistelpflanze für eines der oben erwähnten MLB-polypeptide kodiert, mit der folgenden Sequenz zur Verfügung gestellt:

### III) MLB-Sequenz:

Die Nukleotide sind gemäß dem IUPAC-IUB Code definiert Z₁ bezeichnet zusätzlich die Nukleotidsequenz GAT AGA oder fehlt, während Z₂ die Nukleotidsequenz GGC bezeichnet oder fehlt

Ein spezifisches Nukleinsäuremolekül, das in einer Mistellzelle oder in einer transgenen Mistelpflanze exprimiert werden soll und für ML-1 kodiert, ist in Abbildung 1a dargestellt Desweiteren sind spezifische Nukleinsäuremoleküle zur Expression in Mistelzellen oder einer transgenen Mistelpflanze, die für MLA-Polypeptide kodieren, in Abbildungen 2a und 3a dargestellt; in Abbildungen 7a bis 12a werden Nukleinsäuresequenzen gezeigt, die für MLB-Polypeptide, wie oben beschrieben, kodieren und ebenfalls zur Expression in Mistelzellen und/oder transgenen Mistelpflanzen vorgesehen sind. Die Expression dieser rekombinanten Nukleinsäuren, die die im natürlichen Mistelextrakt vorgefundenen posttranskriptionalen Änderungen der Polypeptidketten bereits in ihrer DNA-Sequenz kodiert haben, hat gegenüber der Expression der natürlichen ML-I-DNA den Vorteil, daß erstens eine größere Menge an rekombinantem Protein erzeugt wird, da der teilweise limitierende Prozeß der posttranskriptionalen Modifikation entfällt und zweitens die Zusammensetzung eines Mistellektin I-Gemisches wesentlich besser kontrolliert werden kann. Es ist ebenfalls beabsichtigt, Misteltektin-Nukleinsäuremoleküle in Mistelzellen und/oder transgenen Mistelpflanzen zu exprimieren, die in ihrer Kodonverwendung so modifiziert sind, daß dadurch die Expressionsrate optimiert wird.

Des weiteren stellt die vorliegende Erfindung ein Verfahren zum Herstellen eines der oben beschriebenen Polypeptide zur Verfügung, welches das Modifizieren von Zuckerseitenketten durch enzymatische und/oder chemische Ergänzung, Entfernung und/oder Änderung einer oder mehrerer Seitenketten umfaßt (Macindoe W. M. et al., Carbohydrate Research, 1995, 269 (2):227-57; Meynial-Salles I. & Combes D., J. Biotechnol., 1996, 46(1), 1-14; Wong S.Y., Current Opinion in Structural Biology, 1995, 5(5), 599-604). Dadurch kann die in vivo-Aktivität einzelner MLA- und/oder MLB-Ketten verstärkt oder abgeschwächt werden bzw. bei eventuellen Abweichungen in Abhängigkeit vom Expressionssystem den natürlichen Mistellektinen optimal nachempfunden werden. Es ist ebenfalls vorgesehen, derartig modifizierte Mistellektine einer erfindungsgemäßen pharmazeutischen Zusammensetzung zuzusetzen.

Die folgenden Abbildungen und Beispiele erläutem die Erfindung:
- Abb. A:: Darstellung eines Mistellektin-I-Dimers.
- Abb. 1:: Darstellung der (a) Nukleinsäuresequenz und (b) Aminosäuresequenz von ML I.

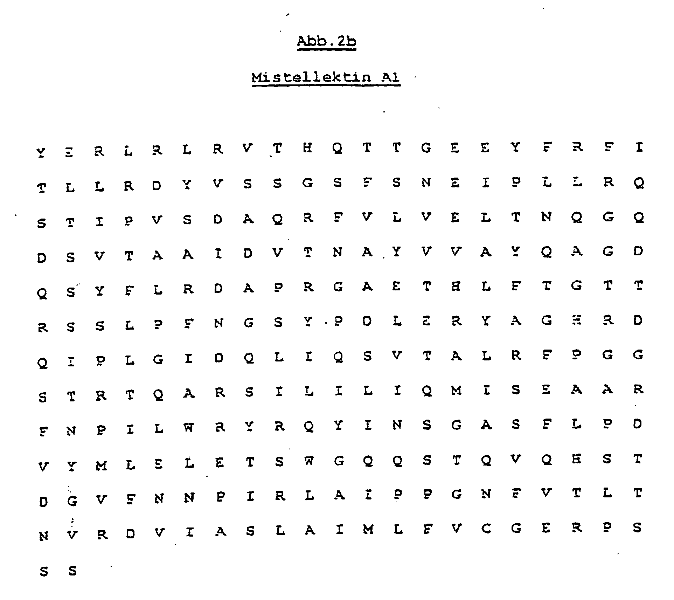

- Abb. 2:: Darstellung der (a) Nukleinsäuresequenz und (b) Aminosäuresequenz von Mistellektin A1.
- Abb. 3:: Darstellung der (a) Nukleinsäuresequenz und (b) Aminosäuresequenz von Mistellektin A2.
- Abb.4:: Darstellung der Nukleinsäuresequenz von ML I, wobei die Nükleinsäuresequenz dem Kodongebrauch von *Brassica* angepaßt ist.

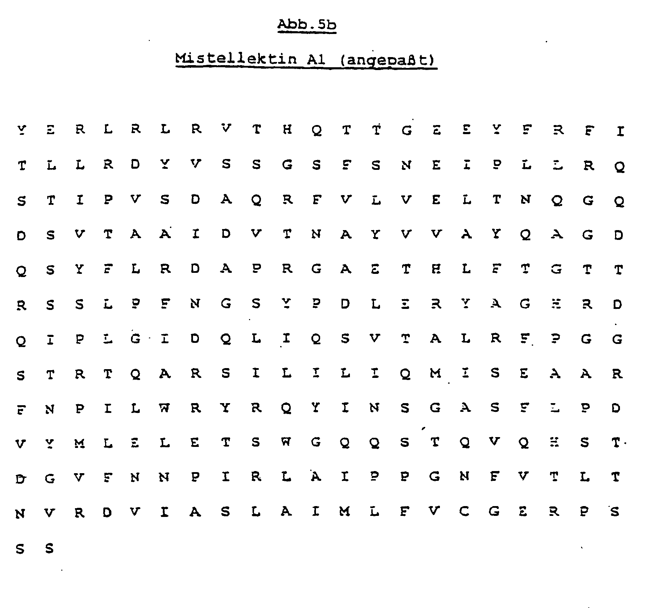

- Abb.5:: Darstellung der Nukleinsäuresequenz von Mistellektin A1, wobei die Nukleinsäuresequenz dem Kodongebrauch von *Brassica* angepaßt ist

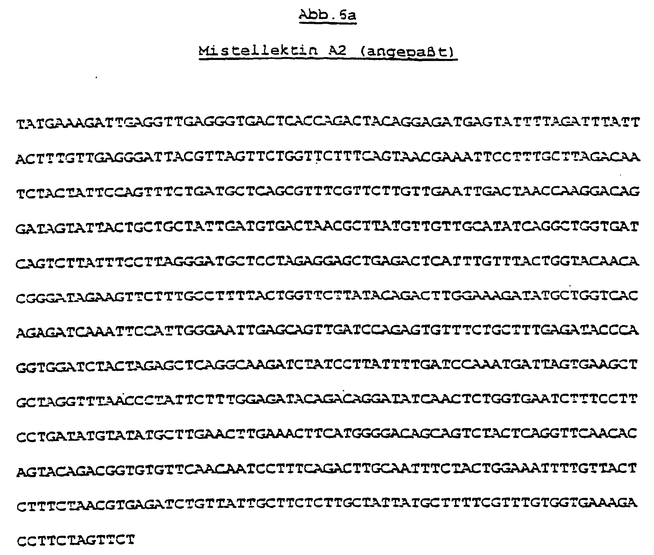

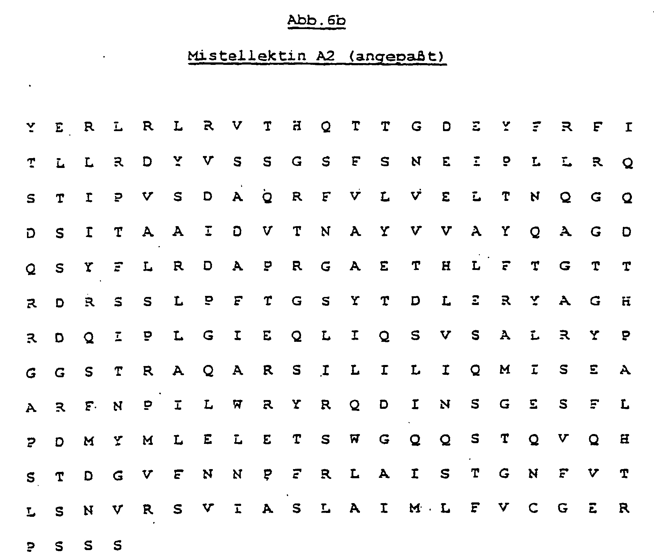

- Abb.6:: Darstellung der Nukleinsauresequenz von Mistellektion A2, wobei die Nukleinsäuresequenz dem Kodongebrauch von *Brassica* angepaßt ist.

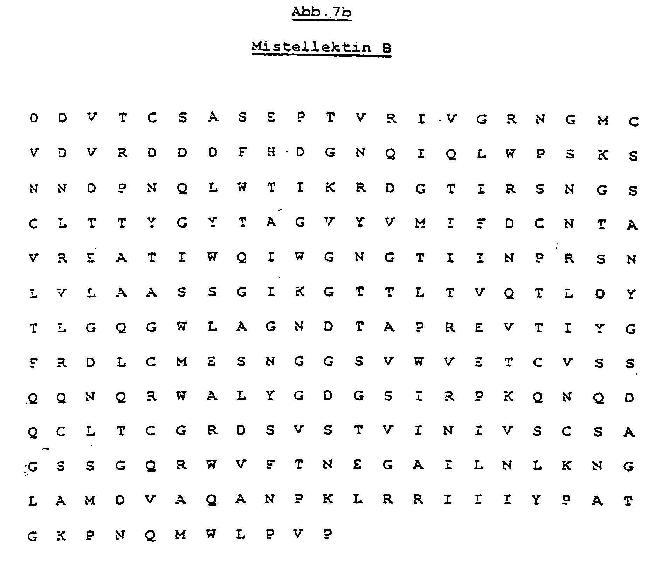

- Abb.7:: Darstellung der (a) Nukleinsäuresequenz und (b) Aminosäuresequenz von Mistellektin B.

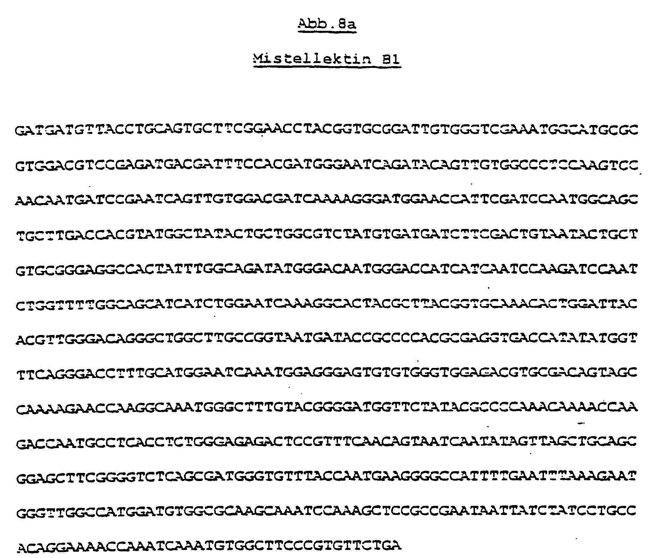

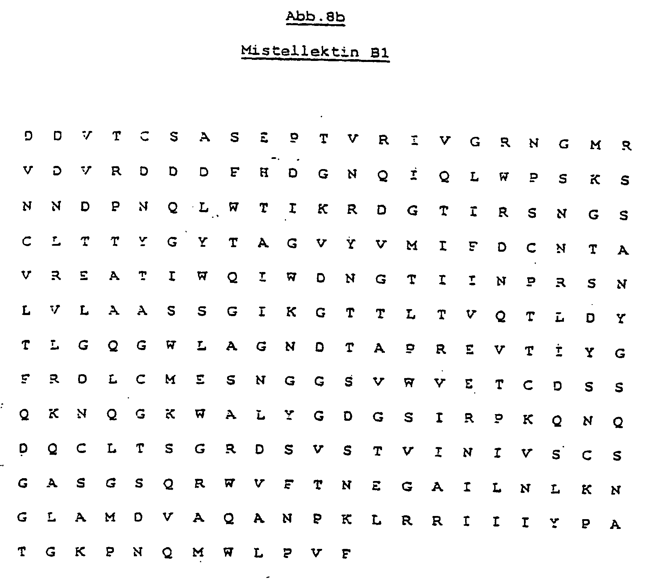

- Abb.8:: Darstellung der (a) Nukleinsäuresequenz und (b) Aminosäuresequenz von Mistellektin B1.

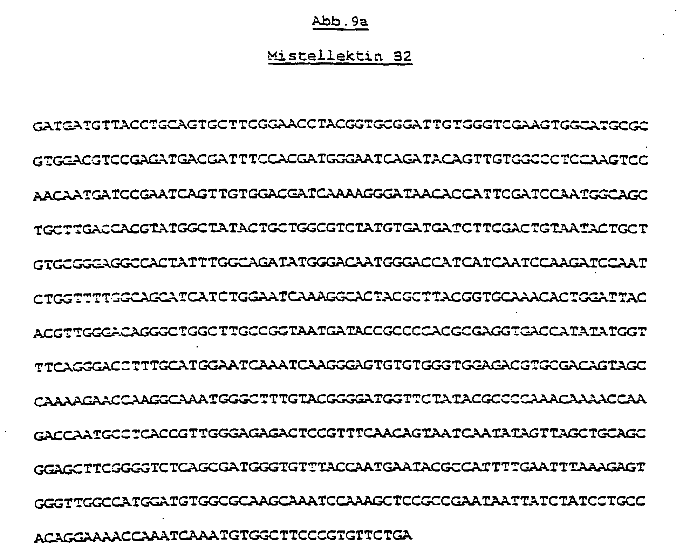

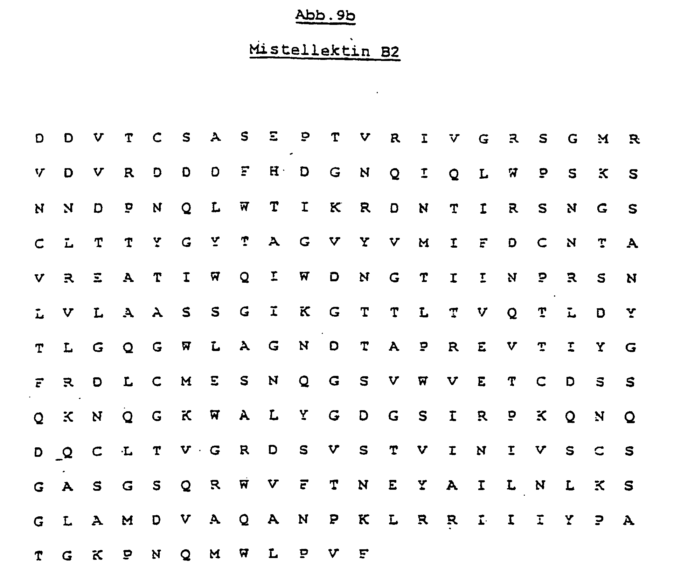

- Abb.9:: Darstellung der (a) Nukleinsäuresequenz und (b) Aminosäuresequenz von Mistellektin B2.

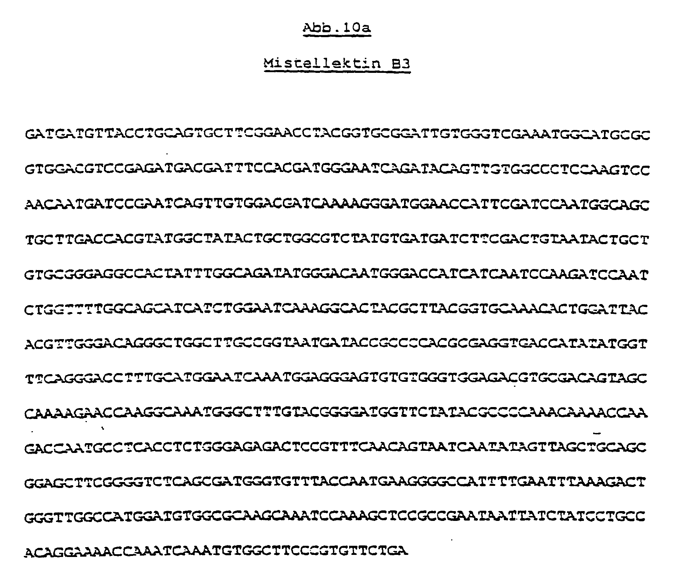

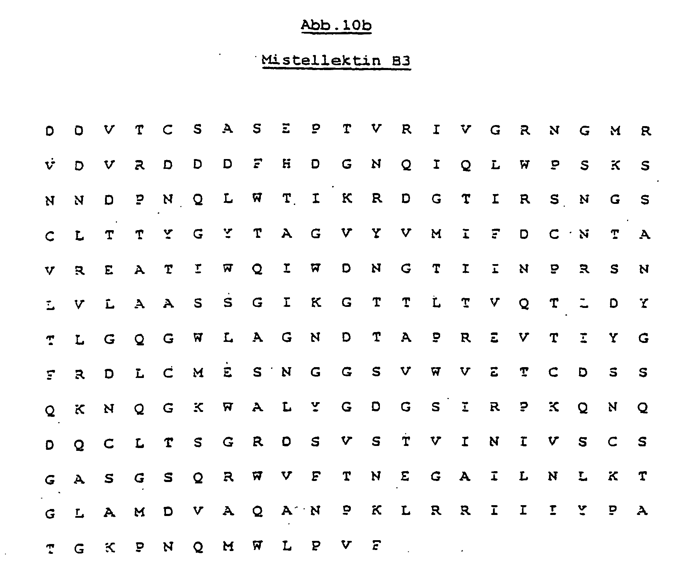

- Abb. 10:: Darstellung der (a) Nukleinsäuresequenz und (b) Aminosäuresequenz von Mistellektin B3.

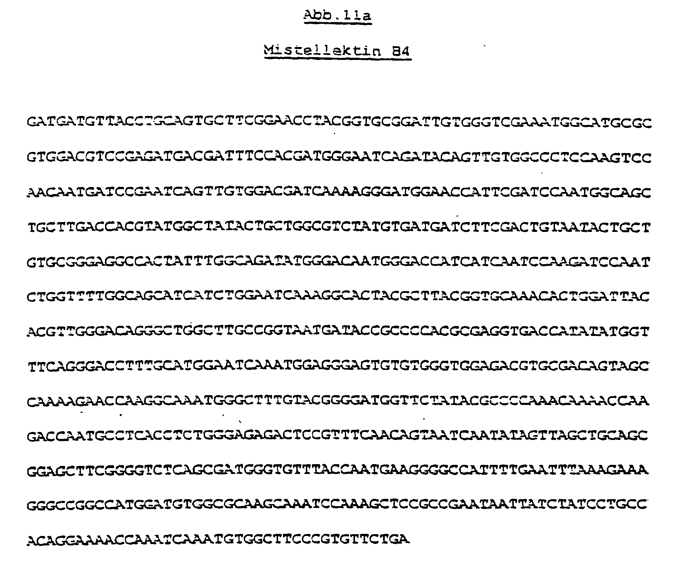

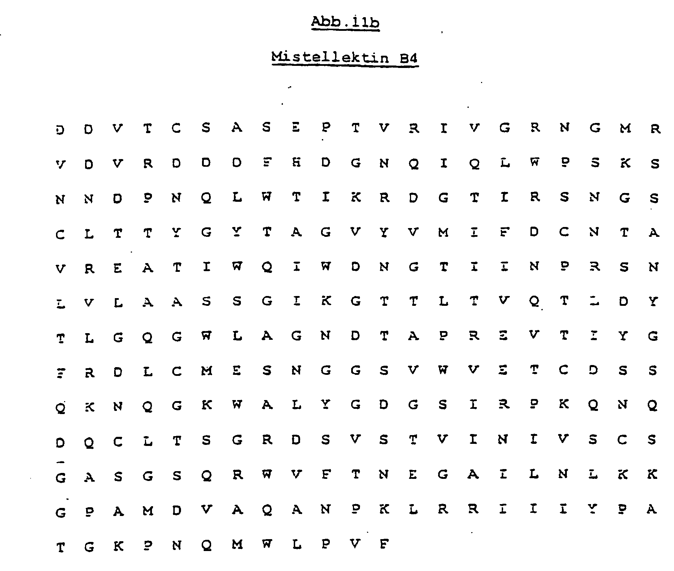

- Abb. 11:: Darstellung der (a) Nukleinsäuresequenz und (b) Aminosäuresequenz von Mistellektin B4.

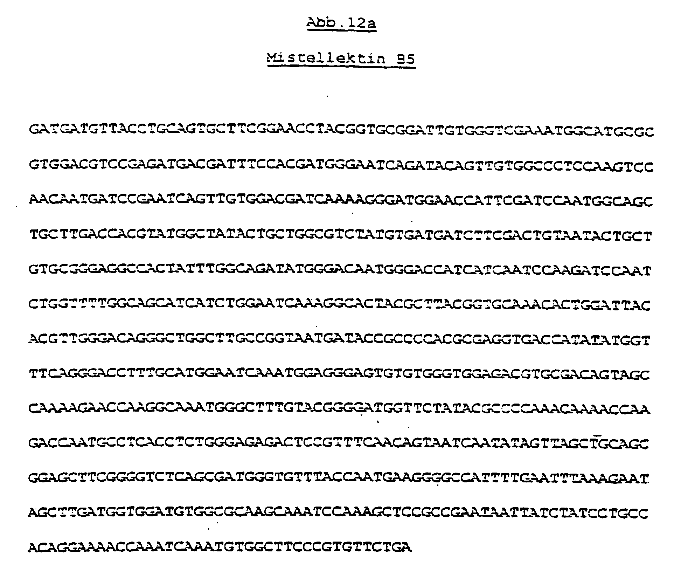

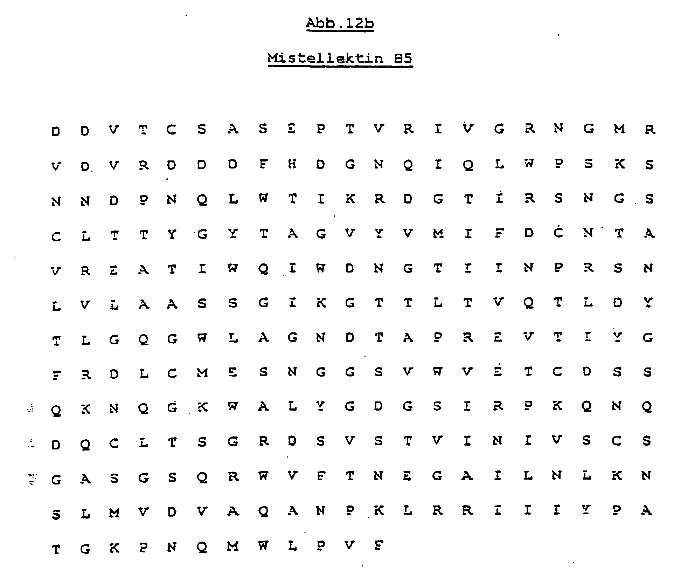

- Abb. 12:: Darstellung der (a) Nukleinsäuresequenz und (b) Aminosäuresequenz von Mistellektin B5.

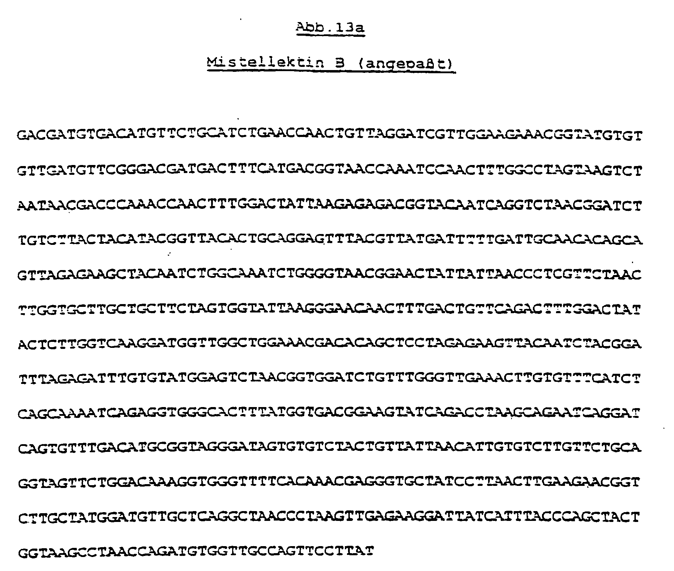

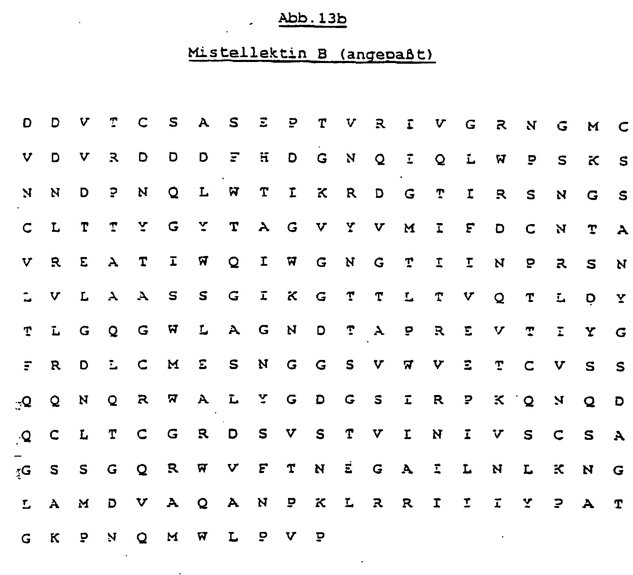

- Abb. 13:: Darstellung der Nukleinsäuresequenz von Mistellektin B, wobei die Nukleinsäuresequenz dem Kodongebrauch von *Brassica* angepaßt ist.

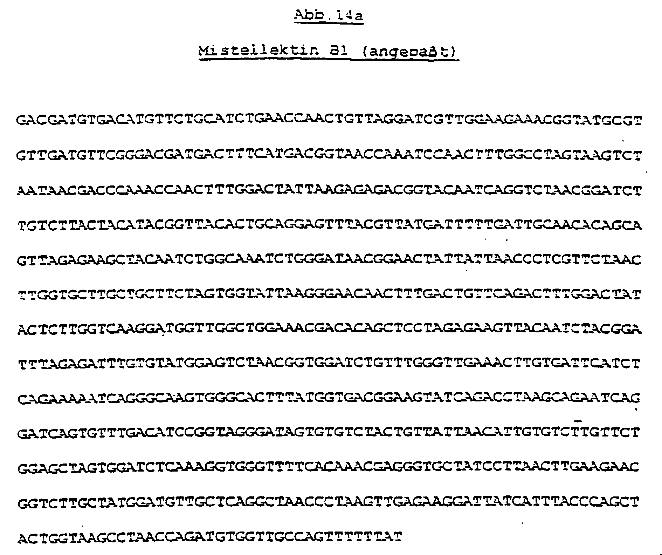

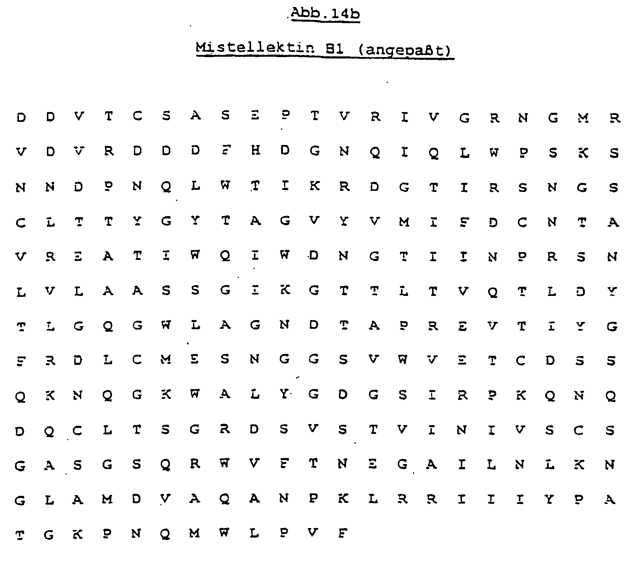

- Abb. 14:: Darstellung der Nukleinsäuresequenz von Mistellektin B1, wobei die Nukleinsäuresequenz dem Kodongebrauch von *Brassica* angepaßt ist.

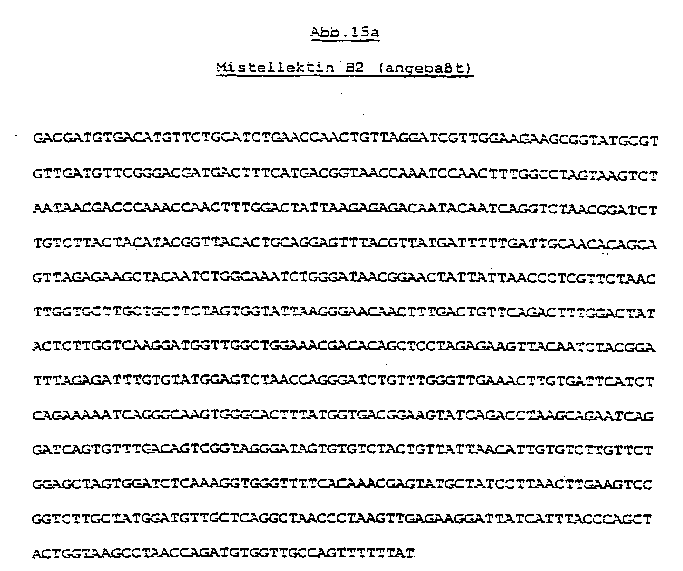

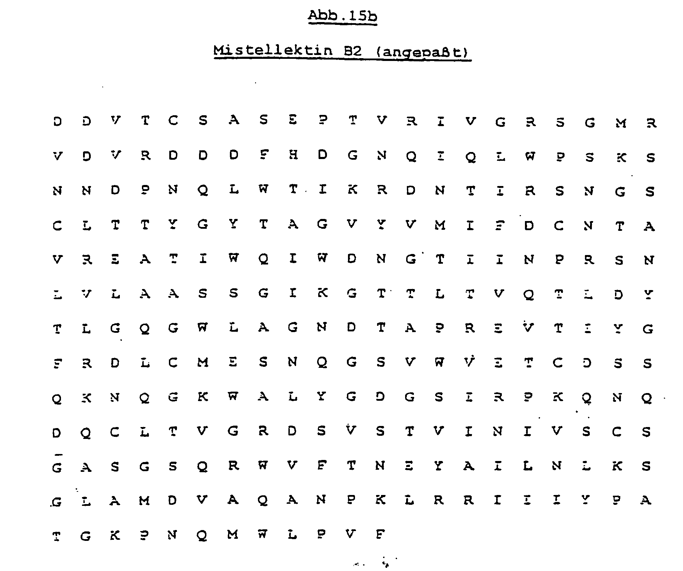

- Abb. 15:: Darstellung der Nukleinsäuresequenz von Mistellektin B2, wobei die Nukleinsäuresequenz dem Kodongebrauch von *Brassica* angepaßt ist.

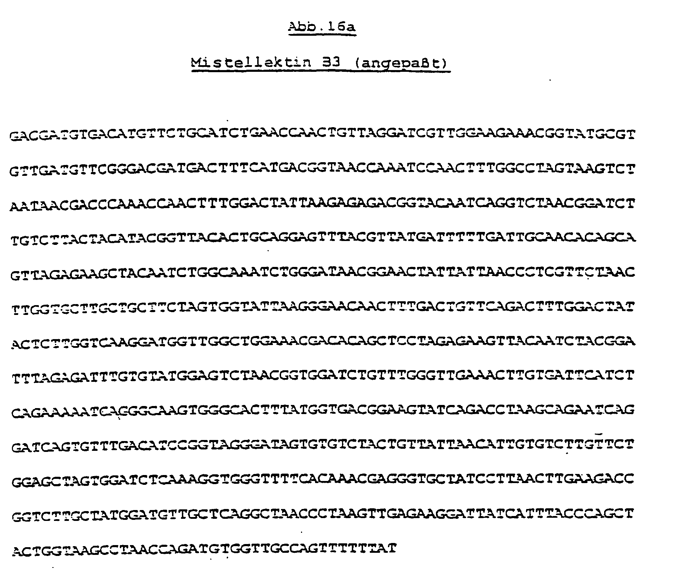

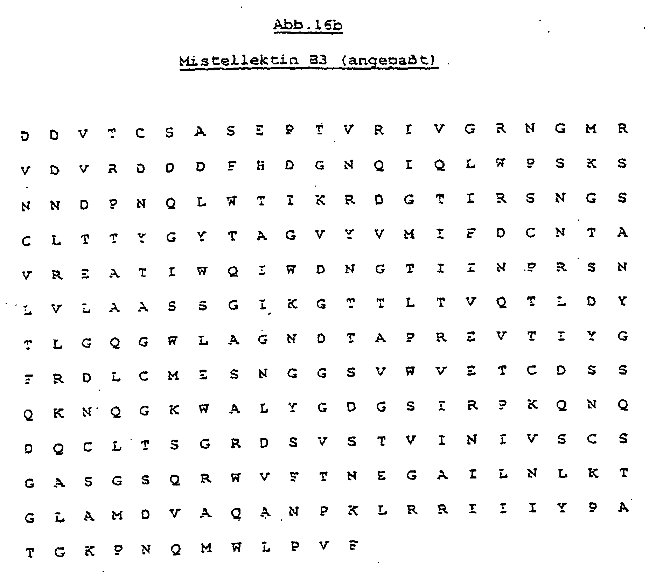

- Abb. 16:: Darstellung der Nukleinsäuresequenz von Mistellektin B3, wobei die Nukleinsäuresequenz dem Kodongebrauch von *Brassica* angepaßt ist.

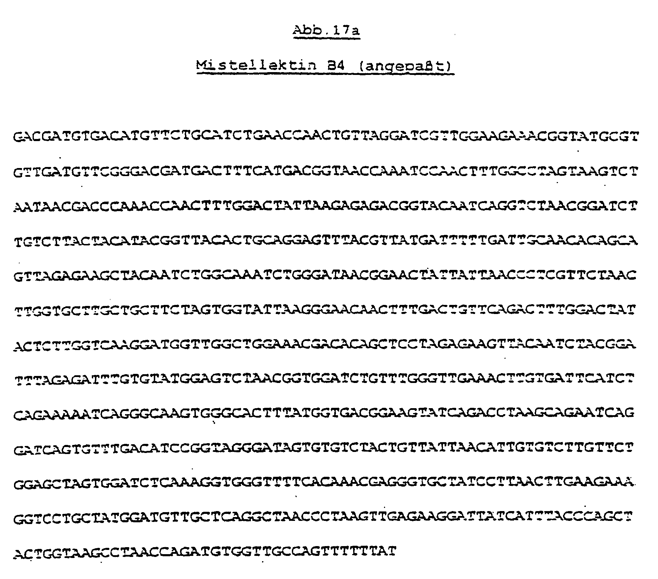

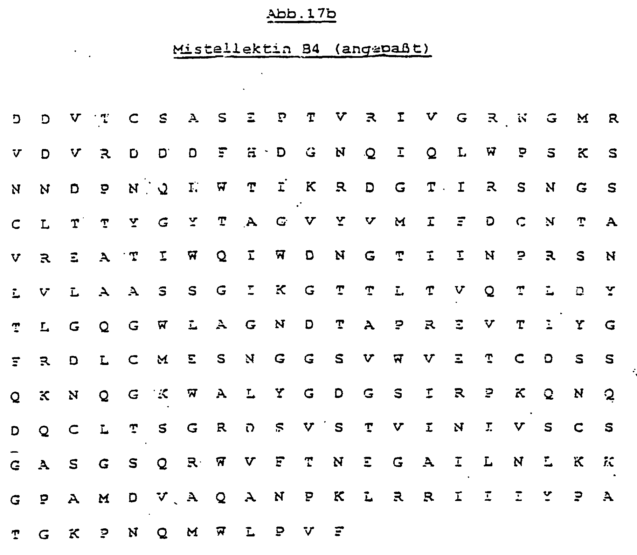

- Abb. 17:: Darstellung der Nukleinsäuresequenz von Mistellektin B4, wobei die Nukleinsäuresequenz dem Kodongebrauch von Brassica angepaßt ist

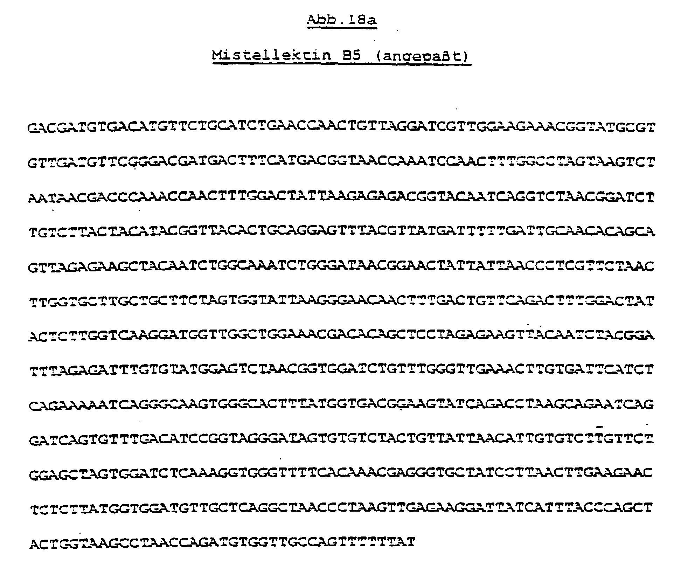

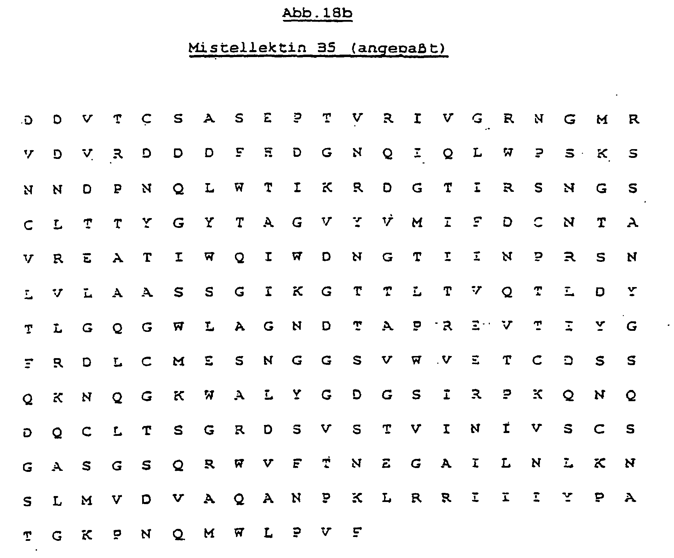

- Abb. 18:: Darstellung der Nukleinsäuresequenz von Mistellektin B5. wobei die Nukleinsäuresequenz dem Kodongebrauch von *Brassica* angepaßt ist

### Beispiel 1

Es wurden Mistelpflanzen der Art *Viscum album L ssp*. *platyspermum* Kell von im Elsaß wachsenden Pappeln geerntet und direkt nach der Emte eingefroren. Das Pflanzenmaterial wurde im Labor in flüssigem Stickstoff zerstoßen und anschließend die DNA von 100 mg Pflanzenmaterial durch das im DNeasy Plant Mini-Handbook 09/96 von Quiagen beschriebene Verfahren isoliert.

### Beispiel 2

### PCR-Bedingungen zur Amplifikation von Mistellektin-I-DNA:

Für die Amplifikation von genomischer Mistellektin-I-DNA wurden 100 ng Template-DNA, präpariert wie in Beispiel 1 angegeben, in einer PCR-Reaktion mit 30 Zyklen unter Verwendung von Taq-Polymerase (Boehringer Mannheim) verwendet. Der Template-DNA wurden 1 µg Primer, MgCl₂ (Endkonzentration 2 mM), Nukleotidgemisch A, T, C, G (Endkonzentration 0,2 mM) und 2,5 Einheiten Taq-Polymerase zugesetzt. Die Reaktion wurde durch einen Denaturierungsschritt der DNA für 5 Minuten bei 94°C als Hotstart-PCR gestartet. Dabei vermischten sich das Enzym und die restlichen Reagenzien erst nach dem eine Wachsbarriere zwischen den Komponenten geschmolzen war. Die 30 anschließenden Zyklen werden unter folgenden Bedingungen durchgeführt

| | | |
|---|---|---|
| Denaturierung | 94°C | 30 Sekunden |
| Annealing | 55°C | 30 Sekunden |
| Amplifikation | 72°C | 1 Minute |

Im Anschluß an die 30 Zyklen wurde noch eine 7-minütige Verlängerungsreaktion bei 72°C durchgeführt, bevor die Reaktionsmischung auf 4°C heruntergekühlt wurde.

Die in der PCR-Reaktion eingesetzten Primer hybridisierten mit Fragmenten der für MLB kodierenden genomischen DNA und hatten die folgenden Sequenzen:

Die Nukleotide sind gemäß dem IUPAC-IUB-Code definiert.

Das Oligonukleotid B1 hybridisierte an den Nukleinsäurebereich, der den Aminosäuren 24 bis 30 der MLB-Sequenz entspricht, während das Oligonukleotid B2 an die für die Aminosäuren 253-258 von MLB kodierende komplementäre DNA-Sequenz hybridisierte.

### Beispiel 3

Um die angrenzenden 3'- und 5'-Sequenzen der in Beispiel 2 amplifizierten DNA zu ermitteln, wurde die RACE-Technik verwendet 2 µg RNA-Template wurden in cDNA-Synthese Puffer (Endkonzentrabon: 20mM Tris-HCl, 8 mM MgCl₂, 30 mM KCl, 1 mM Dithiotreitol; pH 8,5 (20°C)) mit AMV reverser Transkriptase, den Deoxynukleotiden und dem spezifischen Primer (s.u.) versetzt und 60 min bei 65°C inkubiert. Anschließend wurde die Probe 10 min bei 65°C inkubiert. Nach der Aufreinigung des ersten cDNA-Stranges wurde mit 2/5 der synthetisierten cDNA die "Tailing"-Reaktion mit terminaler Transferase durchgeführt. Nach der Tailing-Reaktion erfolgte eine PCR mit dem OligodT-Anker-Primer und dem spezifischen Primer (Inkubationsbedingungen s.o., mit Ausnahme der Annealingtemperatur, die auf 50°C gesenkt wurde).

Zur Ermittlung der 5'-Bereiche der in Beispiel 2 amplifizierten Nukleinsäuremoleküle wird das Oligonukleotid mit folgender Sequenz benutzt:

Eine zu diesem Oligonukleotid komplementäre DNA-Sequenz kodiert die Aminosäuresequenz 79-85 des MLB-Polypeptides. Um in einem entsprechenden Experiment die 3'-Bereiche der amplifizierten. Nukleinsäuremoleküle zu ermitteln, wurde das Oligonukleotid mit der folgenden Sequenz eingesetzt:

Diese Nukleinsäuresequenz kodiert für den Aminosäurebereich 74-81 des MLB-Polypeptids. Für die 3'-RACE-Reaktion wurden dieselben Inkubationsbedingungen wie bei der 5'-RACE verwandt, mit Ausnahme der "Taiting"-Reaktion, die hier wegen des polyA-Schwanzes der mRNA nicht nötig ist. In beiden Verfahren wurde als zweiter Primer der Olgio-dT-Anker-Primer des Boehringer Mannheim Kits verwendet.

### Beispiel 4

### Pharmazeutische Zusammensetzung mit zytotoxischer Wirkung:

Mistel-, Tabak- und Rapszellen werden mit RNA-Vektoren, die für MLA1 und MLA2 kodieren, transfiziert, die Zellen jeweils nach einigen Tagen geerntet und die MLA1 - und MLA2-Proteine durch Affinitätschromatographie gereinigt. Als Gelmaterial wird Divinylsulfon (DVS)-aktivierte Lactose-gekoppelte Sepharose 4B (Pharmacia) eingesetzt. Durch Behandlung mit 0,2 M HCl wird das Material aktiviert, d.h. die Sepharose-Struktur wird teilweise hydrolysiert und Zuckerbindungsstellen werden freigelegt, an welche die Lektine binden können. 100 ml Gelmaterial werden mit 0,2 M HCl in einem Büchner-Trichter gewaschen und in 200 ml 0,2 M HCl suspendiert. Durch 3,5-stündige Inkubation der Suspension bei 50°C im Wasserbad erfolgt die Hydrolyse des Gelmaterials. Die Suspension wird mit Wasser und anschließend mit Peptideluent (0,05 M K₂HPO₄ x 3H₂O, 0, 15 M NaCl, pH 7,0) säurefrei gewaschen. Dann wird die Suspension entgast, der Peptideluent abgesaugt, das zähflüssige Gelmaterial in eine Leersäule XK50/30 (3 x 50 cm; Pharmacia) gefüllt und mit Peptideluent pH 7,0 mit einer Fließgeschwindigkeit von zuerst 2,5 ml/min und dann 5 ml/min gepackt. Die Säule wird mit dem gleichen Eluent bei einer Fließgeschwindigkeit von 1 ml/min equilibriert. Das aus den transfizierten Mistelzellen gewonnene Zellextrakt wird zentrifugiert und der Überstand auf die Säule geladen. Die Auftrennung wird bei einer Fließgeschwindigkeit von 1 ml/min mit Peptideluent pH 7,0 durchgeführt. Die Lektine werden vom Säulenmaterial mit einem Puffer aus 0,2 M Lactose in Peptideluent pH 7,0 bei einer Fließgeschwindigkeit von 2 ml/min eluiert. Die Elution des Lektins von der Säule wird durch Messung der Absorption bei 206 nm gemessen. Die Lektin enthaltenden Fraktionen werden gesammelt, eingefroren und lyophilisiert. Falls gewünscht, kann ein weiterer Reinigungsschritt über eine HPLC-Säule erfolgen. Dazu eignet sich eine Vydac C4 300 A-Säule, die bei einer Fließgeschwindigkeit von 300 µl/min und einem Gradienten von 20% bis 100% B in 60 Minuten gefahren wird, wobei Elutionsmittel A 0,17% TFA in Wasser ist und Elutionsmittel B 0,15% TFA in 80% CH₃CN in Wasser ist. Die Elution der Misteltektine wird bei einer Wellenlänge von 214 nm detektiert.

Die gereinigten MLA1- und MLA 2-Polypeptide werden an ein geeignetes Zellerkennungsmolekül gekoppelt. Ist das Zellerkennungsmolekül ein mono- oder polyklonaler Antikörper, kann dieser z.B. unter Verwendung von Glutaraldehyd an das zytotoxische MLA1 oder MLA2 gebunden werden oder direkt als chimäres Fusionprotein (Antikörper-MLA) in den entsprechenden Expressionssystemen exprimiert werden.

### Beispiel 5

### Pharmazeutische Zusammensetzung:

Mistetzellen werden mit RNA-Vektoren transfiziert, die für die Mistellektine MLA1 und MLA2 sowie Mistellektine MLB bis MLB6 kodieren, transfiziert. Nach einigen Tagen werden die Mistellektinmonomere bzw. -dimere durch Verfahren wie in Beispiel 4 beschrieben aus den Mistelzellen extrahiert und gereinigt. Die so erhaltenen Monomere können in vitro zu heterologen und homologen Dimeren fusioniert werden. Auf diese Weise entsteht eine Velzahl von unterschiedlichen Kombinationen der einzelnen MLA- und MLB-Polypeptide. Das so hergestellte heterogene Gemisch an ML-I-Dimeren und -Monomeren wird lyophilisiert und zur Formulierung mit einem geeigneten Träger verwendet.

## Patentansprüche

1. Verfahren zum Herstellen eines Mistellektin-Polypeptides im heterologen System mit der Sequenz: umfassend den Schritt des Exprimierens mit Hilfe eines eukaryontischen oder prokaryontischen Vektors, in den eine nach dem üblichen genetischen Code für das Mistellektin-Polypeptid kodierende Nukleinsäure kloniert ist, in einem geeigneten heterologen eukaryontischen oder prokaryontischen Wirt,
wobei X1 D oder E ist; X2 G oder Q ist; X3 I oder V ist; X4 L oder A ist: X5 DR ist oder fehlt, X6 T ist; X7 P oder T ist; X8 D oder E ist; X9 S oder T ist; X10 F oder Y ist; X11 T oder A ist; X12 A oder Y ist; X13 Y oder D ist; X14 A oder E ist; X15 V oder M ist; X16 I oder F ist; X17 P oder S ist X18 P oder T ist X19 T oder S ist X20 D oder S ist; X21 N oder S ist; X22 C oder R ist; X23 G oder N ist; X24 G oder D ist; X25 G oder Q ist; X26 V oder D ist; X27 Q oder K ist X28 G ist oder fehlt, X29 R oder K ist; X30 C oder S oder V ist; X31 A oder G ist: X32 G oder A ist, X33 S oder G ist; X34 G oder S ist X35 G oder Y ist, X36 N oder S oder T oder K. ist; X37 S oder G ist, X38. L oder P ist; X39 A oder M ist; X40 M oder V ist; X41 P oder F ist.

2. Verfahren nach Anspruch 1, wobei das Mistellektin-Polypeptid der Mistellektin-A-Kette (MLA) entspricht und die folgende Sequenz umfaßt: wobei X1 bis X20 die oben angegebene Bedeutung haben.

3. Verfahren nach Anspruch 1, wobei das Mistellektin-Polypeptid der Mistellektin-B-Kette (MLB) entspricht und die folgende Sequenz umfaßt:
wobei X21 bis X41 die oben angegebene Bedeutung haben.

4. Mistellektin-Polypeptid mit
I) der in Abb. 1b dargestellten Sequenz,
II) der in Abb. 3b dargestellten Sequenz,
III) der in Abb. 7b dargestellten Sequenz,
IV) der in Abb. 8b dargestellten Sequenz,
V) der in Abb. 9b dargestellten Sequenz,
VI) der in Abb. 10b dargestellten Sequenz,
VII) der in Abb. 11b dargestellten Sequenz, oder
VIII) der in Abb. 12b dargestellten Sequenz.

5. Verfahren zum Bereitstellen eines Nukteinsäuremoleküls, das in einem heterologen Wirt für ein Mistellektin-Polypeptid der Sequenz unten kodiert, umfassend die Schritte:
a) Präparieren von Mistelzell-RNA oder chromosomaler Mistetzell-DNA und
b) Amplifizieren von Mistelzell-RNA oder chromosomaler Mistellektin-DNA durch PCR unter Verwendung von Oligonukleotiden, die von dem in Abb.1 b dargestellten Mistellektin-Polypeptid abgeleitet sind, und
c) gegebenenfalls Identifizieren von Sequenzen, die 5' und 3' von der amplifizierten Nukleinsäure liegen sowie deren Amplifikation, und
d) Isolieren der in Schritt b) und/oder c) amplifizierten Nukleinsäuremoleküle, und
e) gegebenenfalls Ligieren mehrerer der in Schritt b) und/oder c) amplifizierten Nukleinsäuremoleküle, so daß ein Nukleinsäuremolekül mit vollständigem offenen Leseraster erhalten wird und
f) gezieltes Mutieren des erhaltenen Nukleinsäuremoleküls, um das Nukleinsäuremolekül dem üblichen genetischen Code des heterologen Wirtes für eine der in Mistelzellen identifzierten Mistellektin-Polypeptidisoformen anzupassen,
wobei die Sequenz des Mistellektins wie folgt ist:
wobei X1 D oder E ist; X2 G oder Q ist; X3 I oder V ist X4 L oder A ist X5 DR ist oder fehlt, X6 T ist; X7 P oder T ist X8 D oder E ist; X9 S oder T ist; X10 F oder Y ist; X11 T oder A ist; X 12 A oder Y ist; X13 Y oder D ist; X14 A oder E. ist; X15 V oder M ist; X16 I oder F ist; X17 P oder S ist; X18 P oder T ist; X19.T oder S ist; X20 D oder S ist; X21 N oder S ist X22 C oder R ist X23 G oder N ist; X24 G oder D ist; X25 G oder Q ist; X26 V oder D ist, X27 Cl oder K ist; X28 G ist oder fehlt; X29 R oder K ist; X30 C oder S oder V ist; X31 A oder G ist; X32 G oder A ist; X33 S oder G ist X34 G oder S ist; X35 G oder Y ist; X36 N oder S oder T oder K ist; X37 S oder G ist; X38 L oder P ist, X39 A oder M ist; X40 M oder V ist X41 P oder F ist

6. Nukleinsäuremolekül mit
I) der in Abb. 1 a dargestellten Sequenz,
II) der in Abb. 2a dargestellten Sequenz,
III) der in Abb. 3a dargestellten Sequenz,
IV) der in Abb. 7a dargestellten Sequenz,
V) der in Abb. 8a dargestellten Sequenz,
VI) der in Abb. 9a dargestellten Sequenz,
VII) der in Abb. 10a dargestellten Sequenz,
VIII) der in Abb. 11a dargestellten Sequenz, oder
IX) der in Abb. 12a dargestellten Sequenz.

7. Verfahren zum Herstellen eines Mistellektin-Polypeptides in Mistelzellen und/oder einer transgenen Mistelpflanze mit der Sequenz: umfassend den Schritt des Exprimierens mit Hilfe eines eukaryontischen Vektors, der eine für das Mistellektin-Polypeptid kodierende Nukleinsäure mit der ursprünglich in Mistelzell-DNA gefundenen Nukleinsäuresequenz enthält, in einer Mistetzelle und/oder einer transgenen Mistelpflanze, wobei das Transkriptionsprodukt dieses Nukleinsäuremoleküls in Mistelzellen und/oder transgenen Mistelpflanzen durch posttranskriptionale und/oder posttranslationale Mechanismen modifiziert wird,
wobei X1 D oder E ist; X2 G oder Q ist X3 Ioder V ist X4 L oder A ist; X5 DR ist oder fehlt; X6 T ist; X7 P oder T ist; X8 D oder E ist; X9 S oder T ist X10 F oder Y ist; X11 T oder A ist; X12 A oder Y ist; X13 Y oder D ist; X14 A oder E ist; X15 V oder M ist, X16 I oder F ist; X17 P oder S ist; X18 P oder T ist; X19 T oder S ist; X20 D oder S ist; X21 N oder S ist; X22 C oder R ist; X23 G oder N ist; X24 G oder D ist; X25 G oder Q ist; X26 V oder D ist; X27 Q oder K ist; X28 G ist oder fehlt; X29 R oder K ist; X30 C oder S oder V ist X31 A oder G ist; X32 G oder A ist; X33 S oder G ist; X34 G oder S ist; X35 G oder Y ist; X36 N oder S oder T oder K ist; X37 S oder G ist; X38 L oder P ist; X39 A oder M ist; X40 M oder V ist; X41 P oder F ist.

8. Verfahren nach Anspruch 7, wobei das Mistellektin-Polypeptid der mistellektin-A-Kette entspricht und die folgende Sequenz umfasst: wobei X1 bis X20 die oben angegebene Bedeutung haben.

9. Verfahren nach Anspruch 7, wobei das Mistellektin-Polypeptid der Mistellektin-B-Kette entspricht und die folgende Sequenz umfässt: wobei X21 bis X41 die oben angegebene Bedeutung haben.

10. Verfahren zum Bereitstellen eines Nukleinsäuremoleküls, das in einer Mistelzelle und/oder einer transgenen Mistelpflanze für ein Mistellektin-Polypeptid der Sequenz unten kodiert, umfassend die Schritte:
a) Präparieren von Mistelzell-RNA oder chromosomaler Mistelzelt-DNA und
b) Amplifizieren von Mistelzell-RNA oder chromosomaler Misteltektin-DNA durch PCR unter Verwendung von Oligonukleotiden, die von dem in Abb.1b dargestellten Mistellektin-Polypeptid abgeleitet sind, und
c) gegebenenfalls Identifizieren von Sequenzen, die 5' und 3' von der amplifizierten Nukleinsäure liegen sowie deren Amplfikation, und
d) Isolieren der in Schritt b) und/oder c) amplifizierten Nukleinsäuremoleküle, und
e) gegebenenfalls Ligieren mehrerer der in Schritt b) und/oder c) isolierten Nukleinsäuremoleküle, so daß ein Nukleinsäuremolekül mit vollständigem offenen Leseraster erhalten wird, und
f) gegebenenfalls gezieltes Mutieren des erhaltenen Nukleinsäuremoleküls, um das Nukleinsäuremolekül dem üblichen genetischen Code für eine der in Mistelzellen identifizierten Misteltektin-Polypeptidisoformen anzupassen und/oder die Expression zu optimieren
wobei das Mistellektin-Polypeptid folgende Sequenz umfasst:
wobei X1 D oder E ist; X2 G oder Q ist; X3 I oder V ist; X4 L oder A ist; X5 DR ist oder fehlt; X6 ist; X7 P oder T ist; X8 D oder E ist; X9 S oder T ist; X1 0 F oder Y ist; X11 T oder A ist; X12 A oder Y ist; X13 Y oder D ist; X14 A oder E ist; X15 V oder M ist; X16 I oder F ist; X17 P oder S ist; X18 P oder T ist; X19 T oder S ist; X20 D oder S ist; X21 N oder S ist; X22 C oder R ist; X23 G oder N ist; X24 G oder D ist; X25 G oder Q ist; X26 V oder D ist; X27 Q oder K ist; X28 G ist oder fehlt; X29 R oder K ist; X30 C oder S oder V ist; X31 A oder G ist; X32 G oder A ist; X33 S oder G ist; X34 G oder S ist; X35 G oder Y ist; X36 N oder S oder T oder K ist; X37 S oder G ist; X38 L oder P ist; X39 A oder M ist; X40 M oder V ist; X41 P oder F ist.

11. Verfahren zum Herstellen eines Polypeptids nach einer der Ansprüche 1 bis 3 oder 7 bis 9, umfassend als weiteren Schritt das Modifizieren von Zuckerseitenketten durch enzymatisches und/oder chemisches Ergänzen, Entfernen und/oder Ändern einer oder mehrerer Seitenketten.

12. Verfahren nach Anspruch 11, wobei das Ergänzen, Entfernen und/oder Ändern der Zuckerseitenketten zur Angleichung an die natürlichen Proteine führt.

## Claims

1. Process for the production of a mistletoe lectin polypeptide in the heterologous system having the sequence: comprising the step of expressing by means of a eukaryotic or prokaryotic vector, into which a nucleic acid coding for the mistletoe lectin polypeptide according to the usual genetic code is cloned, in a suitable heterologous eukaryotic or prokaryotic host,
wherein X1 is D or E, X2 is G or Q, X3 is I or V, X4 is L or A, X5 is DR or missing, X6 is T, X7 is P or T, X8 is D or E, X9 is S or T, X10 is F or Y, X11 is T or A, X12 is A or Y, X13 is Y or D, X14 is A or E, X15 is V or M, X16 is I or F, X17 is P or S, X18 is P or T, X19 is T or S, X20 is D or S, X21 is N or S, X22 is C or R, X23 is G or N, X24 is G or D, X25 is G or Q, X26 is V or D, X27 is Q or K, X28 is G or missing, X29 is R or K, X30 is C or S or V, X31 is A or G, X32 is G or A, X33 is S or G, X34 is G or S, X35 is G or Y, X36 is N or S or T or K, X37 is S or G, X38 is L or P, X39 is A or M, X40 is M or V and X41 is P or F.

2. Process according to Claim 1, wherein the mistletoe lectin polypeptide corresponds to the mistletoe lectin A-chain (MLA) and contains the following sequence
wherein X1 to X20 have the meaning stated above.

3. Process according to Claim 1, wherein the mistletoe lectin polypeptide corresponds to the mistletoe lectin B-chain (MLB) and contains the following sequence
wherein X21 to X41 have the meaning stated above.

4. Mistletoe lectin polypeptide having
(I) the sequence as set forth in Fig. 1 b,
(II) the sequence as set forth in Fig. 3b,
(III) the sequence as set forth in Fig. 7b,
(IV) the sequence as set forth in Fig. 8b,
(V) the sequence as set forth in Fig. 9b,
(VI) the sequence as set forth in Fig. 10b,
(VII) the sequence as set forth in Fig. 11b, or
(VIII) the sequence as set forth in Fig. 12b.

5. Process for the preparation of a nucleic acid molecule which codes for a mistletoe lectin polypeptide according to the sequence below in a heterlogous host, comprising the steps:
a) preparing of mistletoe cell RNA or chromosomal mistletoe cell DNA and
b) amplifying mistletoe cell RNA or chromosomal mistletoe lectin DNA by PCR using oligonucleotides which are derived from the mistletoe lectin polypeptide shown in Fig.1b, and
c) if necessary, identifying of sequences which lie 5' and 3' from the amplified nucleic acid and amplification thereof, and
d) isolating of the nucleic acid molecules amplified in step b) and/or c), and
e) if necessary, ligating several of the nucleic acid molecules amplified in step b) and/or c), such that a nucleic acid molecule with a complete open reading frame is obtained and
f) targeted mutation of the nucleic acid molecule obtained in order to match the nucleic acid molecule to the usual genetic code of the heterologous host for one of the mistletoe lectin polypeptide isoforms identified in mistletoe cells,
wherein the mistletoe lectin sequence is as follows: wherein X1 is D or E, X2 is G or Q, X3 is I or V, X4 is L or A, X5 is DR or missing, X6 is T, X7 is P or T, X8 is D or E, X9 is S or T, X10 is F or Y, X11 is T or A, X12 is A or Y, X13 is Y or D, X14 is A or E, X15 is V or M, X16 is I or F, X17 is P or S, X18 is P or T, X19 is T or S, X20 is D or S, X21 is N or S, X22 is C or R, X23 is G or N, X24 is G or D, X25 is G or Q, X26 is V or D, X27 is Q or K, X28 is G or missing, X29 is R or K, X30 is C or S or V, X31 is A or G, X32 is G or A, X33 is S or G, X34 is G or S, X35 is G or Y, X36 is N or S or T or K, X37 is S or G, X38 is L or P, X39 is A or M, X40 is M or V and X41 is P or F.

6. A nucleic acid molecule having
(I) the sequence as set forth in Fig. 1a,
(II) the sequence as set forth in Fig. 2a,
(III) the sequence as set forth in Fig. 3a,
(IV) the sequence as set forth in Fig. 7a,
(V) the sequence as set forth in Fig. 8a,
(VI) the sequence as set forth in Fig. 9a,
(VII) the sequence as set forth in Fig. 10a,
(VIII) the sequence as set forth in Fig. 11a, or
(IX) the sequence as set forth in Fig. 12a.

7. Process for the production of a mistletoe lectin polypeptide in mistletoe cells and/or a transgenic mistletoe plant having the sequence: comprising the step of expressing by means of a eukaryotic vector, which contains a nucleic acid coding for the mistletoe lectin polypeptide having the nucleic acid sequence originally found in mistletoe cell DNA, in a mistletoe cell and/or a transgenic mistletoe plant, wherein the transcription product of this nucleic acid molecule is modified in mistletoe cells and/or transgenic mistletoe plants by postranscriptional and/or posttranslational mechanisms, wherein X1 is D or E, X2 is G or Q, X3 is I or V, X4 is L or A, X5 is DR or missing, X6 is T, X7 is P or T, X8 is D or E, X9 is S or T, X10 is F or Y, X11 is T or A, X12 is A or Y, X13 is Y or D, X14 is A or E, X15 is V or M, X16 is I or F, X17 is P or S, X18 is P or T, X19 is T or S, X20 is D or S, X21 is N or S, X22 is C or R, X23 is G or N, X24 is G or D, X25 is G or Q, X26 is V or D, X27 is Q or K, X28 is G or missing, X29 is R or K, X30 is C or S or V, X31 is A or G, X32 is G or A, X33 is S or G, X34 is G or S, X35 is G or Y, X36 is N or S or T or K, X37 is S or G, X38 is L or P, X39 is A or M, X40 is M or V and X41 is P or F.

8. Process according to Claim 7, wherein the mistletoe lectin polypeptide corresponds to the mistletoe lectin A-chain and includes the following sequence
wherein X1 to X20 have the meaning stated above.

9. Process according to Claim 7, wherein the mistletoe lectin polypeptide corresponds to the mistletoe lectin B-chain and includes the following sequence
wherein X21 to X41 have the meaning stated above.

10. Process for the preparation of a nucleic acid molecule, which codes for a mistletoe lectin according to the sequence below a mistletoe cell and/or a transgenic mistletoe plant, comprising the steps:
a) preparing of mistletoe cell RNA or chromosomal mistletoe cell DNA and
b) amplifying mistletoe cell RNA or chromosomal mistletoe lectin DNA by PCR using oligonucleotides which are derived from the mistletoe lectin polypeptide shown in Fig. 1b, and
c) if necessary, identifying of sequences which lie 5' and 3' from the amplified nucleic acid and amplification thereof, and
d) isolating of the nucleic acid molecules amplified in step b) and/or c), and
e) if necessary, ligating several of the nucleic acid molecules isolated in step b) and/or c), such that a nucleic acid molecule with a complete open reading frame is obtained and
f) if necessary, targeted mutation of the nucleic acid molecule obtained in order to match the nucleic acid molecule to the usual genetic code for one of the mistletoe lectin polypeptide isoforms identified in mistletoe cells and/or to optimise expression,
wherein the mistletoe lectin sequence is as follows:
wherein X1 is D or E, X2 is G or Q, X3 is I or V, X4 is L or A, X5 is DR or missing, X6 is T, X7 is P or T, X8 is D or E, X9 is S or T, X10 is For Y, X11 is Tor A, X12 is A or Y, X13 is Y or D, X14 is A or E, X15 is V or M, X16 is I or F, X17 is P or S, X18 is P or T, X 19 is T or S, X20 is D or S, X21 is N or S, X22 is C or R, X23 is G or N, X24 is G or D, X25 is G or Q, X26 is V or D, X27 is Q or K, X28 is G or missing, X29 is R or K, X30 is C or S or V, X31 is A or G, X32 is G or A, X33 is S or G, X34 is G or S, X3S is G or Y, X36 is N or S or T or K, X37 is S or G, X38 is L or P, X39 is A or M, X40 is M or V and X41 is P or F.

11. Process for production of a polypeptide according to one of Claims 1 to 3 or 7 to 9 including as a further step the modification of sugar side-chains by enzymatic and/or chemical addition, removal and/or modification of one or several side-chains.

12. Process according to Claim 11, wherein the addition, removal and/or modification of the sugar side-chains leads to matching to the natural proteins.

## Revendications

1. Procédé de préparation dans un système hétérologue d'un polypeptide de lectine de gui ayant la séquence : comprenant l'étape d'expression à l'aide d'un vecteur eucaryote ou procaryote, dans lequel a été cloné un acide nucléique codant le polypeptide de lectine de gui selon le code génétique habituel, dans un hôte eucaryote ou procaryote hétérologue approprié,
dans laquelle X1 représente D ou E ; X2 représente G ou Q ; X3 représente I ou V ; X4 représente L ou A ; X5 représente DR ou est absent ; X6 représente T ; X7 représente P ou T; X8 représente D ou E ; X9 représente S ou T ; X10 représente F ou Y ; X11 représente T ou A ; X12 représente A ou Y ; X13 représente Y ou D ; X14 représente A ou E ; X15 représente V ou M ; X16 représente I ou F ; X17 représente P ou S ; X18 représente P ou T ; X19 représente T ou S ; X20 représente D ou S ; X21 représente N ou S ; X22 représente C ou R ; X23 représente G ou N ; X24 représente G ou D ; X25 représente G ou Q ; X26 représente V ou D ; X27 représente Q ou K ; X28 représente G ou est absent ; X29 représente R ou K ; X30 représente C ou S ou V ; X31 représente A ou G ; X32 représente G ou A ; X33 représente S ou G ; X34 représente G ou S ; X35 représente G ou Y ; X36 représente N ou S ou T ou K ; X37 représente S ou G ; X38 représente L ou P ; X39 représente A ou M ; X40 représente M ou V ; X41 représente P ou F.

2. Procédé selon la revendication 1, dans lequel le polypeptide de lectine de gui correspond à la chaîne A de lectine de gui (MLA) et comprend la séquence suivante :
dans laquelle X1 à X20 ont les significations susmentionnées.

3. Procédé selon la revendication 1, dans lequel le polypeptide de lectine de gui correspond à la chaîne B de lectine de gui (MLB) et comprend la séquence suivante :
dans laquelle X21 à X41 ont les significations susmentionnées.

4. Polypeptide de lectine de gui ayant
I) la séquence illustrée sur la figure 1b,
II) la séquence illustrée sur la figure 3b,
III) la séquence illustrée sur la figure 7b,
IV) la séquence illustrée sur la figure 8b,
V) la séquence illustrée sur la figure 9b,
VI) la séquence illustrée sur la figure 10b,
VII) la séquence illustrée sur la figure 11b ou
VIII) la séquence illustrée sur la figure 12b.

5. Procédé de préparation d'une molécule d'acide nucléique qui code un polypeptide de lectine de gui ayant la séquence ci-dessous dans un hôte hétérologue, comprenant les étapes consistant à :
a) préparer de l'ARN de cellules de gui ou de l'ADN chromosomique de cellules de gui et
b) amplifier l'ARN de cellules de gui ou l'ADN chromosomique de lectine de gui par PCR en utilisant des oligonucléotides qui sont dérivés du polypeptide de lectine de gui illustré sur la figure 1b, et
c) éventuellement, identifier des séquences qui sont situées en position 5' et 3' de l'acide nucléique amplifié et les amplifier, et
d) isoler les molécules de l'acide nucléique amplifié dans l'étape b) et/ou c), et
e) éventuellement, ligaturer plusieurs des molécules de l'acide nucléique amplifié dans l'étape b) et/ou c), de sorte que l'on obtient une molécule d'acide nucléique présentant un cadre de lecture ouvert complet et
f) provoquer une mutation ciblée de la molécule d'acide nucléique obtenue pour adapter la molécule d'acide nucléique au code génétique habituel de l'hôte hétérologue de l'une des isoformes du polypeptide de lectine de gui identifiées dans les cellules de gui,
dans lequel la séquence de la lectine de gui est la suivante :
dans laquelle X1 représente D ou E ; X2 représente G ou Q ; X3 représente I ou V ; X4 représente L ou A ; X5 représente DR ou est absent ; X6 représente T ; X7 représente P ou T ; X8 représente D ou E ; X9 représente S ou T ; X10 représente F ou Y ; X11 représente T ou A ; X12 représente A ou Y ; X13 représente Y ou D ; X14 représente A ou E ; X15 représente V ou M ; X16 représente I ou F ; X17 représente P ou S ; X18 représente P ou T ; X19 représente T ou S ; X20 représente D ou S ; X21 représente N ou S ; X22 représente C ou R ; X23 représente G ou N ; X24 représente G ou D ; X25 représente G ou Q ; X26 représente V ou D ; X27 représente Q ou K ; X28 représente G ou est absent ; X29 représente R ou K ; X30 représente C ou S ou V ; X31 représente A ou G ; X32 représente G ou A ; X33 représente S ou G ; X34 représente G ou S ; X35 représente G ou Y ; X36 représente N ou S ou T ou K ; X37 représente S ou G ; X38 représente L ou P ; X39 représente A ou M ; X40 représente M ou V ; X41 représente P ou F.

6. Molécule d'acide nucléique ayant
I) la séquence illustrée sur la figure 1a,
II) la séquence illustrée sur la figure 2b,
III) la séquence illustrée sur la figure 3a,
IV) la séquence illustrée sur la figure 7a,
V) la séquence illustrée sur la figure 8a,
VI) la séquence illustrée sur la figure 9a,
VII) la séquence illustrée sur la figure 10a,
VIII) la séquence illustrée sur la figure 11a ou
IX) la séquence illustrée sur la figure 12a.

7. Procédé de préparation d'un polypeptide de lectine de gui dans des cellules de gui et/ou dans un plant de gui transgénique ayant la séquence : comprenant l'étape d'expression à l'aide d'un vecteur eucaryote qui contient un acide nucléique codant le polypeptide de lectine de gui ayant la séquence d'acide nucléique initialement découverte dans l'ADN de cellules de gui, dans une cellule de gui et/ou dans un plant de gui transgénique, le produit de transcription de cette molécule d'acide nucléique étant modifié dans des cellules de gui et/ou dans des plants de gui transgéniques par des mécanismes post-transcriptionnels et/ou post-traductionnels,
dans laquelle X1 représente D ou E ; X2 représente G ou Q ; X3 représente I ou V ; X4 représente L ou A ; X5 représente DR ou est absent ; X6 représente T ; X7 représente P ou T; X8 représente D ou E ; X9 représente S ou T ; X10 représente F ou Y ; X11 représente T ou A ; X12 représente A ou Y ; X13 représente Y ou D ; X14 représente A ou E ; X15 représente V ou M ; X16 représente I ou F ; X17 représente P ou S ; X18 représente P ou T ; X19 représente T ou 5 ; X20 représente D ou S ; X21 représente N ou S ; X22 représente C ou R ; X23 représente G ou N ; X24 représente G ou D ; X25 représente G ou Q ; X26 représente V ou D ; X27 représente Q ou K ; X28 représente G ou est absent : X29 représente R ou K ; X30 représente C ou S ou V ; X31 représente A ou G ; X32 représente G ou A ; X33 représente S ou G ; X34 représente G ou S ; X35 représente G ou Y ; X36 représente N ou S ou T ou K ; X37 représente S ou G ; X38 représente L ou P ; X39 représente A ou M ; X40 représente M ou V ; X41 représente P ou F.

8. Procédé selon la revendication 7, dans lequel le polypeptide de lectine de gui correspond à la chaîne A de lectine de gui et comprend la séquence suivante :
dans laquelle X1 à X20 ont les significations susmentionnées.

9. Procédé selon la revendication 7, dans lequel le polypeptide de lectine de gui correspond à la chaîne B de lectine de gui et comprend la séquence suivante :
dans laquelle X21 à X41 ont les significations susmentionnées.

10. Procédé de préparation d'une molécule d'acide nucléique qui code un polypeptide de lectine de gui ayant la séquence ci-dessous dans une cellule de gui et/ou dans un plant de gui transgénique, comprenant les étapes consistant à :
a) préparer de l'ARN de cellules de gui ou de l'ADN chromosomique de cellules de gui et
b) amplifier l'ARN de cellules de gui ou l'ADN chromosomique de lectine de gui par PCR en utilisant des oligonucléotides qui sont dérivés du polypeptide de lectine de gui illustré sur la figure 1b, et
c) éventuellement, identifier des séquences qui sont situées en position 5' et 3' de l'acide nucléique amplifié et les amplifier, et
d) isoler les molécules de l'acide nucléique amplifié dans l'étape b) et/ou c), et
e) éventuellement, ligaturer plusieurs des molécules d'acide nucléique isolées dans l'étape b) et/ou c), de sorte que l'on obtient une molécule d'acide nucléique présentant un cadre de lecture ouvert complet, et
f) éventuellement, provoquer une mutation ciblée de la molécule d'acide nucléique obtenue pour adapter la molécule d'acide nucléique au code génétique habituel de l'une des isoformes du polypeptide de lectine de gui identifiées dans les cellules de gui et/ou pour optimiser l'expression,
dans lequel le polypeptide de lectine de gui comprend la séquence suivante:
dans laquelle X1 représente D ou E ; X2 représente G ou Q ; X3 représente I ou V ; X4 représente L ou A ; X5 représente DR ou est absent ; X6 représente T ; X7 représente P ou T; X8 représente D ou E ; X9 représente S ou T ; X10 représente F ou Y ; X11 représente T ou A ; X12 représente A ou Y ; X13 représente Y ou D; X14 représente A ou E ; X15 représente V ou M ; X16 représente I ou F ; X17 représente P ou S ; X18 représente P ou T ; X19 représente T ou S ; X20 représente D ou S ; X21 représente N ou S ; X22 représente C ou R ; X23 représente G ou N ; X24 représente G ou D ; X25 représente G ou Q ; X26 représente V ou D ; X27 représente Q ou K ; X28 représente G ou est absent ; X29 représente R ou K ; X30 représente C ou S ou V ; X31 représente A ou G ; X32 représente G ou A ; X33 représente S ou G ; X34 représente G ou S ; X35 représente G ou Y ; X36 représente N ou S ou T ou K ; X37 représente S ou G ; X38 représente L ou P ; X39 représente A ou M ; X40 représente M ou V ; X41 représente P ou F.

11. Procédé de préparation d'un polypeptide selon l'une quelconque des revendications 1 à 3 ou 7 à 9, comprenant, en tant qu'étape supplémentaire, la modification de chaînes latérales glucidiques par addition, suppression et/ou altération enzymatique et/ou chimique d'une ou plusieurs chaînes latérales.

12. Procédé selon la revendication 11, dans lequel l'addition, la suppression et/ou l'altération des chaînes latérales glucidiques conduit à l'adaptation aux protéines naturelles.


### SEQUENZPROTOKOLL

(1) ALLGEMEINE ANGABEN:
   (i) ANMELDER:
      (A) NAME: biosyn Arzneimittel GmbH
      (B) STRASSE: Schorndorfer Str. 32
      (C) ORT: Fellbach bei Stuttgart
      (E) LAND: Deutschland
      (F) POSTLEITZAHL: 70734
   (ii) BEZEICHNUNG DER ERFINDUNG: Rekombinante Mistellektine
   (iii) ANZAHL DER SEQUENZEN: 36
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 533 Aminosäuren
      (B) ART: Aminosäure
      (C) STRANGFORM: Einzelstrang
      (D) TOPOLOGIE: linear
   (ii) ART DES MOLEKÜLS: Protein
   (ix) MERKMAL:
      (A) NAME/SCHLÜSSEL: Modified-site
      (B) LAGE:15
      (D) SONSTIGE ANGABEN:/product= "Xaa ist Asp oder Glu"
         /label= Xaa1
   (ix) MERKMAL:
      (A) NAME/SCHLÜSSEL: Modified-site
      (B) LAGE:63
      (D) SONSTIGE ANGABEN:/product= "Xaa ist Gly oder Gln"
         /label= Xaa2
   (ix) MERKMAL:
      (A) NAME/SCHLÜSSEL: Modified-site
      (B) LAGE: 66
      (D) SONSTIGE ANGABEN:/product= "Xaa ist Ile oder Val"
         /label= Xaa3
   (ix) MERKMAL:
      (A) NAME/SCHLÜSSEL: Modified-site
      (B) LAGE:75
      (D) SONSTIGE ANGABEN:/product= "Xaa ist Leu oder Ala"
         /label= Xaa4
   (ix) MERKMAL:
      (A) NAME/SCHLÜSSEL: Modified-site
      (B) LAGE:107
      (D) SONSTIGE ANGABEN:/product= "Xaa ist Asp-Arg oder fehlt"
         /label= Xaa5
   (ix) MERKMAL:
      (A) NAME/SCHLÜSSEL: Modified-site
      (B) LAGE:113
      (D) SONSTIGE ANGABEN:/product= "Xaa ist Asn oder Thr"
         /label= Xaa6
   (ix) MERKMAL:
      (A) NAME/SCHLÜSSEL: Modified-site
      (B) LAGE:117
      (D) SONSTIGE ANGABEN:/product= "Xaa ist Pro oder Thr"
         /label= Xaa7
   (ix) MERKMAL:
      (A) NAME/SCHLÜSSEL: Modified-site
      (B) LAGE:134
      (D) SONSTIGE ANGABEN:/product= "Xaa ist Asp oder Glu"
         /label= Xaa8
   (ix) MERKMAL:
      (A) NAME/SCHLÜSSEL: Modified-site
      (B) LAGE:141
      (D) SONSTIGE ANGABEN:/product= "Xaa ist Ser oder Thr"
         /label= Xaa9
   (ix) MERKMAL:
      (A) NAME/SCHLÜSSEL: Modified-site
      (B) LAGE:145
      (D) SONSTIGE ANGABEN:/product= "Xaa ist Phe oder Tyr"
         /label= Xaa10
   (ix) MERKMAL:
      (A) NAME/SCHLÜSSEL: Modified-site
      (B) LAGE:152
      (D) SONSTIGE ANGABEN:/product= "Xaa ist Thr oder Ala"
         /label= Xaa11
   (ix) MERKMAL:
      (A) NAME/SCHLÜSSEL: Modified-site
      (B) LAGE:177
      (D) SONSTIGE ANGABEN:/product= "Xaa ist Ala oder Tyr"
         /label= Xaa12
   (ix) MERKMAL:
      (A) NAME/SCHLÜSSEL: Modified-site
      (B) LAGE:180
      (D) SONSTIGE ANGABEN:/product= "Xaa ist Tyr oder Asp"
         /label= Xaa13
   (ix) MERKMAL:
      (A) NAME/SCHLÜSSEL: Modified-site
      (B) LAGE:185
      (D) SONSTIGE ANGABEN:/product= "Xaa ist Ala oder Glu"
         /label= Xaa14
   (ix) MERKMAL:
      (A) NAME/SCHLÜSSEL: Modified-site
      (B) LAGE:191
      (D) SONSTIGE ANGABEN:/product= "Xaa ist Val oder Met"
         /label= Xaa15
   (ix) MERKMAL:
      (A) NAME/SCHLÜSSEL: Modified-site
      (B) LAGE:219
      (D) SONSTIGE ANGABEN:/product= "Xaa ist Ile oder Phe"
         /label= Xaa16
   (ix) MERKMAL:
      (A) NAME/SCHLÜSSEL: Modified-site
      (B) LAGE:224
      (D) SONSTIGE ANGABEN:/product= "Xaa ist Pro oder Ser"
         /label= Xaa17
   (ix) MERKMAL:
      (A) NAME/SCHLÜSSEL: Modified-site
      (B) LAGE:225
      (D) SONSTIGE ANGABEN:/product= "Xaa ist Pro oder Thr"
         /label= Xaa18
   (ix) MERKMAL:
      (A) NAME/SCHLÜSSEL: Modified-site
      (B) LAGE:232
      (D) SONSTIGE ANGABEN:/product= "Xaa ist Thr oder Ser"
         /label= Xaa19
   (ix) MERKMAL:
      (A) NAME/SCHLÜSSEL: Modified-site
      (B) LAGE:236
      (D) SONSTIGE ANGABEN:/product= "Xaa ist Asp oder Ser"
         /label= Xaa20
   (ix) MERKMAL:
      (A) NAME/SCHLÜSSEL: Modified-site
      (B) LAGE:287
      (D) SONSTIGE ANGABEN:/product= "Xaa ist Asn oder Ser"
         /label= Xaa21
   (ix) MERKMAL:
      (A) NAME/SCHLÜSSEL: Modified-site
      (B) LAGE:290
      (D) SONSTIGE ANGABEN:/product= "Xaa ist Cys oder Arg"
         /label= Xaa22
   (ix) MERKMAL:
      (A) NAME/SCHLÜSSEL: Modified-site
      (B) LAGE:325
      (D) SONSTIGE ANGABEN:/product= "Xaa ist Gly oder Asn"
         /label= Xaa23
   (ix) MERKMAL:
      (A) NAME/SCHLÜSSEL: Modified-site
      (B) LAGE:364
      (D) SONSTIGE ANGABEN:/product= "Xaa ist Gly oder Asp"
         /label= Xaa24
   (ix) MERKMAL:
      (A) NAME/SCHLÜSSEL: Modified-site
      (B) LAGE:426
      (D) SONSTIGE ANGABEN:/product= "Xaa ist Gly oder Gln"
         /label= Xaa25
   (ix) MERKMAL:
      (A) NAME/SCHLÜSSEL: Modified-site
      (B) LAGE:435
      (D) SONSTIGE ANGABEN:/product= "Xaa ist Val oder Asp"
         /label= Xaa26
   (ix) MERKMAL:
      (A) NAME/SCHLÜSSEL: Modified-site
      (B) LAGE:439
      (D) SONSTIGE ANGABEN:/product= "Xaa ist Gln oder Lys"
         /label= Xaa27
   (ix) MERKMAL:
      (A) NAME/SCHLÜSSEL: Modified-site
      (B) LAGE:442
      (D) SONSTIGE ANGABEN:/product= "Xaa ist Gly oder fehlt"
         /label= Xaa28
   (ix) MERKMAL:
      (A) NAME/SCHLÜSSEL: Modified-site
      (B) LAGE:443
      (D) SONSTIGE ANGABEN:/product= "Xaa ist Arg oder Lys"
         /label= Xaa29
   (ix) MERKMAL:
      (A) NAME/SCHLÜSSEL: Modified-site
      (B) LAGE:464
      (D) SONSTIGE ANGABEN:/product= "Xaa ist Cys oder Ser oder Val"
         /label= Xaa30
   ( ix ) MERKMAL:
      (A) NAME/SCHLÜSSEL: Modified-site
      (B) LAGE:480
      (D) SONSTIGE ANGABEN:/product= "Xaa ist Ala oder Gly"
         /label= Xaa
   (ix) MERKMAL:
      (A) NAME/SCHLÜSSEL: Modified-site
      (B) LAGE:481
      (D) SONSTIGE ANGABEN:/product= "Xaa ist Gly oder Ala"
         /label= Xaa32
   (ix) MERKMAL:
      (A) NAME/SCHLÜSSEL: Modified-site
      (B) LAGE:483
      (D) SONSTIGE ANGABEN:/product= "Xaa ist Ser oder Gly"
         /label= Xaa33
   (ix) MERKMAL:
      (A) NAME/SCHLÜSSEL: Modified-site
      (B) LAGE:484
      (D) SONSTIGE ANGABEN:/product= "Xaa ist Gly oder Ser"
         /label= Xaa34
   (ix) MERKMAL:
      (A) NAME/SCHLÜSSEL: Modified-site
      (B) LAGE:493
      (D) SONSTIGE ANGABEN:/product= "Xaa ist Gly oder Tyr"
         /label= Xaa35
   (ix) MERKMAL:
      (A) NAME/SCHLÜSSEL: Modified-site
      (B) LAGE:500
      (D) SONSTIGE ANGABEN:/product= "Xaa ist Asn oder Ser oder Thr oder Leu"
         /label=xaa36
   (ix) MERKMAL:
      (A) NAME/SCHLÜSSEL: Modified-site
      (B) LAGE:501
      (D) SONSTIGE ANGABEN:/product= "Xaa ist Ser oder Gly"
         /label= Xaa37
   (ix) MERKMAL:
      (A) NAME/SCHLÜSSEL: Modified-site
      (B) LAGE:502
      (D) SONSTIGE ANGABEN:/product= "Xaa ist Leu oder Pro"
         /label= Xaa38
   (ix) MERKMAL:
      (A) NAME/SCHLÜSSEL: Modified-site
      (B) LAGE:503
      (D) SONSTIGE ANGABEN:/product= "Xaa ist Ala oder Met"
         /label= Xaa39
   (ix) MERKMAL:
      (A) NAME/SCHLÜSSEL: Modified-site
      (B) LAGE:504
      (D) SONSTIGE ANGABEN:/product= "Xaa ist Met oder Val"
         /label= Xaa40
   (ix) MERKMAL:
      (A) NAME/SCHLÜSSEL: Modified-site
      (B) LAGE:533
      (D) SONSTIGE ANGABEN:/product= "Xaa ist Pro oder Phe"
         /label= Xaa41
   (xi) SEQUENZBESCHREIBUNG: SEQ ID NO: 1:
   Tyr Glu Arg Leu Arg Leu Arg Val Thr His Gln Thr Thr Gly Xaa Glu
   1 5 10 15
   Tyr Phe Arg Phe Ile Thr Leu Leu Arg Asp Tyr Val Ser Ser Gly Ser
   20 25 30
   Phe Ser Asn Glu Ile Pro Leu Leu Arg Gln Ser Thr Ile Pro Val Ser
   35 40 45
   Asp Ala Gln Arg Phe Val Leu Val Glu Leu Thr Asn Gln Gly Xaa Asp
   50 55 60
   Ser Xaa Thr Ala Ala Ile Asp Val Thr Asn Xaa Tyr Val Val Ala Tyr
   65 70 75 80
   Gln Ala Gly Asp Gln Ser Tyr Phe Leu Arg Asp Ala Pro Arg Gly Ala
   85 90 95
   Glu Thr His Leu Phe Thr Gly Thr Thr Arg Xaa Ser Ser Leu Pro Phe
               100                     105                     110
   Xaa Gly Ser Tyr Xaa Asp Leu Glu Arg Tyr Ala Gly His Arg Asp Gln
           115                 120                 125
   Ile Pro Leu Gly Ile Xaa Gln Leu Ile Gln Ser Val Xaa Ala Leu Arg
       130                 135                 140
   Xaa Pro Gly Gly Ser Thr Arg Xaa Gln Ala Arg Ser Ile Leu Ile Leu
   145                 150                 155                 160
   Ile Gln Met Ile Ser Glu Ala Ala Arg Phe Asn Pro Ile Leu Trp Arg
                   165                 170                 175
   Xaa Arg Gln Xaa Ile Asn Ser Gly Xaa Ser Phe Leu Pro Asp Xaa Tyr
               180                 185                 190
   Met Leu Glu Leu Glu Thr Ser Trp Gly Gln Gln Ser Thr Gln Val Gln
           195                 200                 205
   His Ser Thr Asp Gly Val Phe Asn Asn Pro Xaa Arg Leu Ala Ile Xaa
       210                 215                 220
   Xaa Gly Asn Phe Val Thr Leu Xaa Asn Val Arg Xaa Val Ile Ala Ser
   225                 230                 235                 240
   Leu Ala Ile Met Leu Phe Val Cys Gly Glu Arg Pro Ser Ser Ser Asp
                   245                 250                 255
   Val Arg Tyr Trp Pro Leu Val Ile Arg Pro Val Ile Ala Asp Asp Val
               260                 265                 270
   Thr Cys Ser Ala Ser Glu Pro Thr Val Arg Ile Val Gly Arg Xaa Gly
           275                 280                 285
   Met Xaa Val Asp Val Arg Asp Asp Asp Phe His Asp Gly Asn Gln Ile
       290                 295                 300
   Gln Leu Trp Pro Ser Lys Ser Asn Asn Asp Pro Asn Gln Leu Trp Thr
   305                 310                 315                 320
   Ile Lys Arg Asp Xaa Thr Ile Arg Ser Asn Gly Ser Cys Leu Thr Thr
                   325                 330                 335
   Tyr Gly Tyr Thr Ala Gly Val Tyr Val Met Ile Phe Asp Cys Asn Thr
               340                 345                 350
   Ala Val Arg Glu Ala Thr Ile Trp Gln Ile Trp Xaa Asn Gly Thr Ile
           355                 360                 365
   Ile Asn Pro Arg Ser Asn Leu Val Leu Ala Ala Ser Ser Gly Ile Lys
       370                 375                 380
   Gly Thr Thr Leu Thr Val Gln Thr Leu Asp Tyr Thr Leu Gly Gln Gly
   385                 390                 395                 400
   Trp Leu Ala Gly Asn Asp Thr Ala Pro Arg Glu Val Thr Ile Tyr Gly
                   405                 410                 415
   Phe Arg Asp Leu Cys Met Glu Ser Asn Xaa Gly Ser Val Trp Val Glu
               420                 425                 430
   Thr Cys Xaa Ser Ser Gln Xaa Asn Gln Xaa Xaa Trp Ala Leu Tyr Gly
           435                 440                 445
   Asp Gly Ser Ile Arg Pro Lys Gln Asn Gln Asp Gln Cys Leu Thr Xaa
       450                 455                 460
   Gly Arg Asp Ser Val Ser Thr Val Ile Asn Ile Val Ser Cys Ser Xaa
   465                 470                 475                     480
   Xaa Ser Xaa Xaa Gln Arg Trp Val Phe Thr Asn Glu Xaa Ala Ile Leu
                   485                 490                     495
   Asn Leu Lys Xaa Xaa Xaa Xaa Xaa Asp Val Ala Gln Ala Asn Pro Lys
               500                 505                 510
   Leu Arg Arg Ile Ile Ile Tyr Pro Ala Thr Gly Lys Pro Asn Gln Met
           515                 520                 525
   Trp Leu Pro Val Xaa
       530
(2) ANGABEN ZU SEQ ID NO: 2:
   (i) SEQUENZKENNZEICHEN:
      (A) LÄNGE: 255 Aminosäuren
      (B) ART: Aminosäure
      (C) STRANGFORM: Einzelstrang
      (D) TOPOLOGIE: linear
   (ii) ART DES MOLEKÜLS: Protein
   (ix) MERKMAL:
      (A) NAME/SCHLÜSSEL: Modified-site
      (B) LAGE:15
      (D) SONSTIGE ANGABEN:/product= "Xaa ist Asp oder Glu"
         /label= Xaa1
   (ix) MERKMAL:
      (A) NAME/SCHLÜSSEL: Modified-site
      (B) LAGE:63
      (D) SONSTIGE ANGABEN:/product= "Xaa ist Gly oder Gln"
         /label= Xaa2
   (ix) MERKMAL:
      (A) MAME/SCHLÜSSEL: Modified-site
      (B) LAGE:66
      (D) SONSTIGE ANGABEN:/product= "Xaa ist Ile oder Val"
         /label= Xaa3
   (ix) MERKMAL:
      (A) NAME/SCHLÜSSEL: Modified-site
      (B) LAGE:75
      (D) SONSTIGE ANGABEN :/product= "Xaa ist Leu oder Ala"
         /label= Xaa4
   (ix) MERKMAL:
      (A) NAME/SCHLÜSSEL: Modified-site
      (B) LAGE:107
      (D) SONSTIGE ANGABEN :/product= "Xaa ist Asp-Arg oder fehlt"
         /label= Xaa5
   (ix) MERKMAL:
      (A) NAME/SCHLÜSSEL: Modified-site
      (B) LAGE:113
      (D) SONSTIGE ANGABEN:/product= "Xaa ist Asn oder Thr"
         /label= Xaa6
   (ix) MERKMAL:
      (A) NAME/SCHLÜSSEL: Modified-site
      (B) LAGE:117
      (D) SONSTIGE ANGABEN:/product= "Xaa ist Pro oder Thr"
         /label= Xaa7
   (ix) MERKMAL:
      (A) NAME/SCHLÜSSEL: Modified-site
      (B) LAGE:134
      (D) SONSTIGE ANGABEN:/product= "Xaa ist Asp oder Glu"
         /label= Xaa8
   (ix) MERKMAL:
      (A) NAME/SCHLÜSSEL: Modified-site
      (B) LAGE:141
      (D) SONSTIGE ANGABEN:/product= "Xaa ist Ser oder Thr"
         /label= Xaa9
   (ix) MERKMAL:
      (A) NAME/SCHLÜSSEL: Modified-site
      (B) LAGE:145
      (D) SONSTIGE ANGABEN:/product= "Xaa ist Phe oder Tyr"
         /label= Xaa10
   (ix) MERKMAL:
      (A) NAME/SCHLÜSSEL: Modified-site
      (B) LAGE:152
      (D) SONSTIGE ANGABEN:/product= "Xaa ist Thr oder Ala"
         /label= Xaa11
   (ix) MERKMAL:
      (A) NAME/SCHLÜSSEL: Modified-site
      (B) LAGE:177
      (D) SONSTIGE ANGABEN:/product= "Xaa ist Ala oder Tyr"
         /label= Xaa12
   (ix) MERKMAL:
      (A) NAME/SCHLÜSSEL: Modified-site
      (B) LAGE:180
      (D) SONSTIGE ANGABEN:/product= "Xaa ist Tyr oder Asp"
         /label= Xaa13
   (ix) MERKMAL:
      (A) NAME/SCHLÜSSEL: Modified-site
      (B) LAGE:185
      (D) SONSTIGE ANGABEN:/product= "Xaa ist Ala oder Glu"
         /label= Xaa14
   (ix) MERKMAL:
      (A) NAME/SCHLÜSSEL: Modified-site
      (B) LAGE:191
      (D) SONSTIGE ANGABEN:/product= "Xaa ist Val oder Met"
         /label= Xaa15
   (ix) MERKMAL:
      (A) NAME/SCHLÜSSEL: Modified-site
      (B) LAGE:219
      (D) SONSTIGE ANGABEN:/product= "Xaa ist Ile oder Phe"
         /label= Xaa16
   (ix) MERKMAL:
      (A) NAME/SCHLÜSSEL: Modified-site
      (B) LAGE:224
      (D) SONSTIGE ANGABEN:/product= "Xaa ist Pro oder Ser"
         /label= Xaa17
   (ix) MERKMAL:
      (A) NAME/SCHLÜSSEL: Modified-site
      (B) LAGE:225
      (D) SONSTIGE ANGABEN:/product= "Xaa ist Pro oder Thr"
         /label= Xaa18
   (ix) MERKMAL:
      (A) NAME/SCHLÜSSEL: Modified-site
      (B) LAGE:232
      (D) SONSTIGE ANGABEN:/product= "Xaa ist Thr oder Ser"
         /label= Xaa19
   (ix) MERKMAL:
      (A) NAME/SCHLÜSSEL: Modified-site
      (B) LAGE:236
      (D) SONSTIGE ANGABEN:/product= "Xaa ist Asp oder Ser"
         /label= Xaa20
   (xi) SEQUENZBESCHREIBUNG: SEQ ID NO: 2:
   Tyr Glu Arg Leu Arg Leu Arg Val Thr His Gln Thr Thr Gly Xaa Glu
   1               5                   10                  15
   Tyr Phe Arg Phe Ile Thr Leu Leu Arg Asp Tyr Val Ser Ser Gly Ser
               20                  25                  30
   Phe Ser Asn Glu Ile Pro Leu Leu Arg Gln Ser Thr Ile Pro Val Ser
           35                  40                  45
   Asp Ala Gln Arg Phe Val Leu Val Glu Leu Thr Asn Gln Gly Xaa Asp
       50                  55                  60
   Ser Xaa Thr Ala Ala Ile Asp Val Thr Asn Xaa Tyr Val Val Ala Tyr
   65                  70                  75                  80
   Gln Ala Gly Asp Gln Ser Tyr Phe Leu Arg Asp Ala Pro Arg Gly Ala
                   85                  90                  95
   Glu Thr His Leu Phe Thr Gly Thr Thr Arg Xaa Ser Ser Leu Pro Phe
               100                 105                 110
   Xaa Gly Ser Tyr Xaa Asp Leu Glu Arg Tyr Ala Gly His Arg Asp Gln
           115                 120                 125
   Ile Pro Leu Gly Ile Xaa Gln Leu Ile Gln Ser Val Xaa Ala Leu Arg
       130                 135                 140
   Xaa Pro Gly Gly Ser Thr Arg Xaa Gln Ala Arg Ser Ile Leu Ile Leu
   145                 150                 155                 160
   Ile Gln Met Ile Ser Glu Ala Ala Arg Phe Asn Pro Ile Leu Trp Arg
                   165                 170                 175
   Xaa Arg Gln Xaa Ile Asn Ser Gly Xaa Ser Phe Leu Pro Asp Xaa Tyr
               180                 185                 190
   Met Leu Glu Leu Glu Thr Ser Trp Gly Gln Gln Ser Thr Gln Val Gln
           195                 200                 205
   His Ser Thr Asp Gly Val Phe Asn Asn Pro Xaa Arg Leu Ala Ile Xaa
       210                 215                 220
   Xaa Gly Asn Phe Val Thr Leu Xaa Asn Val Arg Xaa Val Ile Ala Ser
   225                 230                 235                 240
   Leu Ala Ile Met Leu Phe Val Cys Gly Glu Arg Pro Ser Ser Ser
                   245                 250                 255
(2) ANGABEN ZU SEQ ID NO: 3:
   (i) SEQUENZKENNZEICHEN;
      (A) LÄNGE: 264 Aminosäuren
      (B) ART: Aminosäure
      (C) STRANGFORM: Einzelstrang
      (D) TOPOLOGIE: linear
   (ii) ART DES MOLEKÜLS: Protein
   (ix) MERKMAL:
      (A) NAME/SCHLÜSSEL: Modified-site
      (B) LAGE:18
      (D) SONSTIGE ANGABEN:/product= "Xaa ist Asn oder Ser"
         /label= Xaa1
   (ix) MERKMAL:
      (A) NAME/SCHLÜSSEL: Modified-site
      (B) LAGE:21
      (D) SONSTIGE ANGABEN:/product= "Xaa ist Cys oder Arg"
         /label= X2
   (ix) MERKMAL:
      (A) NAME/SCHLÜSSEL: Modified-site
      (B) LAGE:56
      (D) SONSTIGE ANGABEN: /product= "Xaa ist Gly oder Asn"
         /label= Xaa3
   (ix) MERKMAL:
      (A) NAME/SCHLÜSSEL: Modified-site
      (B) LAGE:95
      (D) SONSTIGE ANGABEN:/product= "Xaa ist Gly oder Asp"
         /label= Xaa4
   (ix) MERKMAL:
      (A) NAME/SCHLÜSSEL: Modified-site
      (B) LAGE:157
      (D) SONSTIGE ANGABEN:/produce= "Xaa ist Gly oder Gln"
         /label= Xaa5
   (ix) MERKMAL:
      (A) NAME/SCHLÜSSEL: Modified-site
      (B) LAGE:166
      (D) SONSTIGE ANGABEN:/product= "Xaa ist Val oder Asp"
         /label= Xaa6
   (ix) MERKMAL:
      (A) NAME/SCHLÜSSEL: Modified-site
      (B) LAGE:170
      (D) SONSTIGE ANGABEN:/product= "Xaa ist Gln oder Lys"
         /label= Xaa7
   (ix) MERKMAL:
      (A) NAME/SCHLÜSSEL: Modified-site
      (B) LAGE:173
      (D) SONSTIGE ANGABEN:/product= "Xaa ist Gly oder fehlt"
         /label= Xaa8
   (ix) MERKMAL:
      (A) NAME/SCHLÜSSEL: Modified-site
      (B) LAGE:1.74
      (D) SONSTIGE ANGABEN:/product= "Xaa ist Arg oder Lys"
         /label= Xaa9
   (ix) MERKMAL:
      (A) NAME/SCHLÜSSEL: Modified-site
      (B) LAGE:195
      (D) SONSTIGE ANGABEN:/product= "Xaa ist Cys oder Ser oder Val"
         /label= Xaa10
   (ix) MERKMAL:
      (A) NAME/SCHLÜSSEL: Modified-site
      (B) LAGE:211
      (D) SONSTIGE ANGABEN:/product= "Xaa ist ala oder Gly"
         /label= Xaa11
   (ix) MERKMAL:
      (A) NAME/SCHLÜSSEL: Modified-site
      (B) LAGE:212
      (D) SONSTIGE ANGABEN:/product= "Xaa ist Gly oder Ala"
         /label= Xaa12
   (ix) MERKMAL:
      (A) NAME/SCHLÜSSEL: Modified-site
      (B) LAGE:214
      (D) SONSTIGE ANGABEN:/product= "Xaa ist Ser oder Gly"
         /label= Xaa13
   (ix) MERKMAL:
      (A) NAME/SCHLÜSSEL: Modified-site
      (B) LAGE:215
      (D) SONSTIGE ANGABEN:/product= "Xaa ist Gly oder Ser"
         /label= Xaa14
   (ix) MERKMAL:
      (A) NAME/SCHLÜSSEL: Modified-site
      (B) LAGE:224
      (D) SONSTIGE ANGABEN:/product= "Xaa ist Gly oder Tyr"
         /label= Xaa15
   (ix) MERKMAL:
      (A) NAME/SCHLÜSSEL: Modified-site
      (B) LAGE:231
      (D) SONSTIGE ANGABEN:/product= "Xaa ist Asn oder Ser oder Thr oder Lys"
         /label= Xaa16
   (ix) MERKMAL:
      (A) NAME/SCHLÜSSEL: Modified-site
      (B) LAGE:232
      (D) SONSTIGE ANGABEN :/product= "Xaa ist Ser oder Gly"
         /label= Xaa17
   (ix) MERKMAL:
      (A) NAME/SCHLÜSSEL: Modified-site
      (B) LAGE:233
      (D) SONSTIGE ANGABEN:/product= "Xaa ist Leu oder Pro"
         /label= Xaa17
   (ix) MERKMAL:
      (A) NAME/SCHLÜSSEL: Modified-site
      (B) LAGE:234
      (D) SONSTIGE ANGABEN:/product= "Xaa ist Ala oder Met"
         /label= Xaa19
   (ix) MERKMAL:
      (A) NAME/SCHLÜSSEL: Modified-site
      (B) LAGE:235
      (D) SONSTIGE ANGABEN:/product= "Xaa ist Met oder Val"
         /label= Xaa20
   (ix) MERKMAL:
      (A) NAME/SCHLÜSSEL: Modified-site
      (B) LAGE:264
      (D) SONSTIGE ANGABEN:/product= "Xaa ist Pro oder Phe"
         /label= Xaa21
   (xi) SEQUENZBESCHREIBUNG: SEQ ID NO: 3:
   Asp Asp Val Thr Cys Ser Ala Ser Glu Pro Thr Val Arg Ile Val Gly
   1               5                   10                  15
   Arg Xaa Gly Met Xaa Val Asp Val Arg Asp Asp Asp Phe His Asp Gly
               20                  25                  30
   Asn Gln Ile Gln Leu Trp Pro Ser Lys Ser Asn Asn Asp Pro Asn Gln
           35                  40                  45
   Leu Trp Thr Ile Lys Arg Asp Xaa Thr Ile Arg Ser Asn Gly Ser Cys
       50                  55                  60
   Leu Thr Thr Tyr Gly Tyr Thr Ala Gly Val Tyr Val Met Ile Phe Asp
   65                  70                  75                  80
   Cys Asn Thr Ala Val Arg Glu Ala Thr Ile Trp Gln Ile Trp Xaa Asn
                   85                  90                  95
   Gly Thr Ile Ile Asn Pro Arg Ser Asn Leu Val Leu Ala Ala Ser Ser
               100                 105                 110
   Gly Ile Lys Gly Thr Thr Leu Thr Val Gln Thr Leu Asp Tyr Thr Leu
           115                 120                 125
   Gly Gln Gly Trp Leu Ala Gly Asn Asp Thr Ala Pro Arg Glu Val Thr
       130                 135                 140
   Ile Tyr Gly Phe Arg Asp Leu Cys Met Glu Ser Asn Xaa Gly Ser Val
   145                 150                 155                 160
   Trp Val Glu Thr Cys Xaa Ser Ser Gln Xaa Asn Gln Xaa Xaa Trp Ala
                   165                 170                 175
   Leu Tyr Gly Asp Gly Ser Ile Arg Pro Lys Gln Asn Gln Asp Gln Cys
               180                 185                 190
   Leu Thr Xaa Gly Arg Asp Ser Val Ser Thr Val Ile Asn Ile Val Ser
           195                 200                 205
   Cys Ser Xaa Xaa Ser Xaa Xaa Gln Arg Trp Val Phe Thr Asn Glu Xaa
       210                 215                 220
   Ala Ile Leu Asn Leu Lys Xaa Xaa Xaa Xaa Xaa Asp Val Ala Gln Ala
   225                 230                 235                 240
   Asn Pro Lys Leu Arg Arg Ile Ile Ile Tyr Pro Ala Thr Gly Lys Pro
                   245                 250                 255
   Asn Gln Met Trp Leu Pro Val Xaa
               260
(2) ANGABEN ZU SEQ ID NO: 4:
   (i) SEQUENZKENNZEICHEN :
      (A) LÄNGE: 531 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 4:
   Tyr Glu Arg Leu Arg Leu Arg Val Thr His Gln Thr Thr Gly Glu Glu
   1 5 10 15
   Tyr Phe Arg Phe Ile Thr Leu Leu Arg Asp Tyr Val Ser Ser Gly Ser
   20 25 30
   Phe Ser Asn Glu Ile Pro Leu Leu Arg Gln Ser Thr Ile Pro Val Ser
   35 40 45
   Asp Ala Gln Arg Phe Val Leu Val Glu Leu Thr Asn Gln Gly Gly Asp
   50 55 60
   Ser Ile Thr Ala Ala Ile Asp Val Thr Asn Leu Tyr Val Val Ala Tyr
   65 70 75 80
   Gln Ala Gly Asp Gln Ser Tyr Phe Leu Arg Asp Ala Pro Arg Gly Ala
   85 90 95
   Glu Thr His Leu Phe Thr Gly Thr Thr Arg Ser Ser Leu Pro Phe Asn
   100 105 110
   Gly Ser Tyr Pro Asp Leu Glu Arg Tyr Ala Gly His Arg Asp Gln Ile
   115 120 125
   Pro Leu Gly Ile Asp Gln Leu Ile Gln Ser Val Thr Ala Leu Arg Phe
   130 135 140
   Pro Gly Gly Ser Thr Arg Thr Gln Ala Arg Ser Ile Leu Ile Leu Ile
   145 150 155 160
   Gln Met Ile Ser Glu Ala Ala Arg Phe Asn Pro Ile Leu Trp Arg Ala
   165 170 175
   Arg Gln Tyr Ile Asn Ser Gly Ala Ser Phe Leu Pro Asp Val Tyr Met
   180 185 190
   Leu Glu Leu Glu Thr Ser Trp Gly Gln Gln Ser Thr Gln Val Gln His
   195 200 205
   Ser Thr Asp Gly Val Phe Asn Asn Pro Ile Arg Leu Ala Ile Pro Pro
   210 215 220
   Gly Asn Phe Val Thr Leu Thr Asn Val Arg Asp Val Ile Ala Ser Leu
   225 230 235 240
   Ala Ile Met Leu Phe Val Cys Gly Glu Arg Pro Ser Ser Ser Asp Val
   245 250 255
   Arg Tyr Trp Pro Leu Val Ile Arg Pro Val Ile Ala Asp Asp Val Thr
   260 265 270
   Cys Ser Ala Ser Glu Pro Thr Val Arg Ile Val Gly Arg Asn Gly Met
   275 280 285
   Cys Val Asp Val Arg Asp Asp Asp Phe His Asp Gly Asn Gln Ile Gln
   290 295 300
   Leu Trp Pro Ser Lys Ser Asn Asn Asp Pro Asn Gln Leu Trp Thr Ile
   305 310 315 320
   Lys Arg Asp Gly Thr Ile Arg Ser Asn Gly Ser Cys Leu Thr Thr Tyr
   325 330 335
   Gly Tyr Thr Ala Gly Val Tyr Val Met Ile Phe Asp Cys Asn Thr Ala
   340 345 350
   Val Arg Glu Ala Thr Ile Trp Gln Ile Trp Gly Asn Gly Thr Ile Ile
   355 360 365
   Asn Pro Arg Ser Asn Leu Val Leu Ala Ala Ser Ser Gly Ile Lys Gly
   370 375 380
   Thr Thr Leu Thr Val Gln Thr Leu Asp Tyr Thr Leu Gly Gln Gly Trp
   385 390 395 400
   Leu Ala Gly Asn Asp Thr Ala Pro Arg Glu Val Thr Ile Tyr Gly Phe
                   405                 410                 415
   Arg Asp Leu Cys Met Glu Ser Asn Gly Gly Ser Val Trp Val Glu Thr
               420                 425                 430
   Cys Val Ser Ser Gln Gln Asn Gln Arg Trp Ala Leu Tyr Gly Asp Gly
           435                 440                 445
   Ser Ile Arg Pro Lys Gln Asn Gln Asp Gln Cys Leu Thr Cys Gly Arg
       450                 455                 460
   Asp Ser Val Ser Thr Val Ile Asn Ile Val Ser Cys Ser Ala Gly Ser
   465                 470                 475                 480
   Ser Gly Gln Arg Trp Val Phe Thr Asn Glu Gly Ala Ile Leu Asn Leu
                   485                 490                 495
   Lys Asn Gly Leu Ala Met Asp Val Ala Gln Ala Asn Pro Lys Leu Arg
               500                 505                 510
   Arg Ile Ile Ile Tyr Pro Ala Thr Gly Lys Pro Asn Gln Met Trp Leu
           515                 520                 525
   Pro Val Pro
       530
(2) ANGABEN ZU SEQ ID NO: 5:
   (i) SEQUENZKENNZEICHEN:
      (A) LÄNGE: 256 Aminosäuren
      (B) ART: Aminosäure
      (C) STRANGFORM: Einzelstrang
      (D) TOPOLOGIE: linear
   (ii) ART DES MOLEKULS: Protein
   (xi) SEQUENZBESCHREIBUNG: SEQ ID NO: 5:
   Tyr Glu Arg Leu Arg Leu Arg Val Thr His Gln Thr Thr Gly Asp Glu
   1               5                   10                  15
   Tyr Phe Arg Phe Ile Thr Leu Leu Arg Asp Tyr Val Ser Ser Gly Ser
               20                  25                  30
   Phe Ser Asn Glu Ile Pro Leu Leu Arg Gln Ser Thr Ile Pro Val Ser
           35                  40                  45
   Asp Ala Gln Arg Phe Val Leu Val Glu Leu Thr Asn Gln Gly Gln Asp
       50                  55                  60
   Ser Ile Thr Ala Ala Ile Asp Val Thr Asn Ala Tyr Val Val Ala Tyr
   65                  70                  75                  80
   Gln Ala Gly Asp Gln Ser Tyr Phe Leu Arg Asp Ala Pro Arg Gly Ala
                   85                  90                  95
   Glu Thr His Leu Phe Thr Gly Thr Thr Arg Asp Arg Ser Ser Leu Pro
   100 105 110
   Phe Thr Gly Ser Tyr Thr Asp Leu Glu Arg Tyr Ala Gly His Arg Asp
   115 120 125
   Gln Ile Pro Leu Gly Ile Glu Gln Leu Ile Gln Ser Val Ser Ala Leu
   130 135 140
   Arg Tyr Pro Gly Gly Ser Thr Arg Ala Gln Ala Arg Ser Ile Leu Ile
   145 150 155 160
   Leu Ile Gln Met Ile Ser Glu Ala Ala Arg Phe Asn Pro Ile Leu Trp
   165 170 175
   Arg Tyr Arg Gln Asp Ile Asn Ser Gly Glu Ser Phe Leu Pro Asp Met
   180 185 190
   Tyr Met Leu Glu Leu Glu Thr Ser Trp Gly Gln Gln Ser Thr Gln Val
   195 200 205
   Gln His Ser Thr Asp Gly Val Phe Asn Asn Pro Phe Arg Leu Ala Ile
   210 215 220
   Ser Thr Gly Asn Phe Val Thr Leu Ser Asn Val Arg Ser Val Ile Ala
   225 230 235 240
   Ser Leu Ala Ile Met Leu Phe Val Cys Gly Glu Arg Pro Ser Ser Ser
   245 250 255
(2) ANGABEN ZU SEQ ID NO: 6:
   (i) SEQUENZKENNZEICHEN:
      (A) LÄNGE: 263 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 6:
   Asp Asp Val Thr Cys Ser Ala Ser Glu Pro Thr Val Arg Ile Val Gly
   1 5 10 15
   Arg Asn Gly Met Cys Val Asp Val Arg Asp Asp Asp Phe His Asp Gly
   20 25 30
   Asn Gln Ile Gln Leu Trp Pro Ser Lys Ser Asn Asn Asp Pro Asn Gln
   35 40 45
   Leu Trp Thr Ile Lys Arg Asp Gly Thr Ile Arg Ser Asn Gly Ser Cys
   50 55 60
   Leu Thr Thr Tyr Gly Tyr Thr Ala Gly Val Tyr Val Met Ile Phe Asp
   65 70 75 80
   Cys Asn Thr Ala Val Arg Glu Ala Thr Ile Trp Gln Ile Trp Gly Asn
                   85                  90                  95
   Gly Thr Ile Ile Asn Pro Arg Ser Asn Leu Val Leu Ala Ala Ser Ser
               100                 105                 110
   Gly Ile Lys Gly Thr Thr Leu Thr Val Gln Thr Leu Asp Tyr Thr Leu
           115                 120                 125
   Gly Gln Gly Trp Leu Ala Gly Asn Asp Thr Ala Pro Arg Glu Val Thr
       130                 135                 140
   Ile Tyr Gly Phe Arg Asp Leu Cys Met Glu Ser Asn Gly Gly Ser Val
   145                 150                 155                 160
   Trp Val Glu Thr Cys Val Ser Ser Gln Gln Asn Gln Arg Trp Ala Leu
                   165                 170                 175
   Tyr Gly Asp Gly Ser Ile Arg Pro Lys Gln Asn Gln Asp Gln Cys Leu
               180                 185                 190
   Thr Cys Gly Arg Asp Ser Val Ser Thr Val Ile Asn Ile Val Ser Cys
           195                 200                 205
   Ser Ala Gly Ser Ser Gly Gln Arg Trp Val Phe Thr Asn Glu Gly Ala
       210                 215                 220
   Ile Leu Asn Leu Lys Asn Gly Leu Ala Met Asp Val Ala Gln Ala Asn
   225                 230                 235                 240
   Pro Lys Leu Arg Arg Ile Ile Ile Tyr Pro Ala Thr Gly Lys Pro Asn
                   245                 250                 255
   Gln Met Trp Leu Pro Val Pro
               260
(2) ANGABEN ZU SEQ ID NO: 7:
   (i) SEQUENZKENNZEICHEN:
      (A) LÄNGE: 264 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 7:
   Asp Asp Val Thr Cys Ser Ala Ser Glu Pro Thr Val Arg Ile Val Gly
   1 5 10 15
   Arg Asn Gly Met Arg Val Asp Val Arg Asp Asp Asp Phe His Asp Gly
   20 25 30
   Asn Gln Ile Gln Leu Trp Pro Ser Lys Ser Asn Asn Asp Pro Asn Gln
   35 40 45
   Leu Trp Thr Ile Lys Arg Asp Gly Thr Ile Arg Ser Asn Gly Ser Cys
   50 55 60
   Leu Thr Thr Tyr Gly Tyr Thr Ala Gly Val Tyr Val Met Ile Phe Asp
   65 70 75 80
   Cys Asn Thr Ala Val Arg Glu Ala Thr Ile Trp Gln Ile Trp Asp Asn
   85 90 95
   Gly Thr Ile Ile Asn Pro Arg Ser Asn Leu Val Leu Ala Ala Ser Ser
   100 105 110
   Gly Ile Lys Gly Thr Thr Leu Thr Val Gln Thr Leu Asp Tyr Thr Leu
   115 120 125
   Gly Gln Gly Trp Leu Ala Gly Asn Asp Thr Ala Pro Arg Glu Val Thr
   130 135 140
   Ile Tyr Gly Phe Arg Asp Leu Cys Met Glu Ser Asn Gly Gly Ser Val
   145 150 155 160
   Trp Val Glu Thr Cys Asp Ser Ser Gln Lys Asn Gln Gly Lys Trp Ala
   165 170 175
   Leu Tyr Gly Asp Gly Ser Ile Arg Pro Lys Gln Asn Gln Asp Gln Cys
   180 185 190
   Leu Thr Ser Gly Arg Asp Ser Val Ser Thr Val Ile Asn Ile Val Ser
   195 200 205
   Cys Ser Gly Ala Ser Gly Ser Gln Arg Trp Val Phe Thr Asn Glu Gly
   210 215 220
   Ala Ile Leu Asn Leu Lys Asn Gly Leu Ala Met Asp Val Ala Gln Ala
   225 230 235 240
   Asn Pro Lys Leu Arg Arg Ile Ile Ile Tyr Pro Ala Thr Gly Lys Pro
   245 250 255
   Asn Gln Met Trp Leu Pro Val Phe
   260
(2) ANGABEN ZU SEQ ID NO: 8:
   (i) SEQUENZKENNZEICHEN:
      (A) LÄNGE: 264 Aminosäuren
      (B) ART: Aminosäure
      (C) STRANGFORM: Einzelstrang
      (D) TOPOLOGIE: linear
   (ii) ART DES MOLEKÜLS; Protein
   (xi) SEQUENZBESCHREIBUNG: SEQ ID NO: 8:
   Asp Asp Val Thr Cys Ser Ala Ser Glu Pro Thr Val Arg Ile Val Gly
   1               5                   10                  15
   Arg Ser Gly Met Arg Val Asp Val Arg Asp Asp Asp Phe His Asp Gly
               20                  25                  30
   Asn Gln Ile Gln Leu Trp Pro Ser Lys Ser Asn Asn Asp Pro Asn Gln
           35                  40                  45
   Leu Trp Thr Ile Lys Arg Asp Asn Thr Ile Arg Ser Asn Gly Ser Cys
       50                  55                  60
   Leu Thr Thr Tyr Gly Tyr Thr Ala Gly Val Tyr Val Met Ile Phe Asp
   65                  70                  75                  80
   Cys Asn Thr Ala Val Arg Glu Ala Thr Ile Trp Gln Ile Trp Asp Asn
                   85                  90                  95
   Gly Thr Ile Ile Asn Pro Arg Ser Asn Leu Val Leu Ala Ala Ser Ser
               100                 105                 110
   Gly Ile Lys Gly Thr Thr Leu Thr Val Gln Thr Leu Asp Tyr Thr Leu
           115                 120                 125
   Gly Gln Gly Trp Leu Ala Gly Asn Asp Thr Ala Pro Arg Glu Val Thr
       130                 135                 140
   Ile Tyr Gly Phe Arg Asp Leu Cys Met Glu Ser Asn Gln Gly Ser Val
   145                 150                 155                 160
   Trp Val Glu Thr Cys Asp Ser Ser Gln Lys Asn Gln Gly Lys Trp Ala
                   165                 170                 175
   Leu Tyr Gly Asp Gly Ser Ile Arg Pro Lys Gln Asn Gln Asp Gln Cys
               180                 185                 190
   Leu Thr Val Gly Arg Asp Ser Val Ser Thr Val Ile Asn Ile Val Ser
           195                 200                 205
   Cys Ser Gly Ala Ser Gly Ser Gln Arg Trp Val Phe Thr Asn Glu Tyr
       210                 215                 220
   Ala Ile Leu Asn Leu Lys Ser Gly Leu Ala Met Asp Val Ala Gln Ala
   225                 230                 235                 240
   Asn Pro Lys Leu Arg Arg Ile Ile Ile Tyr Pro Ala Thr Gly Lys Pro
                   245                 250                 255
   Asn Gln Met Trp Leu Pro Val Phe
               260
(2) ANGABEN ZU SEQ ID NO: 9:
   (i) SEQUENZKENNZEICHEN:
      (A) LÄNGE: 264 Aminosäuren.
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 9:
   Asp Asp Val Thr Cys Ser Ala Ser Glu Pro Thr Val Arg Ile Val Gly
   1               5                   10                  15
   Arg Asn Gly Met Arg Val Asp Val Arg Asp Asp Asp Phe His Asp Gly
               20                  25                  30
   Asn Gln Ile Gln Leu Trp Pro Ser Lys Ser Asn Asn Asp Pro Asn Gln
           35                  40                  45
   Leu Trp Thr Ile Lys Arg Asp Gly Thr Ile Arg Ser Asn Gly Ser Cys
       50                  55                  60
   Leu Thr Thr Tyr Gly Tyr Thr Ala Gly Val Tyr Val Met Ile Phe Asp
   65                  70                  75                  80
   Cys Asn Thr Ala Val Arg Glu Ala Thr Ile Trp Gln Ile Trp Asp Asn
                   85                  90                  95
   Gly Thr Ile Ile Asn Pro Arg Ser Asn Leu Val Leu Ala Ala Ser Ser
               100                 105                 110
   Gly Ile Lys Gly Thr Thr Leu Thr Val Gln Thr Leu Asp Tyr Thr Leu
           115                 120                 125
   Gly Gln Gly Trp Leu Ala Gly Asn Asp Thr Ala Pro Arg Glu Val Thr
       130                 135                 140
   Ile Tyr Gly Phe Arg Asp Leu Cys Met Glu Ser Asn Gly Gly Ser Val
   145                 150                 155                 160
   Trp Val Glu Thr Cys Asp Ser Ser Gln Lys Asn Gln Gly Lys Trp Ala
                   165                 170                 175
   Leu Tyr Gly Asp Gly Ser Ile Arg Pro Lys Gln Asn Gln Asp Gln Cys
               180                 185                 190
   Leu Thr Ser Gly Arg Asp Ser Val Ser Thr Val Ile Asn Ile Val Ser
           195                 200                 205
   Cys Ser Gly Ala Ser Gly Ser Gln Arg Trp Val Phe Thr Asn Glu Gly
       210                 215                 220
   Ala Ile Leu Asn Leu Lys Thr Gly Leu Ala Met Asp Val Ala Gln Ala
   225                 230                 235                 240
   Asn Pro Lys Leu Arg Arg Ile Ile Ile Tyr Pro Ala Thr Gly Lys Pro
                   245                 250                 255
   Asn Gln Met Trp Leu Pro Val Phe
               260
(2) ANGABEN ZU SEQ ID NO: 10:
   (i) SEQUENZKENNZEICHEN:
      (A) LÄNGE: 264 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 10:
   Asp Asp Val Thr Cys Ser Ala Ser Glu Pro Thr Val Arg Ile Val Gly
   1               5                   10                  15
   Arg Asn Gly Met Arg Val Asp Val Arg Asp Asp Asp Phe His Asp Gly
               20                  25                  30
   Asn Gln Ile Gln Leu Trp Pro Ser Lys Ser Asn Asn Asp Pro Asn Gln
           35                  40                  45
   Leu Trp Thr Ile Lys Arg Asp Gly Thr Ile Arg Ser Asn Gly Ser Cys
       50                  55                  60
   Leu Thr Thr Tyr Gly Tyr Thr Ala Gly Val Tyr Val Met Ile Phe Asp
   65                  70                  75                  80
   Cys Asn Thr Ala Val Arg Glu Ala Thr Ile Trp Gln Ile Trp Asp Asn
                   85                  90                  95
   Gly Thr Ile Ile Asn Pro Arg Ser Asn Leu Val Leu Ala Ala Ser Ser
               100                 105                 110
   Gly Ile Lys Gly Thr Thr Leu Thr Val Gln Thr Leu Asp Tyr Thr Leu
           115                 120                 125
   Gly Gln Gly Trp Leu Ala Gly Asn Asp Thr Ala Pro Arg Glu Val Thr
       130                 135                 140
   Ile Tyr Gly Phe Arg Asp Leu Cys Met Glu Ser Asn Gly Gly Ser Val
   145                 150                 155                 160
   Trp Val Glu Thr Cys Asp Ser Ser Gln Lys Asn Gln Gly Lys Trp Ala
                   165                 170                 175
   Leu Tyr Gly Asp Gly Ser Ile Arg Pro Lys Gln Asn Gln Asp Gln Cys
               180                 185                 190
   Leu Thr Ser Gly Arg Asp Ser Val Ser Thr Val Ile Asn Ile Val Ser
           195                 200                 205
   Cys Ser Gly Ala Ser Gly Ser Gln Arg Trp Val Phe Thr Asn Glu Gly
       210                 215                 220
   Ala Ile Leu Asn Leu Lys Lys Gly Pro Ala Met Asp Val Ala Gln Ala
   225                 230                 235                 240
   Asn Pro Lys Leu Arg Arg Ile Ile Ile Tyr Pro Ala Thr Gly Lys Pro
                   245                 250                 255
   Asn Gln Met Trp Leu Pro Val Phe
               260
(2) ANGABEN ZU SEQ ID NO: 11:
   (i) SEQUENZKENNZEICHEN:
      (A) LÄNGE: 264 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 11:
   Asp Asp Val Thr Cys Ser Ala Ser Glu Pro Thr Val Arg Ile Val Gly
   1               5                   10                  15
   Arg Asn Gly Met Arg Val Asp Val Arg Asp Asp Asp Phe His Asp Gly
               20                  25                  30
   Asn Gln Ile Gln Leu Trp Pro Ser Lys Ser Asn Asn Asp Pro Asn Gln
           35                  40                  45
   Leu Trp Thr Ile Lys Arg Asp Gly Thr Ile Arg Ser Asn Gly Ser Cys
       50                  55                  60
   Leu Thr Thr Tyr Gly Tyr Thr Ala Gly Val Tyr Val Met Ile Phe Asp
   65                  70                  75                  80
   Cys Asn Thr Ala Val Arg Glu Ala Thr Ile Trp Gln Ile Trp Asp Asn
                   85                  90                  95
   Gly Thr Ile Ile Asn Pro Arg Ser Asn Leu Val Leu Ala Ala Ser Ser
               100                 105                 110
   Gly Ile Lys Gly Thr Thr Leu Thr Val Gln Thr Leu Asp Tyr Thr Leu
           115                 120                 125
   Gly Gln Gly Trp Leu Ala Gly Asn Asp Thr Ala Pro Arg Glu Val Thr
       130                 135                 140
   Ile Tyr Gly Phe Arg Asp Leu Cys Met Glu Ser Asn Gly Gly Ser Val
   145                 150                 155                 160
   Trp Val Glu Thr Cys Asp Ser Ser Gln Lys Asn Gln Gly Lys Trp Ala
                   165                 170                 175
   Leu Tyr Gly Asp Gly Ser Ile Arg Pro Lys Gln Asn Gln Asp Gln Cys
               180                 185                 190
   Leu Thr Ser Gly Arg Asp Ser Val Ser Thr Val Ile Asn Ile Val Ser
           195                 200                 205
   Cys Ser Gly Ala Ser Gly Ser Gln Arg Trp Val Phe Thr Asn Glu Gly
       210                 215                 220
   Ala Ile Leu Asn Leu Lys Asn Ser Leu Met Val Asp Val Ala Gln Ala
   225                 230                 235                 240
   Asn Pro Lys Leu Arg Arg Ile Ile Ile Tyr Pro Ala Thr Gly Lys Pro
                   245                 250                 255
   Asn Gln Met Trp Leu Pro Val Phe
               260
(2) ANGABEN ZU SEQ ID NO: 12:
   (i) SEQUENZKENNZEICHEN:
      (A) LANGE: 1598 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (ix) MERKMAL:
      (A) NAME/SCHLOSSEL: misc_feature
      (B) LAGE:319
      (D) SONSTIGE ANGABEN:/product= "N ist GAT AGA oder fehlt"
         /label= Z1
   (ix) MERKMAL:
      (A) NAME/SCHLÜSSEL: misc_feature
      (B) LAGE:1322
      (D) SONSTIGE ANGABEN:/product= "N ist GGC oder fehlt"
         /label= Z2
   (xi) SEQUENZBESCHREIBUNG: SEQ ID NO: 12:
   TACGAGAGGC TAAGACTCAG AGTTACGCAT CAAACCACGG GCGAKGAATA CTTCCGGTTC     60
   ATCACGCTTC TCCGAGATTA TGTCTCAAGC GGAAGCTTTT CCAATGAGAT ACCACTCTTG    120
   CGTCAGTCTA CGATCCCCGT CTCCGATGCG CAAAGATTTG TCTTGGTGGA GCTCACCAAC    180
   CAGGGGSRRG ACTCGRTYAC GGCCGCCATC GACGTTACCA ATSYKTACGT CGTGGCTTAC    240
   CAAGCAGGCG ACCAATCCTA CTTTTTGCGC GACGCACCAC GCGGCGCGGA AACGCACCTC    300
   TTCACCGGCA CCACCCGANT CCTCTCTCCC ATTCAMYGGA AGCTACMCYG ATCTGGAGCG    360
   ATACGCCGGA CATAGGGACC AGATCCCTCT CGGTATAGAS CAACTCATTC AATCCGTCWC    420
   KGCGCTTCGT TWYCCGGGCG GCAGCACGCG TRCYCAAGCT CGTTCGATTT TAATCCTCAT    480
   TCAGATGATC TCCGAGGCCG CCAGATTCAA TCCCATCTTA TGGAGGKMYC GCCAAKAYAT    540
   TAACAGTGGG GMRTCATTTC TGCCAGACRT GTACATGCTG GAGCTGGAGA CGAGTTGGGG    600
   CCAACAATCC ACGCAAGTCC AGCATTCAAC CGATGGCGTT TTTAATAACC CAWTYCGGTT    660
   GGCTATAYCY MCYGGTAACT TCGTGACGTT GWCYAATGTT CGCKMYGTGA TCGCCAGCTT    720
   GGCGATCATG TTGTTTGTAT GCGGAGAGCG GCCATCTTCC TCTGACGTGC GCTATTGGCC    780
   GCTGGTCATA CGACCCGTGA TAGCCGATGA TGTTACCTGC AGTGCTTCGG AACCTACGGT    840
   GCGGATTGTG GGTCGAARTG GCATGYGCGT GGACGTCCGA GATGACGATT TCCACGATGG    900
   GAATCAGATA CAGTTGTGGC CCTCCAAGTC CAACAATGAT CCGAATCAGT TGTGGACGAT    960
   CAAAAGGGAT RRMACCATTC GATCCAATGG CAGCTGCTTG ACCACGTATG GCTATACTGC   1020
   TGGCGTCTAT GTGATGATCT TCGACTGTAA TACTGCTGTG CGGGAGGCCA CTATTTGGCA 1080
   GATATGGGRC AATGGGACCA TCATCAATCC AAGATCCAAT CTGGTTTTGG CAGCATCATC 1140
   TGGAATCAAA GGCACTACGC TTACGGTGCA AACACTGGAT TACACGTTGG GACAGGGCTG 1200
   GCTTGCCGGT AATGATACCG CCCCACGCGA GGTGACCATA TATGGTTTCA GGGACCTTTG 1260
   CATGGAATCA AATSRAGGGA GTGTGTGGGT GGAGACGTGC GWSAGTAGCC AAMAGAACCA 1320
   ANARATGGGC TTTGTACGGG GATGGTTCTA TACGCCCCAA ACAAAACCAA GACCAATGCC 1380
   TCACCKBTGG GAGAGACTCC GTTTCAACAG TAATCAATAT AGTTAGCTGC AGCGSWGSWT 1440
   CGKSKKSKCA GCGATGGGTG TTTACCAATG AAKRSGCCAT TTTGAATTTA AAGAVWRGSY 1500
   YGRYSRTGGA TGTGGCGCAA GCAAATCCAA AGCTCCGCCG AATAATTATC TATCCTGCCA 1560
   CAGGAAAACC AAATCAAATG TGGCTTCCCG TGYYMTGA 1598
(2) ANGABEN ZU SEQ ID NO: 13:
   (i) SEQUENZKENNZEICHEN:
      (A) LÄNGE: 763 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (ix) MERKMAL:
      (A) NAME/SCHLÜSSEL: misc_feature
      (B) LAGE:319
      (D) SONSTIGE ANGABEN:/product= "N ist GAT AGA oder fehlt"
         /label= Z1
   (xi) SEQUENZBESCHREIBUNG: SEQ ID NO: 13:
   TACGAGAGGC TAAGACTCAG AGTTACGCAT CAAACCACGG GCGAKGAATA CTTCCGGTTC 60
   ATCACGCTTC TCCGAGATTA TGTCTCAAGC GGAAGCTTTT CCAATGAGAT ACCACTCTTG 120
   CGTCAGTCTA CGATCCCCGT CTCCGATGCG CAAAGATTTG TCTTGGTGGA GCTCACCAAC 180
   CAGGGGSRRG ACTCGRTYAC GGCCGCCATC GACGTTACCA ATSYKTACGT CGTGGCTTAC 240
   CAAGCAGGCG ACCAATCCTA CTTTTTGCGC GACGCACCAC GCGGCGCGGA AACGCACCTC 300
   TTCACCGGCA CCACCCGANT CCTCTCTCCC ATTCAMYGGA AGCTACMCYG ATCTGGAGCG 360
   ATACGCCGGA CATAGGGACC AGATCCCTCT CGGTATAGAS CAACTCATTC AATCCGTCWC 420
   KGCGCTTCGT TWYCCGGGCG GCAGCACGCG TRCYCAAGCT CGTTCGATTT TAATCCTCAT 480
   TCAGATGATC TCCGAGGCCG CCAGATTCAA TCCCATCTTA TGGAGGKMYC GCCAAKAYAT 540
   TAACAGTGGG GMRTCATTTC TGCCAGACRT GTACATGCTG GAGCTGGAGA CGAGTTGGGG 600
   CCAACAATCC ACGCAAGTCC AGCATTCAAC CGATGGCGTT TTTAATAACC CAWTYCGGTT     660
   GGCTATAYCY MCYGGTAACT TCGTGACGTT GWCYAATGTT CGCKMYGTGA TCGCCAGCTT     720
   GGCGATCATG TTGTTTGTAT GCGGAGAGCG GCCATCTTCC TCT                       763
(2) ANGABEN ZU SEQ ID NO: 14:
   (i) SEQUENZKENNZEICHEN:
      (A) LANGE: 793 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (ix) MERKMAL:
      (A) NAME/SCHLÜSSEL: misc_feature
      (B) LAGE:517
      (D) SONSTIGE ANGABEN:/product= "N ist GGC oder fehlt"
         /label= Z2
   (xi) SEQUENZBESCHREIBUNG: SEQ ID NO: 14:
   GATGATGTTA CCTGCAGTGC TTCGGAACCT ACGGTGCGGA TTGTGGGTCG AARTGGCATG     60
   YGCGTGGACG TCCGAGATGA CGATTTCCAC GATGGGAATC AGATACAGTT GTGGCCCTCC    120
   AAGTCCAACA ATGATCCGAA TCAGTTGTGG ACGATCAAAA GGGATRRMAC CATTCGATCC    180
   AATGGCAGCT GCTTGACCAC GTATGGCTAT ACTGCTGGCG TCTATGTGAT GATCTTCGAC    240
   TGTAATACTG CTGTGCGGGA GGCCACTATT TGGCAGATAT GGGRCAATGG GACCATCATC    300
   AATCCAAGAT CCAATCTGGT TTTGGCAGCA TCATCTGGAA TCAAAGGCAC TACGCTTACG    360
   GTGCAAACAC TGGATTACAC GTTGGGACAG GGCTGGCTTG CCGGTAATGA TACCGCCCCA    420
   CGCGAGGTGA CCATATATGG TTTCAGGGAC CTTTGCATGG AATCAAATSR AGGGAGTGTG    480
   TGGGTGGAGA CGTGCGWSAG TAGCCAAMAG AACCAANARA TGGGCTTTGT ACGGGGATGG    540
   TTCTATACGC CCCAAACAAA ACCAAGACCA ATGCCTCACC KBTGGGAGAG ACTCCGTTTC    600
   AACAGTAATC AATATAGTTA GCTGCAGCGS WGSWTCGKSK KSKCAGCGAT GGGTGTTTAC    660
   CAATGAAKRS GCCATTTTGA ATTTAAAGAV WRGSYYGRYS RTGGATGTGG CGCAAGCAAA    720
   TCCAAAGCTC CGCCGAATAA TTATCTATCC TGCCACAGGA AAACCAAATC AAATGTGGCT    780
   TCCCGTGYYM TGA                                                       793
(2) ANGABEN ZU SEQ ID NO: 15:
   (i) SEQUENZKENNZEICHEN:
      (A) LÄNGE: 1596 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 15:
   TACGAGAGGC TAAGACTCAG AGTTACGCAT CAAACCACGG GCGAGGAATA CTTCCGGTTC 60
   ATCACGCTTC TCCGAGATTA TGTCTCAAGC GGAAGCTTTT CCAATGAGAT ACCACTCTTG 120
   CGTCAGTCTA CGATCCCCGT CTCCGATGCG CAAAGATTTG TCTTGGTGGA GCTCACCAAC 180
   CAGGGGGGAG ACTCGATCAC GGCCGCCATC GACGTTACCA ATCTGTACGT CGTGGCTTAC 240
   CAAGCAGGCG ACCAATCCTA CTTTTTGCGC GACGCACCAC GCGGCGCGGA AACGCACCTC 300
   TTCACCGGCA CCACCCGATC CTCTCTCCCA TTCAACGGAA GCTACCCTGA TCTGGAGCGA 360
   TACGCCGGAC ATAGGGACCA GATCCCTCTC GGTATAGACC AACTCATTCA ATCCGTCACG 420
   GCGCTTCGTT TTCCGGGCGG CAGCACGCGT ACCCAAGCTC GTTCGATTTT AATCCTCATT 480
   CAGATGATCT CCGAGGCCGC CAGATTCAAT CCCATCTTAT GGAGGGCTCG CCAATACATT 540
   AACAGTGGGG CGTCATTTCT GCCAGACGTG TACATGCTGG AGCTGGAGAC GAGTTGGGGC 600
   CAACAATCCA CGCAAGTCCA GCATTCAACC GATGGCGTTT TTAATAACCC AATTCGGTTG 660
   GCTATACCCC CCGGTAACTT CGTGACGTTG ACCAATGTTC GCGACGTGAT CGCCAGCTTG 720
   GCGATCATGT TGTTTGTATG CGGAGAGCGG CCATCTTCCT CTGACGTGCG CTATTGGCCG 780
   CTGGTCATAC GACCCGTGAT AGCCGATGAT GTTACCTGCA GTGCTTCGGA ACCTACGGTG 840
   CGGATTGTGG GTCGAAATGG CATGTGCGTG GACGTCCGAG ATGACGATTT CCACGATGGG 900
   AATCAGATAC AGTTGTGGCC CTCCAAGTCC AACAATGATC CGAATCAGTT GTGGACGATC 960
   AAAAGGGATG GAACCATTCG ATCCAATGGC AGCTGCTTGA CCACGTATGG CTATACTGCT 1020
   GGCGTCTATG TGATGATCTT CGACTGTAAT ACTGCTGTGC GGGAGGCCAC TATTTGGCAG 1080
   ATATGGGGCA ATGGGACCAT CATCAATCCA AGATCCAATC TGGTTTTGGC AGCATCATCT 1140
   GGAATCAAAG GCACTACGCT TACGGTGCAA ACACTGGATT ACACGTTGGG ACAGGGCTGG 1200
   CTTGCCGGTA ATGATACCGC CCCACGCGAG GTGACCATAT ATGGTTTCAG GGACCTTTGC 1260
   ATGGAATCAA ATGGAGGGAG TGTGTGGGTG GAGACGTGCG TGAGTAGCCA ACAGAACCAA 1320
   AGATGGGCTT TGTACGGGGA TGGTTCTATA CGCCCCAAAC AAAACCAAGA CCAATGCCTC 1380
   ACCTGTGGGA GAGACTCCGT TTCAACAGTA ATCAATATAG TTAGCTGCAG CGCTGGATCG 1440
   TCTGGGCAGC GATGGGTGTT TACCAATGAA GGGGCCATTT TGAATTTAAA GAATGGGTTG 1500
   GCCATGGATG TGGCGCAAGC AAATCCAAAG CTCCGCCGAA TAATTATCTA TCCTGCCACA 1560
   GGAAAACCAA ATCAAATGTG GCTTCCCGTG CCATGA 1596
(2) ANGABEN ZU SEQ ID NO: 16:
   (i) SEQUENZKENNZEICHEN:
      (A) LÄNGE: 762 Basenpaare
      (B) ART: Nucleotid
      (C) STRANGFORM: Einzelstrang
      (D) TOPOLOGIE: linear
   (ii) ART DES MOLEKULS: Sonstige Nucleinsäure
   (xi) SEQUENZBESCHREIBUNG: SEQ ID NO: 16:
   TACGAGAGGC TAAGACTCAG AGTTACGCAT CAAACCACGG GCGAGGAATA CTTCCGGTTC   60
   ATCACGCTTC TCCGAGATTA TGTCTCAAGC GGAAGCTTTT CCAATGAGAT ACCACTCTTG  120
   CGTCAGTCTA CGATCCCCGT CTCCGATGCG CAAAGATTTG TCTTGGTGGA GCTCACCAAC  180
   CAGGGGCAGG ACTCGGTTAC GGCCGCCATC GACGTTACCA ATGCTTACGT CGTGGCTTAC  240
   CAAGCAGGCG ACCAATCCTA CTTTTTGCGC GACGCACCAC GCGGCGCGGA AACGCACCTC  300
   TTCACCGGCA CCACCCGATC CTCTCTCCCA TTCAACGGAA GCTACCCTGA TCTGGAGCGA  360
   TACGCCGGAC ATAGGGACCA GATCCCTCTC GGTATAGACC AACTCATTCA ATCCGTCACG  420
   GCGCTTCGTT TTCCGGGCGG CAGCACGCGT ACCCAAGCTC GTTCGATTTT AATCCTCATT  480
   CAGATGATCT CCGAGGCCGC CAGATTCAAT CCCATCTTAT GGAGGTACCG CCAATACATT  540
   AACAGTGGGG CGTCATTTCT GCCAGACGTG TACATGCTGG AGCTGGAGAC GAGTTGGGGC  600
   CAACAATCCA CGCAAGTCCA GCATTCAACC GATGGCGTTT TTAATAACCC AATTCGGTTG  660
   GCTATACCCC CCGGTAACTT CGTGACGTTG ACCAATGTTC GCGACGTGAT CGCCAGCTTG  720
   GCGATCATGT TGTTTGTATG CGGAGAGCGG CCATCTTCCT CT                     762
(2) ANGABEN ZU SEQ ID NO: 17:
   (i) SEQUENZKENNZEICHEN:
      (A) LÄNGE: 768 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO 17:
   TACGAGAGGC TAAGACTCAG AGTTACGCAT CAAACCACGG GCGATGAATA CTTCCGGTTC   60
   ATCACGCTTC TCCGAGATTA TGTCTCAAGC GGAAGCTTTT CCAATGAGAT ACCACTCTTG  120
   CGTCAGTCTA CGATCCCCGT CTCCGATGCG CAAAGATTTG TCTTGGTGGA GCTCACCAAC  180
   CAGGGGCAGG ACTCGATCAC GGCCGCCATC GACGTTACCA ATGCTTACGT CGTGGCTTAC 240
   CAAGCAGGCG ACCAATCCTA CTTTTTGCGC GACGCACCAC GCGGCGCGGA AACGCACCTC 300
   TTCACCGGCA CCACCCGAGA TAGATCCTCT CTCCCATTCA CTGGAAGCTA CACCGATCTG 360
   GAGCGATACG CCGGACATAG GGACCAGATC CCTCTCGGTA TAGAGCAACT CATTCAATCC 420
   GTCTCTGCGC TTCGTTACCC GGGCGGCAGC ACGCGTGCTC AAGCTCGTTC GATTTTAATC 480
   CTCATTCAGA TGATCTCCGA GGCCGCCAGA TTCAATCCCA TCTTATGGAG GTACCGCCAA 540
   GATATTAACA GTGGGGAATC ATTTCTGCCA GACATGTACA TGCTGGAGCT GGAGACGAGT 600
   TGGGGCCAAC AATCCACGCA AGTCCAGCAT TCAACCGATG GCGTTTTTAA TAACCCATTC 660
   CGGTTGGCTA TATCTACTGG TAACTTCGTG ACGTTGTCTA ATGTTCGCTC TGTGATCGCC 720
   AGCTTGGCGA TCATGTTGTT TGTATGCGGA GAGCGGCCAT CTTCCTCT 768
(2) ANGABEN ZU SEQ ID NO: 18:
   (i) SEQUENZKENNZEICHEN:
      (A) LÄNGE: 1596 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 18:
   TATGAAAGAT TGAGGTTGAG GGTGACTCAC CAGACTACAG GAGAAGAGTA TTTTAGATTT     60
   ATTACTTTGT TGAGGGATTA CGTTAGTTCT GGTTCTTTCA GTAACGAAAT TCCTTTGCTT    120
   AGACAATCTA CTATTCCAGT TTCTGATGCT CAGCGTTTCG TTCTTGTTGA ATTGACTAAC    180
   CAAGGAGGTG ATAGTATTAC TGCTGCTATT GATGTGACTA ACCTTTATGT TGTTGCATAT    240
   CAGGCTGGTG ATCAGTCTTA TTTCCTTAGG GATGCTCCTA GAGGAGCTGA GACTCATTTG    300
   TTTACTGGTA CAACACGGAG TTCTTTGCCT TTTAACGGTT CTTATCCAGA CTTGGAAAGA    360
   TATGCTGGTC ACAGAGATCA AATTCCATTG GGAATTGATC AGTTGATCCA GAGTGTTACT    420
   GCTTTGAGAT TCCCAGGTGG ATCTACTAGA ACACAGGCAA GATCTATCCT TATTTTGATC    480
   CAAATGATTA GTGAAGCTGC TAGGTTTAAC CCTATTCTTT GGAGAGCAAG ACAGTATATC    540
   AACTCTGGTG CTTCTTTCCT TCCTGATGTT TATATGCTTG AACTTGAAAC TTCATGGGGA    600
   CAGCAGTCTA CTCAGGTTCA ACACAGTACA GACGGTGTGT TCAACAATCC TATCAGACTT    660
   GCAATTCCAC CTGGAAATTT TGTTACTCTT ACAAACGTGA GAGATGTTAT TGCTTCTCTT    720
   GCTATTATGC TTTTCGTTTG TGGTGAAAGA CCTTCTAGTT CTGATGTTAG ATACTGGCCA    780
   TTGGTTATTA GGCCTGTTAT CGCTGACGAT GTGACATGTT CTGCATCTGA ACCAACTGTT    840
   AGGATCGTTG GAAGAAACGG TATGTGTGTT GATGTTCGGG ACGATGACTT TCATGACGGT    900
   AACCAAATCC AACTTTGGCC TAGTAAGTCT AATAACGACC CAAACCAACT TTGGACTATT    960
   AAGAGAGACG GTACAATCAG GTCTAACGGA TCTTGTCTTA CTACATACGG TTACACTGCA   1020
   GGAGTTTACG TTATGATTTT TGATTGCAAC ACAGCAGTTA GAGAAGCTAC AATCTGGCAA   1080
   ATCTGGGGTA ACGGAACTAT TATTAACCCT CGTTCTAACT TGGTGCTTGC TGCTTCTAGT   1140
   GGTATTAAGG GAACAACTTT GACTGTTCAG ACTTTGGACT ATACTCTTGG TCAAGGATGG   1200
   TTGGCTGGAA ACGACACAGC TCCTAGAGAA GTTACAATCT ACGGATTTAG AGATTTGTGT   1260
   ATGGAGTCTA ACGGTGGATC TGTTTGGGTT GAAACTTGTG TTTCATCTCA GCAAAATCAG   1320
   AGGTGGGCAC TTTATGGTGA CGGAAGTATC AGACCTAAGC AGAATCAGGA TCAGTGTTTG   1380
   ACATGCGGTA GGGATAGTGT GTCTACTGTT ATTAACATTG TGTCTTGTTC TGCAGGTAGT   1440
   TCTGGACAAA GGTGGGTTTT CACAAACGAG GGTGCTATCC TTAACTTGAA GAACGGTCTT   1500
   GCTATGGATG TTGCTCAGGC TAACCCTAAG TTGAGAAGGA TTATCATTTA CCCAGCTACT   1560
   GGTAAGCCTA ACCAGATGTG GTTGCCAGTT CCTTAT                             1596
(2) ANGABEN ZU SEQ ID NO: 19:
   (i) SEQUENZKENNZEICHEN:
      (A) LÄNGE: 762 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 19:
   TATGAAAGAT TGAGGTTGAG GGTGACTCAC CAGACTACAG GAGAAGAGTA TTTTAGATTT     60
   ATTACTTTGT TGAGGGATTA CGTTAGTTCT GGTTCTTTCA GTAACGAAAT TCCTTTGCTT    120
   AGACAATCTA CTATTCCAGT TTCTGATGCT CAGCGTTTCG TTCTTGTTGA ATTGACTAAC    180
   CAAGGACAGG ATAGTGTTAC TGCTGCTATT GATGTGACTA ACGCTTATGT TGTTGCATAT    240
   CAGGCTGGTG ATCAGTCTTA TTTCCTTAGG GATGCTCCTA GAGGAGCTGA GACTCATTTG    300
   TTTACTGGTA CAACACGGAG TTCTTTGCCT TTTAACGGTT CTTATCCAGA CTTGGAAAGA    360
   TATGCTGGTC ACAGAGATCA AATTCCATTG GGAATTGATC AGTTGATCCA GAGTGTTACT    420
   GCTTTGAGAT TCCCAGGTGG ATCTACTAGA ACACAGGCAA GATCTATCCT TATTTTGATC    480
   CAAATGATTA GTGAAGCTGC TAGGTTTAAC CCTATTCTTT GGAGATACAG ACAGTATATC    540
   AACTCTGGTG CTTCTTTCCT TCCTGATGTT TATATGCTTG AACTTGAAAC TTCATGGGGA    600
   CAGCAGTCTA CTCAGGTTCA ACACAGTACA GACGGTGTGT TCAACAATCC TATCAGACTT    660
   GCAATTCCAC CTGGAAATTT TGTTACTCTT ACAAACGTGA GAGATGTTAT TGCTTCTCTT    720
   GCTATTATGC TTTTCGTTTG TGGTGAAAGA CCTTCTAGTT CT                       762
(2) ANGABEN ZU SEQ ID NO: 20:
   (i) SEQUENZKENNZEICHEN:
      (A) LÄNGE: 768 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 20:
   TATGAAAGAT TGAGGTTGAG GGTGACTCAC CAGACTACAG GAGATGAGTA TTTTAGATTT    60
   ATTACTTTGT TGAGGGATTA CGTTAGTTCT GGTTCTTTCA GTAACGAAAT TCCTTTGCTT   120
   AGACAATCTA CTATTCCAGT TTCTGATGCT CAGCGTTTCG TTCTTGTTGA ATTGACTAAC   180
   CAAGGACAGG ATAGTATTAC TGCTGCTATT GATGTGACTA ACGCTTATGT TGTTGCATAT   240
   CAGGCTGGTG ATCAGTCTTA TTTCCTTAGG GATGCTCCTA GAGGAGCTGA GACTCATTTG   300
   TTTACTGGTA CAACACGGGA TAGAAGTTCT TTGCCTTTTA CTGGTTCTTA TACAGACTTG   360
   GAAAGATATG CTGGTCACAG AGATCAAATT CCATTGGGAA TTGAGCAGTT GATCCAGAGT   420
   GTTTCTGCTT TGAGATACCC AGGTGGATCT ACTAGAGCTC AGGCAAGATC TATCCTTATT   480
   TTGATCCAAA TGATTAGTGA AGCTGCTAGG TTTAACCCTA TTCTTTGGAG ATACAGACAG   540
   GATATCAACT CTGGTGAATC TTTCCTTCCT GATATGTATA TGCTTGAACT TGAAACTTCA   600
   TGGGGACAGC AGTCTACTCA GGTTCAACAC AGTACAGACG GTGTGTTCAA CAATCCTTTC   660
   AGACTTGCAA TTTCTACTGG AAATTTTGTT ACTCTTTCTA ACGTGAGATC TGTTATTGCT   720
   TCTCTTGCTA TTATGCTTTT CGTTTGTGGT GAAAGACCTT CTAGTTCT                768
(2) ANGABEN ZU SEQ ID NO: 21:
   (i) SEQUENZKENNZEICHEN:
      (A) LÄNGE: 792 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 21:
   GATGATGTTA CCTGCAGTGC TTCGGAACCT ACGGTGCGGA TTGTGGGTCG AAATGGCATG    60
   TGCGTGGACG TCCGAGATGA CGATTTCCAC GATGGGAATC AGATACAGTT GTGGCCCTCC   120
   AAGTCCAACA ATGATCCGAA TCAGTTGTGG ACGATCAAAA GGGATGGAAC CATTCGATCC   180
   AATGGCAGCT GCTTGACCAC GTATGGCTAT ACTGCTGGCG TCTATGTGAT GATCTTCGAC   240
   TGTAATACTG CTGTGCGGGA GGCCACTATT TGGCAGATAT GGGGCAATGG GACCATCATC   300
   AATCCAAGAT CCAATCTGGT TTTGGCAGCA TCATCTGGAA TCAAAGGCAC TACGCTTACG   360
   GTGCAAACAC TGGATTACAC GTTGGGACAG GGCTGGCTTG CCGGTAATGA TACCGCCCCA   420
   CGCGAGGTGA CCATATATGG TTTCAGGGAC CTTTGCATGG AATCAAATGG AGGGAGTGTG   480
   TGGGTGGAGA CGTGCGTGAG TAGCCAACAG AACCAAAGAT GGGCTTTGTA CGGGGATGGT   540
   TCTATACGCC CCAAACAAAA CCAAGACCAA TGCCTCACCT GTGGGAGAGA CTCCGTTTCA   600
   ACAGTAATCA ATATAGTTAG CTGCAGCGCT GGATCGTCTG GGCAGCGATG GGTGTTTACC   660
   AATGAAGGGG CCATTTTGAA TTTAAAGAAT GGGTTGGCCA TGGATGTGGC GCAAGCAAAT   720
   CCAAAGCTCC GCCGAATAAT TATCTATCCT GCCACAGGAA AACCAAATCA AATGTGGCTT   780
   CCCGTGCCAT GA                                                       792
(2) ANGABEN ZU SEQ ID NO: 22:
   (i) SEQUENZKENNZEICHEN:
      (A) LÄNGE: 795 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 22:
   GATGATGTTA CCTGCAGTGC TTCGGAACCT ACGGTGCGGA TTGTGGGTCG AAATGGCATG    60
   CGCGTGGACG TCCGAGATGA CGATTTCCAC GATGGGAATC AGATACAGTT GTGGCCCTCC   120
   AAGTCCAACA ATGATCCGAA TCAGTTGTGG ACGATCAAAA GGGATGGAAC CATTCGATCC   180
   AATGGCAGCT GCTTGACCAC GTATGGCTAT ACTGCTGGCG TCTATGTGAT GATCTTCGAC   240
   TGTAATACTG CTGTGCGGGA GGCCACTATT TGGCAGATAT GGGACAATGG GACCATCATC   300
   AATCCAAGAT CCAATCTGGT TTTGGCAGCA TCATCTGGAA TCAAAGGCAC TACGCTTACG   360
   GTGCAAACAC TGGATTACAC GTTGGGACAG GGCTGGCTTG CCGGTAATGA TACCGCCCCA   420
   CGCGAGGTGA CCATATATGG TTTCAGGGAC CTTTGCATGG AATCAAATGG AGGGAGTGTG   480
   TGGGTGGAGA CGTGCGACAG TAGCCAAAAG AACCAAGGCA AATGGGCTTT GTACGGGGAT   540
   GGTTCTATAC GCCCCAAACA AAACCAAGAC CAATGCCTCA CCTCTGGGAG AGACTCCGTT   600
   TCAACAGTAA TCAATATAGT TAGCTGCAGC GGAGCTTCGG GGTCTCAGCG ATGGGTGTTT    660
   ACCAATGAAG GGGCCATTTT GAATTTAAAG AATGGGTTGG CCATGGATGT GGCGCAAGCA    720
   AATCCAAAGC TCCGCCGAAT AATTATCTAT CCTGCCACAG GAAAACCAAA TCAAATGTGG    780
   CTTCCCGTGT TCTGA                                                     795
(2) ANGABEN ZU SEQ ID NO: 23:
   (i) SEQUENZKENNZEICHEN:
      (A) LÄNGE: 795 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 23:
   GATGATGTTA CCTGCAGTGC TTCGGAACCT ACGGTGCGGA TTGTGGGTCG AAGTGGCATG     60
   CGCGTGGACG TCCGAGATGA CGATTTCCAC GATGGGAATC AGATACAGTT GTGGCCCTCC    120
   AAGTCCAACA ATGATCCGAA TCAGTTGTGG ACGATCAAAA GGGATAACAC CATTCGATCC    180
   AATGGCAGCT GCTTGACCAC GTATGGCTAT ACTGCTGGCG TCTATGTGAT GATCTTCGAC    240
   TGTAATACTG CTGTGCGGGA GGCCACTATT TGGCAGATAT GGGACAATGG GACCATCATC    300
   AATCCAAGAT CCAATCTGGT TTTGGCAGCA TCATCTGGAA TCAAAGGCAC TACGCTTACG    360
   GTGCAAACAC TGGATTACAC GTTGGGACAG GGCTGGCTTG CCGGTAATGA TACCGCCCCA    420
   CGCGAGGTGA CCATATATGG TTTCAGGGAC CTTTGCATGG AATCAAATCA AGGGAGTGTG    480
   TGGGTGGAGA CGTGCGACAG TAGCCAAAAG AACCAAGGCA AATGGGCTTT GTACGGGGAT    540
   GGTTCTATAC GCCCCAAACA AAACCAAGAC CAATGCCTCA CCGTTGGGAG AGACTCCGTT    600
   TCAACAGTAA TCAATATAGT TAGCTGCAGC GGAGCTTCGG GGTCTCAGCG ATGGGTGTTT    660
   ACCAATGAAT ACGCCATTTT GAATTTAAAG AGTGGGTTGG CCATGGATGT GGCGCAAGCA    720
   AATCCAAAGC TCCGCCGAAT AATTATCTAT CCTGCCACAG GAAAACCAAA TCAAATGTGG    780
   CTTCCCGTGT TCTGA                                                     795
(2) ANGABEN ZU SEQ ID NO: 24:
   (i) SEQUENZKENNZEICHEN:
      (A) LÄNGE: 795 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 24:
   GATGATGTTA CCTGCAGTGC TTCGGAACCT ACGGTGCGGA TTGTGGGTCG AAATGGCATG    60
   CGCGTGGACG TCCGAGATGA CGATTTCCAC GATGGGAATC AGATACAGTT GTGGCCCTCC   120
   AAGTCCAACA ATGATCCGAA TCAGTTGTGG ACGATCAAAA GGGATGGAAC CATTCGATCC   180
   AATGGCAGCT GCTTGACCAC GTATGGCTAT ACTGCTGGCG TCTATGTGAT GATCTTCGAC   240
   TGTAATACTG CTGTGCGGGA GGCCACTATT TGGCAGATAT GGGACAATGG GACCATCATC   300
   AATCCAAGAT CCAATCTGGT TTTGGCAGCA TCATCTGGAA TCAAAGGCAC TACGCTTACG   360
   GTGCAAACAC TGGATTACAC GTTGGGACAG GGCTGGCTTG CCGGTAATGA TACCGCCCCA   420
   CGCGAGGTGA CCATATATGG TTTCAGGGAC CTTTGCATGG AATCAAATGG AGGGAGTGTG   480
   TGGGTGGAGA CGTGCGACAG TAGCCAAAAG AACCAAGGCA AATGGGCTTT GTACGGGGAT   540
   GGTTCTATAC GCCCCAAACA AAACCAAGAC CAATGCCTCA CCTCTGGGAG AGACTCCGTT   600
   TCAACAGTAA TCAATATAGT TAGCTGCAGC GGAGCTTCGG GGTCTCAGCG ATGGGTGTTT   660
   ACCAATGAAG GGGCCATTTT GAATTTAAAG ACTGGGTTGG CCATGGATGT GGCGCAAGCA   720
   AATCCAAAGC TCCGCCGAAT AATTATCTAT CCTGCCACAG GAAAACCAAA TCAAATGTGG   780
   CTTCCCGTGT TCTGA                                                    795
(2) ANGABEN ZU SEQ ID NO: 25:
   (i) SEQUENZKENNZEICHEN:
      (A) LÄNGE: 795 Basenpaare
      (B) ART: Nucleotid
      (C) STRANGFORM: Einzelstrang
      (D) TOPOLOGIE: linear
   (ii) ART DES MOLEKULS: Sonstige Nucleinsäure
   (xi) SEQUENZBESCHREIBUNG: SEQ ID NO: 25:
   GATGATGTTA CCTGCAGTGC TTCGGAACCT ACGGTGCGGA TTGTGGGTCG AAATGGCATG    60
   CGCGTGGACG TCCGAGATGA CGATTTCCAC GATGGGAATC AGATACAGTT GTGGCCCTCC   120
   AAGTCCAACA ATGATCCGAA TCAGTTGTGG ACGATCAAAA GGGATGGAAC CATTCGATCC   180
   AATGGCAGCT GCTTGACCAC GTATGGCTAT ACTGCTGGCG TCTATGTGAT GATCTTCGAC   240
   TGTAATACTG CTGTGCGGGA GGCCACTATT TGGCAGATAT GGGACAATGG GACCATCATC   300
   AATCCAAGAT CCAATCTGGT TTTGGCAGCA TCATCTGGAA TCAAAGGCAC TACGCTTACG   360
   GTGCAAACAC TGGATTACAC GTTGGGACAG GGCTGGCTTG CCGGTAATGA TACCGCCCCA   420
   CGCGAGGTGA CCATATATGG TTTCAGGGAC CTTTGCATGG AATCAAATGG AGGGAGTGTG   480
   TGGGTGGAGA CGTGCGACAG TAGCCAAAAG AACCAAGGCA AATGGGCTTT GTACGGGGAT 540
   GGTTCTATAC GCCCCAAACA AAACCAAGAC CAATGCCTCA CCTCTGGGAG AGACTCCGTT 600
   TCAACAGTAA TCAATATAGT TAGCTGCAGC GGAGCTTCGG GGTCTCAGCG ATGGGTGTTT 660
   ACCAATGAAG GGGCCATTTT GAATTTAAAG AAAGGGCCGG CCATGGATGT GGCGCAAGCA 720
   AATCCAAAGC TCCGCCGAAT AATTATCTAT CCTGCCACAG GAAAACCAAA TCAAATGTGG 780
   CTTCCCGTGT TCTGA 795
(2) ANGABEN ZU SEQ ID NO: 26:
   (i) SEQUENZKENNZEICHEN:
      (A) LÄNGE: 795 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 26:
   GATGATGTTA CCTGCAGTGC TTCGGAACCT ACGGTGCGGA TTGTGGGTCG AAATGGCATG 60
   CGCGTGGACG TCCGAGATGA CGATTTCCAC GATGGGAATC AGATACAGTT GTGGCCCTCC 120
   AAGTCCAACA ATGATCCGAA TCAGTTGTGG ACGATCAAAA GGGATGGAAC CATTCGATCC 180
   AATGGCAGCT GCTTGACCAC GTATGGCTAT ACTGCTGGCG TCTATGTGAT GATCTTCGAC 240
   TGTAATACTG CTGTGCGGGA GGCCACTATT TGGCAGATAT GGGACAATGG GACCATCATC 300
   AATCCAAGAT CCAATCTGGT TTTGGCAGCA TCATCTGGAA TCAAAGGCAC TACGCTTACG 360
   GTGCAAACAC TGGATTACAC GTTGGGACAG GGCTGGCTTG CCGGTAATGA TACCGCCCCA 420
   CGCGAGGTGA CCATATATGG TTTCAGGGAC CTTTGCATGG AATCAAATGG AGGGAGTGTG 480
   TGGGTGGAGA CGTGCGACAG TAGCCAAAAG AACCAAGGCA AATGGGCTTT GTACGGGGAT 540
   GGTTCTATAC GCCCCAAACA AAACCAAGAC CAATGCCTCA CCTCTGGGAG AGACTCCGTT 600
   TCAACAGTAA TCAATATAGT TAGCTGCAGC GGAGCTTCGG GGTCTCAGCG ATGGGTGTTT 660
   ACCAATGAAG GGGCCATTTT GAATTTAAAG AATAGCTTGA TGGTGGATGT GGCGCAAGCA 720
   AATCCAAAGC TCCGCCGAAT AATTATCTAT CCTGCCACAG GAAAACCAAA TCAAATGTGG 780
   CTTCCCGTGT TCTGA 795
(2) ANGABEN ZU SEQ ID NO: 27:
   (i) SEQUENZKENNZEICHEN:
      (A) LÄNGE: 792 Basenpaare
      (B) ART: Nucleotid
      (C) STRANGFORM: Einzelstrang
      (D) TOPOLOGIE: linear
   (ii) ART DES MOLEKULS: Sonstige Nucleinsäure
   (xi) SEQUENZBESCHREIBUNG: SEQ ID NO: 27:
   GACGATGTGA CATGTTCTGC ATCTGAACCA ACTGTTAGGA TCGTTGGAAG AAACGGTATG    60
   TGTGTTGATG TTCGGGACGA TGACTTTCAT GACGGTAACC AAATCCAACT TTGGCCTAGT   120
   AAGTCTAATA ACGACCCAAA CCAACTTTGG ACTATTAAGA GAGACGGTAC AATCAGGTCT   180
   AACGGATCTT GTCTTACTAC ATACGGTTAC ACTGCAGGAG TTTACGTTAT GATTTTTGAT   240
   TGCAACACAG CAGTTAGAGA AGCTACAATC TGGCAAATCT GGGGTAACGG AACTATTATT   300
   AACCCTCGTT CTAACTTGGT GCTTGCTGCT TCTAGTGGTA TTAAGGGAAC AACTTTGACT   360
   GTTCAGACTT TGGACTATAC TCTTGGTCAA GGATGGTTGG CTGGAAACGA CACAGCTCCT   420
   AGAGAAGTTA CAATCTACGG ATTTAGAGAT TTGTGTATGG AGTCTAACGG TGGATCTGTT   480
   TGGGTTGAAA CTTGTGTTTC ATCTCAGCAA AATCAGAGGT GGGCACTTTA TGGTGACGGA   540
   AGTATCAGAC CTAAGCAGAA TCAGGATCAG TGTTTGACAT GCGGTAGGGA TAGTGTGTCT   600
   ACTGTTATTA ACATTGTGTC TTGTTCTGCA GGTAGTTCTG GACAAAGGTG GGTTTTCACA   660
   AACGAGGGTG CTATCCTTAA CTTGAAGAAC GGTCTTGCTA TGGATGTTGC TCAGGCTAAC   720
   CCTAAGTTGA GAAGGATTAT CATTTACCCA GCTACTGGTA AGCCTAACCA GATGTGGTTG   780
   CCAGTTCCTT AT                                                       792
(2) ANGABEN ZU SEQ ID NO: 28:
   (i) SEQUENZKENNZEICHEN:
      (A) LÄNGE: 795 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 28:
(2).ANGABEN ZU SEQ ID NO: 29:
   (i) SEQUENZKENNZEICHEN:
      (A) LÄNGE: 795 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 29:
(2) ANGABEN ZU SEQ ID NO: 30:
   (i) SEQUENZKENNZEICHEN:
      (A) LÄNGE: 795 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 30:
   GACGATGTGA CATGTTCTGC ATCTGAACCA ACTGTTAGGA TCGTTGGAAG AAACGGTATG   60
   CGTGTTGATG TTCGGGACGA TGACTTTCAT GACGGTAACC AAATCCAACT TTGGCCTAGT  120
   AAGTCTAATA ACGACCCAAA CCAACTTTGG ACTATTAAGA GAGACGGTAC AATCAGGTCT  180
   AACGGATCTT GTCTTACTAC ATACGGTTAC ACTGCAGGAG TTTACGTTAT GATTTTTGAT  240
   TGCAACACAG CAGTTAGAGA AGCTACAATC TGGCAAATCT GGGATAACGG AACTATTATT  300
   AACCCTCGTT CTAACTTGGT GCTTGCTGCT TCTAGTGGTA TTAAGGGAAC AACTTTGACT  360
   GTTCAGACTT TGGACTATAC TCTTGGTCAA GGATGGTTGG CTGGAAACGA CACAGCTCCT  420
   AGAGAAGTTA CAATCTACGG ATTTAGAGAT TTGTGTATGG AGTCTAACGG TGGATCTGTT  480
   TGGGTTGAAA CTTGTGATTC ATCTCAGAAA AATCAGGGCA AGTGGGCACT TTATGGTGAC  540
   GGAAGTATCA GACCTAAGCA GAATCAGGAT CAGTGTTTGA CATCCGGTAG GGATAGTGTG  600
   TCTACTGTTA TTAACATTGT GTCTTGTTCT GGAGCTAGTG GATCTCAAAG GTGGGTTTTC  660
   ACAAACGAGG GTGCTATCCT TAACTTGAAG ACCGGTCTTG CTATGGATGT TGCTCAGGCT  720
   AACCCTAAGT TGAGAAGGAT TATCATTTAC CCAGCTACTG GTAAGCCTAA CCAGATGTGG  780
   TTGCCAGTTT TTTAT                                                   795
(2) ANGABEN ZU SEQ ID NO: 31:
   (i) SEQUENZKENNZEICHEN:
      (A) LÄNGE: 795 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 31:
   GACGATGTGA CATGTTCTGC ATCTGAACCA ACTGTTAGGA TCGTTGGAAG AAACGGTATG   60
   CGTGTTGATG TTCGGGACGA TGACTTTCAT GACGGTAACC AAATCCAACT TTGGCCTAGT  120
   AAGTCTAATA ACGACCCAAA CCAACTTTGG ACTATTAAGA GAGACGGTAC AATCAGGTCT  180
   AACGGATCTT GTCTTACTAC ATACGGTTAC ACTGCAGGAG TTTACGTTAT GATTTTTGAT  240
   TGCAACACAG CAGTTAGAGA AGCTACAATC TGGCAAATCT GGGATAACGG AACTATTATT   300
   AACCCTCGTT CTAACTTGGT GCTTGCTGCT TCTAGTGGTA TTAAGGGAAC AACTTTGACT   360
   GTTCAGACTT TGGACTATAC TCTTGGTCAA GGATGGTTGG CTGGAAACGA CACAGCTCCT   420
   AGAGAAGTTA CAATCTACGG ATTTAGAGAT TTGTGTATGG AGTCTAACGG TGGATCTGTT   480
   TGGGTTGAAA CTTGTGATTC ATCTCAGAAA AATCAGGGCA AGTGGGCACT TTATGGTGAC   540
   GGAAGTATCA GACCTAAGCA GAATCAGGAT CAGTGTTTGA CATCCGGTAG GGATAGTGTG   600
   TCTACTGTTA TTAACATTGT GTCTTGTTCT GGAGCTAGTG GATCTCAAAG GTGGGTTTTC   660
   ACAAACGAGG GTGCTATCCT TAACTTGAAG AAAGGTCCTG CTATGGATGT TGCTCAGGCT   720
   AACCCTAAGT TGAGAAGGAT TATCATTTAC CCAGCTACTG GTAAGCCTAA CCAGATGTGG   780
   TTGCCAGTTT TTTAT                                                    795
(2) ANGABEN ZU SEQ ID NO: 32:
   (i) SEQUENZKENNZEICHEN:
      (A) LÄNGE: 795 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 32:
   GACGATGTGA CATGTTCTGC ATCTGAACCA ACTGTTAGGA TCGTTGGAAG AAACGGTATG     60
   CGTGTTGATG TTCGGGACGA TGACTTTCAT GACGGTAACC AAATCCAACT TTGGCCTAGT    120
   AAGTCTAATA ACGACCCAAA CCAACTTTGG ACTATTAAGA GAGACGGTAC AATCAGGTCT    180
   AACGGATCTT GTCTTACTAC ATACGGTTAC ACTGCAGGAG TTTACGTTAT GATTTTTGAT    240
   TGCAACACAG CAGTTAGAGA AGCTACAATC TGGCAAATCT GGGATAACGG AACTATTATT    300
   AACCCTCGTT CTAACTTGGT GCTTGCTGCT TCTAGTGGTA TTAAGGGAAC AACTTTGACT    360
   GTTCAGACTT TGGACTATAC TCTTGGTCAA GGATGGTTGG CTGGAAACGA CACAGCTCCT    420
   AGAGAAGTTA CAATCTACGG ATTTAGAGAT TTGTGTATGG AGTCTAACGG TGGATCTGTT    480
   TGGGTTGAAA CTTGTGATTC ATCTCAGAAA AATCAGGGCA AGTGGGCACT TTATGGTGAC    540
   GGAAGTATCA GACCTAAGCA GAATCAGGAT CAGTGTTTGA CATCCGGTAG GGATAGTGTG    600
   TCTACTGTTA TTAACATTGT GTCTTGTTCT GGAGCTAGTG GATCTCAAAG GTGGGTTTTC    660
   ACAAACGAGG GTGCTATCCT TAACTTGAAG AACTCTCTTA TGGTGGATGT TGCTCAGGCT    720
   AACCCTAAGT TGAGAAGGAT TATCATTTAC CCAGCTACTG GTAAGCCTAA CCAGATGTGG    780
   TTGCCAGTTT TTTAT                                                     795
(2) ANGABEN ZU SEQ ID NO: 33:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 33:
   GTNMGNGAYG AYGAYTTYCA     20
(2) ANGABEN ZU SEQ ID NO: 34:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 34:
   ATYTGRTTNG GYTTNCCNGT 20
(2) ANGABEN ZU SEQ ID NO: 35:
   (i) SEQUENZKENNZEICHEN:
      (A) LÄNGE: 21 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 35:
   CACAGCAGTA TTACAGTCGA A 21
(2) ANGABEN ZU SEQ ID NO: 36:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 36:
   GTCTATGTGA TGATCTTCGA CTGT 24